(19)

Europäisches
Patentamt

European
Patent Office

Office européen
des brevets

(11)  **EP 4 271 461 B1**

(12)  **EUROPEAN PATENT SPECIFICATION**

(45) Date of publication and mention
of the grant of the patent:
**13.03.2024  Bulletin 2024/11**

(21) Application number: **22772586.8**

(22) Date of filing: **30.08.2022**

(51) International Patent Classification (IPC):
*A61M 60/818* (2021.01)   *A61M 60/824* (2021.01)
*A61M 60/13* (2021.01)   *A61M 60/237* (2021.01)
*A61M 60/414* (2021.01)   *A61M 60/531* (2021.01)
*A61M 60/81* (2021.01)   *A61M 60/816* (2021.01)
*A61M 60/865* (2021.01)

(52) Cooperative Patent Classification (CPC):
**A61M 60/13; A61M 60/237; A61M 60/414;
A61M 60/804; A61M 60/808; A61M 60/818**

(86) International application number:
**PCT/IB2022/058101**

(87) International publication number:
**WO 2023/062453 (20.04.2023 Gazette 2023/16)**

(54) **VENTRICULAR ASSIST DEVICE**

VENTRIKULÄRE HILFSVORRICHTUNG

DISPOSITIF D'ASSISTANCE VENTRICULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.10.2021  US 202163254321 P
07.03.2022  US 202263317199 P**

(43) Date of publication of application:
**08.11.2023  Bulletin 2023/45**

(60) Divisional application:
**23189145.8 / 4 252 825
23189147.4 / 4 252 826
23189148.2 / 4 252 827
23189149.0 / 4 252 828**

(73) Proprietor: **Magenta Medical Ltd.
6092000 Kadima (IL)**

(72) Inventors:
• **TUVAL, Yosi
40500 Even Yehuda (IL)**
• **LUBINSKY, Gad
4069600 Ein Vered (IL)**

• **TROSHIN, Victor
4532109 Hod-Hasharon (IL)**
• **ZEMER HAREL, Hagit
4464630 Kfar Saba (IL)**
• **FRIEDLAND, Ori
6801241 Tel Aviv (IL)**
• **ROSENBLUM, Daniel
4729423 Ramat Hasharon (IL)**
• **ROZENFELD, Avi
3460205 Haifa (IL)**

(74) Representative: **Evens, Paul Jonathan et al
Maguire Boss
24 East Street
St. Ives, Cambridgeshire PE27 5PD (GB)**

(56) References cited:
EP-A1- 3 545 983      GB-A- 2 504 175
US-A1- 2010 049 313   US-A1- 2010 152 523
US-A1- 2012 237 353   US-A1- 2013 085 318
US-A1- 2017 348 470   US-A1- 2019 060 539
US-A1- 2019 209 755   US-A1- 2019 307 561
US-A1- 2020 093 973   US-A1- 2020 237 982
US-A1- 2021 170 081   US-B1- 8 690 749
US-B2- 9 533 084

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 271 461 B1

**Description**

**FIELD OF EMBODIMENTS OF THE INVENTION**

The present invention generally relates to a ventricular assist device.

**BACKGROUND**

Ventricular assist devices are mechanical circulatory support devices designed to assist and unload cardiac chambers in order to maintain or augment cardiac output. They are used in patients suffering from a failing heart and in patients at risk for deterioration of cardiac function during percutaneous coronary interventions. Most commonly, a left-ventricular assist device is applied to a defective heart in order to assist left-ventricular functioning. In some cases, a right-ventricular assist device is used in order to assist right-ventricular functioning. Such ventricular assist devices are either designed to be permanently implanted or mounted on a catheter for temporary placement. US2020/0237982 A1 discloses a typical example of such ventricular assist device.

**SUMMARY OF EMBODIMENTS**

In accordance with the present invention, a ventricular assist device includes a motion-cushioning spring. As described in further detail hereinbelow, typically, during operation of the ventricular assist device, i.e., as an impeller of the ventricular assist device is rotating, the impeller of the ventricular assist device undergoes axial back-and-forth motion. For some applications, as the impeller undergoes the axial back-and-forth motion, the motion-cushioning spring is configured to act as a shock absorber, to provide cushioning to the motion. As the impeller moves distally from a systolic position to a diastolic position, the motion-cushioning spring becomes more compressed. For some applications, the impeller is configured to be radially constrained (i.e., crimped) by becoming axially elongated, and the motion-cushioning spring is configured to become compressed such as to accommodate the axial elongation of the impeller.

For some applications, the motion-cushioning spring is coupled to an elastomeric material (such as polyurethane, and/or silicone), such that at least a portion of an axial shaft of the ventricular assist device that is between a distal end of the impeller and a distal radial bearing is covered by the elastomeric material. For some applications, coupling the elastomeric material to the spring reduces a risk of the generation of thrombi and/or hemolysis by the spring, relative to if the spring is not coupled to the elastomeric material. It is noted that the scope of the present disclosure includes providing the motion-cushioning spring in the absence of elastomeric material, as may be desirable in some cases. For some applications, the spring is coated with the elastomeric material with the elastomeric material extending between adjacent windings of the spring. Alternatively, the spring is embedded within the elastomeric material. Typically, the elastomeric material is coupled to the motion-cushioning spring in such a manner that the elastomeric material changes shape (e.g., by stretching and compressing) to conform to shape changes that the motion-cushioning spring undergoes (e.g., when the motion-cushioning spring undergoes elongation and compression). Further typically, the elastomeric material is configured to undergo the above-described shape changes without the elastomeric material becoming broken or collapsing, and without the elastomeric material becoming creased when the spring is compressed.

For some applications, a proximal motion-cushioning spring is disposed on the proximal side of the impeller. For some such applications, the proximal motion-cushioning spring is disposed around the axial shaft between the proximal end of the impeller and a proximal bearing. For some applications, the pump head includes both a proximal motion-cushioning spring (disposed on a proximal side of the impeller) and a distal motion-cushioning spring disposed on a distal side of the impeller, such that axial movement of the impeller in either the distal or the proximal direction is cushioned by the motion-cushioning springs.

For some applications, a ventricular assist device includes a distal thrust bearing that is configured to prevent an axial shaft of the device from undergoing axial motion in response to variations in the pressure gradient against which the impeller is pumping (and, typically, to thereby prevent the impeller from undergoing axial motion in response to variations in the pressure gradient against which the impeller is pumping). For some applications, the thrust bearing is disposed within a frame of the device. Typically, the thrust bearing is coupled to the frame via connecting struts, which extend radially inwardly from the frame to the thrust bearing. Typically, in order to manufacture the frame, the frame is cut from a tube of a shape-memory alloy, such as nitinol. For some applications, the connecting struts are cut from the tube from which the frame is cut, such that the frame and the connecting struts form a single integral body, without requiring coupling to each other (e.g., via adhesive, welding, etc.). For some applications, the frame and the connecting struts are cut from a single piece of a material such as to form a single integral body. For some applications, the connecting struts as well as the thrust bearing itself are cut from the tube from which the frame is cut, such that the frame, the connecting struts, and the thrust bearing form a single integral body, without requiring coupling to each other (e.g., via adhesive, welding, etc.). In general, for some applications, the frame, the connecting struts, and the thrust bearing itself are cut from a single piece of a material such as to form a single integral body.

For some applications, a spring that is generally similar to the above-described motion-cushioning spring is used in combination with the thrust bearing (or with a differently designed thrust bearing that is disposed distally of the impeller). For some applications, the spring assists in stabilizing the impeller (e.g., the distal end of the impeller) with respect to the thrust bearing, subsequent to the radial expansion of the impeller. Thus, the spring functions as an impeller-stabilizing spring. For some applications, the impeller is configured to be radially constrained (i.e., crimped) by becoming axially elongated, and the spring is configured to become compressed such as to accommodate the axial elongation of the impeller. Typically, when the impeller is in a radially-constrained configuration during insertion of the pump head into the left ventricle, the impeller is axially elongated such that the distal end of the impeller is disposed distally within the and the impeller-stabilizing spring is compressed in order to accommodate the movement of the distal end of the impeller. Typically, the impeller-stabilizing spring is disposed around the axial shaft between the distal end of the impeller and the thrust bearing.

There is therefore provided, in accordance with the present invention, an apparatus including:
a ventricular assist device including:

an impeller configured to be placed inside a left ventricle of a subject, the impeller defining a lumen therethrough;
a frame configured to be disposed around the impeller;
a proximal bearing disposed at a proximal end of the frame and a distal bearing disposed at a distal end of the frame;
an axial shaft passing through the proximal bearing, the lumen defined by the impeller, and the distal bearing; and
a motion-cushioning spring disposed around the axial shaft between a distal end of the impeller and the distal bearing, the motion-cushioning spring being configured to cushion axial motion that the impeller undergoes.

In some applications, the impeller is configured to undergo axial back-and-forth motion while the impeller is rotating, and the motion-cushioning spring is configured to provide cushioning to the axial back-and-forth motion.

In some applications, the impeller is configured to be radially constrained by becoming axially elongated and the motion-cushioning spring is configured to become compressed such as to accommodate the axial elongation of the impeller.

In some applications, the motion-cushioning spring is coupled to a distal end of the impeller.

In some applications, the ventricular assist device further includes a proximal motion-cushioning spring disposed around the axial shaft between a proximal end of the impeller and the proximal bearing, the motion-cushioning spring being configured to cushion axial motion that the impeller undergoes in a proximal direction.

In some applications, the motion-cushioning spring is coupled to the distal bearing. In some applications, the apparatus further includes a distal bearing housing disposed around the distal bearing, the motion-cushioning spring being coupled to the distal bearing via the distal bearing housing.

In some applications, the apparatus further includes an elastomeric material that is coupled to the motion-cushioning spring, such that at least a portion of the axial shaft between a distal end of the impeller and the distal bearing is covered by the elastomeric material. In some applications, the motion-cushioning spring is coated with the elastomeric material. In some applications, the motion-cushioning spring is embedded within the elastomeric material. In some applications, the elastomeric material includes at least one of silicone and polyurethane.

In some applications, the ventricular assist device includes a purging system that is configured to pump a purging fluid through a lumen defined by the axial shaft, such that at least a portion of the purging fluid flows proximally through an interface between the axial shaft and the elastomeric material.

In some applications, the elastomeric material is coupled to the motion-cushioning spring in such a manner that the elastomeric material changes shape to conform to shape changes that the motion-cushioning spring undergoes. In some applications, the elastomeric material is configured to undergo the changes in shape without the elastomeric material becoming broken or collapsing. In some applications, the elastomeric material is configured not to become creased as a result of the motion-cushioning spring being compressed.

In some applications, the ventricular assist device further includes a pump-outlet tube configured to traverse an aortic valve of the subject, such that a proximal portion of the pump-outlet tube is disposed within an aorta of the subject and a distal portion of the pump-outlet tube is disposed within the subject's left ventricle, the distal portion of the pump-outlet tube extending to the distal end of the frame and defining one or more lateral blood inlet openings that are configured to allow blood to flow from the subject's left ventricle into the pump-outlet tube.

In some applications, a porosity of the distal portion of the pump-outlet tube, which defines the blood-inlet openings, is lower within a proximal region of the distal portion of the pump-outlet tube than within a distal region of the distal portion of the pump-outlet tube that is distal to the proximal region. In some applications, the distal portion of the pump-outlet tube has a porosity of more than 40 percent. In some applications, the distal portion of the pump-outlet tube defines more than 10 blood-inlet openings that are sized such as (a) to allow blood to flow from the subject's left ventricle into the tube and (b) to block structures from the subject's left ventricle from entering into the frame. In some applications, the distal portion of the pump-outlet tube defines more than 50 blood-inlet openings that are sized such as (a) to allow blood to flow from the subject's left ventricle into the tube and (b) to block structures from the subject's left ventricle from entering into the frame.

In general, in the specification and in the claims of the present application, the term "proximal" and related terms, when used with reference to a device or a portion thereof, should be interpreted to mean an end of the device or the portion thereof that, when inserted into a subject's body, is typically closer to a location through which the device is inserted into the subject's body. The term "distal" and related terms, when used with reference to a device or a portion thereof, should be interpreted to mean an end of the device or the portion thereof that, when inserted into a subject's body, is typically further from the location through which the device is inserted into the subject's body.

The present invention will be more fully understood from the following detailed description of embodiments thereof, taken together with the drawings, in which:

**BRIEF DESCRIPTION OF THE DRAWINGS**

Figs. 1A, 1B, and 1C are schematic illustrations of a ventricular assist device, a distal end of which is configured to be placed in a subject's left ventricle, in accordance with some applications of the present invention;

Fig. 2 is a schematic illustration of a frame that houses an impeller of a ventricular assist device, in accordance with some applications of the present invention;

Figs. 3A, 3B, 3C, 3D, and 3E are schematic illustrations of an impeller of a ventricular assist device or portions thereof, in accordance with some applications of the present invention;

Fig. 4 is a schematic illustration of an impeller disposed inside a frame of a ventricular assist device, in accordance with some applications of the present invention;

Figs. 5A and 5B are schematic illustrations of the impeller and the frame of the ventricular assist device, respectively in non-radially-constrained and radially-constrained states thereof, in accordance with some applications of the present invention;

Fig. 5C is an enlarged schematic illustration of the proximal end of the frame of the ventricular assist device, in accordance with some applications of the present invention;

Figs. 6A and 6B are schematic illustrations of a ventricular assist device at respective stages of a motion cycle of the impeller of the ventricular assist device with respect to the frame of the ventricular assist device, in accordance with some applications of the present invention;

Figs. 6C and 6D are the only schematic illustrations of a ventricular assist device that includes a motion-cushioning spring, in accordance with the present invention:

Fig. 7A is a schematic illustration of a motor unit of a ventricular assist device, in accordance with some applications of the present invention;

Figs. 7B and 7C are schematic illustrations of a motor unit of a ventricular assist device, in accordance with some applications of the present invention;

Fig. 8A is a graph indicating variations in the length of a drive cable of a ventricular assist device as a pressure gradient against which the impeller of the blood pump varies, as measured in experiments performed in accordance with some applications of the present invention;

Figs. 8B and 8C are graphs indicating variations in magnetic phase measurements performed upon a blood pump as a pressure gradient against which the impeller of the blood pump varies, as measured in experiments performed in accordance with some applications of the present invention;

Figs. 9A and 9B are schematic illustrations of a ventricular assist device that includes one or more blood-pressure-measurement tubes and/or fibers, in accordance with some applications of the present invention;

Figs. 10A and 10B are schematic illustrations of a ventricular assist device that includes an inner lining on the inside of the frame that houses the impeller, in accordance with some applications of the present invention;

Figs. 11A, 11B, 11C, 11D, and 11E are schematic illustrations of a pump-outlet tube that defines blood-inlet openings at a distal end thereof, in accordance with some applications of the present invention;

Fig. 12A, 12B, and 12C are schematic illustrations of a drive cable of a ventricular assist device, in accordance with some applications of the present invention;

Fig. 12D is a schematic illustration of a drive cable bearing tube, in accordance with some applications of the present invention;

Fig. 13 is a schematic illustration of a ventricular assist device with a guidewire disposed within a guidewire lumen, in accordance with some applications of the present invention;

Figs. 14A and 14B are schematic illustrations of a two-state guidewire, in accordance with some applications of the present invention;

Fig. 15 is a schematic illustration of a ventricular assist device with a distal thrust bearing, in accordance with some applications of the present invention; and

Figs. 16A, 16B, 16C, and 16D are schematic illustrations of packaging for packaging a ventricular assist device, in accordance with some applications of the present invention.

## DETAILED DESCRIPTION OF EMBODIMENTS

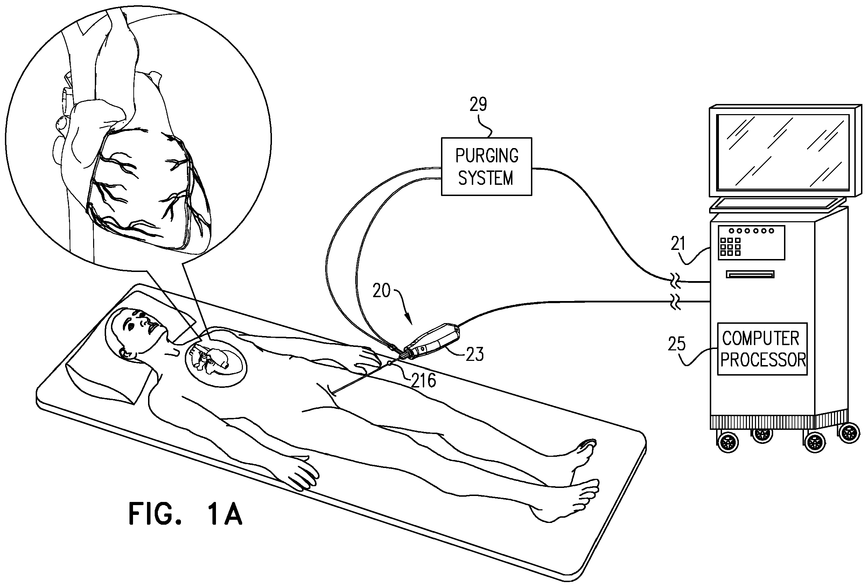

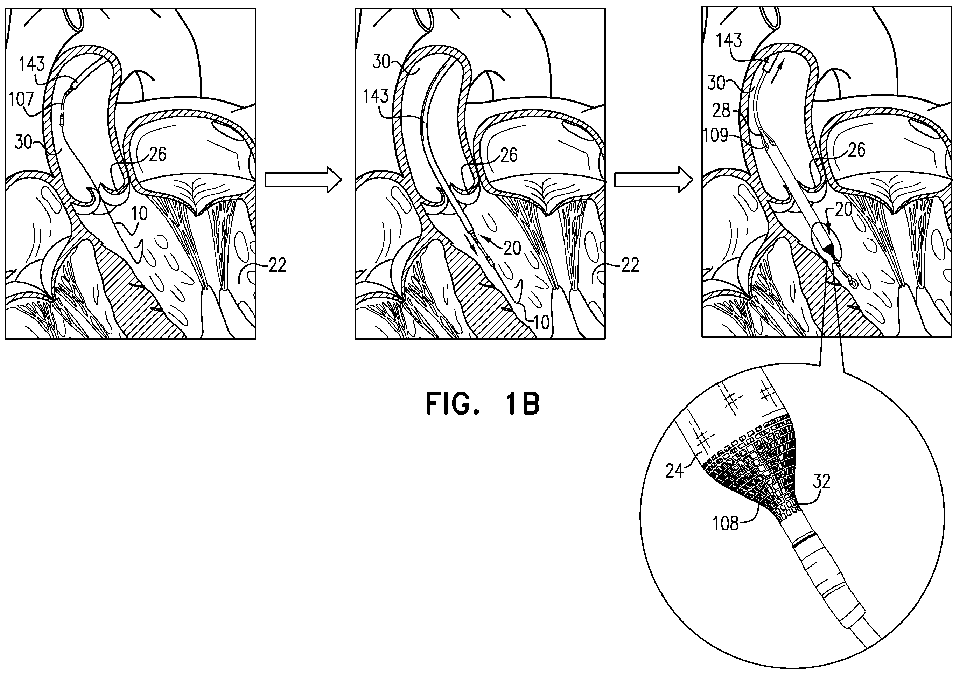

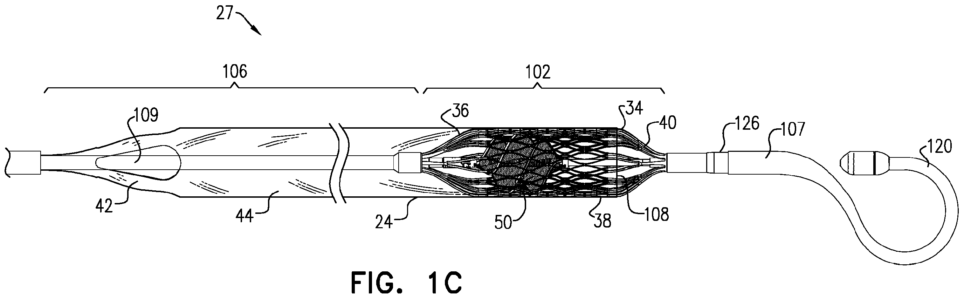

Reference is now made to Figs. 1A, 1B, and 1C, which are schematic illustrations of a ventricular assist device 20, a distal end of which is configured to be disposed in a subject's left ventricle 22, in accordance with some applications of the present invention. Fig. 1A shows an overview of the ventricular assist device system including a control console 21, and a motor unit 23. Fig. 1B shows the ventricular assist device being inserted into the subject's left ventricle, and Fig. 1C shows a pump-head portion 27 of the ventricular assist device in greater detail. The ventricular assist device includes a pump-outlet tube 24, which traverses an aortic valve 26 of the subject, such that a proximal end 28 of the pump-outlet tube is disposed in an aorta 30 of the subject and a distal end 32 of the pump-outlet tube is disposed within left ventricle 22. Pump-outlet tube 24 (which is sometimes referred to herein as a "blood-pump tube") is typically an elongate tube, an axial length of the pump-outlet tube typically being substantially larger than its diameter. The scope of the present invention includes using the apparatus and methods described herein in anatomical locations other than the left ventricle and the aorta. Therefore, the ventricular assist device and/or portions thereof are sometimes referred to herein (in the specification and the claims) as a blood pump.

For some applications, the ventricular assist device is used to assist the functioning of a subject's left ventricle during a percutaneous coronary intervention. In such cases, the ventricular assist device is typically used for a period of up to six hours (e.g., up to ten hours), during a period in which there is risk of developing hemodynamic instability (e.g., during or immediately following the percutaneous coronary intervention). Alternatively or additionally, the ventricular assist device is used to assist the functioning of a subject's left ventricle for a longer period (e.g., for example, 2-20 days, e.g., 4-14 days) upon a patient suffering from cardiogenic shock, which may include any low-cardiac-output state (e.g., acute myocardial infarction, myocarditis, cardiomyopathy, post-partum, etc.). For some applications, the ventricular assist device is used to assist the functioning of a subject's left ventricle for yet a longer period (e.g., several weeks or months), e.g., in a "bridge to recovery" treatment. For some such applications, the ventricular assist device is permanently or semi-permanently implanted, and the impeller of the ventricular assist device is powered transcutaneously, e.g., using an external antenna that is magnetically coupled to the impeller.

As shown in Fig. 1B, which shows steps in the deployment of the ventricular assist device in the left ventricle, typically the distal end of the ventricular assist device is guided to the left ventricle over a guidewire 10. During the insertion of the distal end of the device to the left ventricle, a delivery catheter 143 is disposed over the distal end of the device. Once the distal end of the device is disposed in the left ventricle, the delivery catheter is typically retracted to the aorta, and the guidewire is withdrawn from the subject's body. The retraction of the delivery catheter typically causes self-expandable components of the distal end of the device to assume non-radially-constrained configurations, as described in further detail hereinbelow. Typically, the ventricular assist device is inserted into the subject's body in order to provide an acute treatment to the subject. For some applications, in order to withdraw the left ventricular device from the subject's body at the end of the treatment, the delivery catheter is advanced over the distal end of the device, which causes the self-expandable components of the distal end of the device to assume radially-constrained configurations. Alternatively or additionally, the distal end of the device is retracted into the delivery catheter which causes the self-expandable components of the distal end of the device to assume radially-constrained configurations.

For some applications (not shown), the ventricular assist device and/or delivery catheter 143 includes an ultrasound transducer at its distal end and the ventricular assist device is advanced toward the subject's ventricle under ultrasound guidance.

Reference is made to Fig. 1C, which shows pump-head portion 27 of ventricular assist device 20, in accordance with some applications of the present invention, in greater detail. Typically, an impeller 50 is disposed within a distal portion 102 of pump-outlet tube 24 and is configured to pump blood from the left ventricle into the aorta by rotating. The pump-outlet tube typically defines one or more blood-inlet openings 108 at the distal end of the pump-outlet tube, via which blood flows into the pump-outlet tube from the left ventricle, during operation of the impeller. As shown in Fig. 1C, for some applications, the pump-outlet tube defines a single axially-facing blood-inlet opening. Alternatively, the pump-outlet tube defines a plurality of lateral blood-inlet openings (e.g., as shown in Fig. 1B), as described in further detail hereinbelow. For some applications, proximal portion 106 of the pump-outlet tube defines one or more blood-outlet openings 109, via which blood flows from the pump-outlet tube into the ascending aorta, during operation of the impeller.

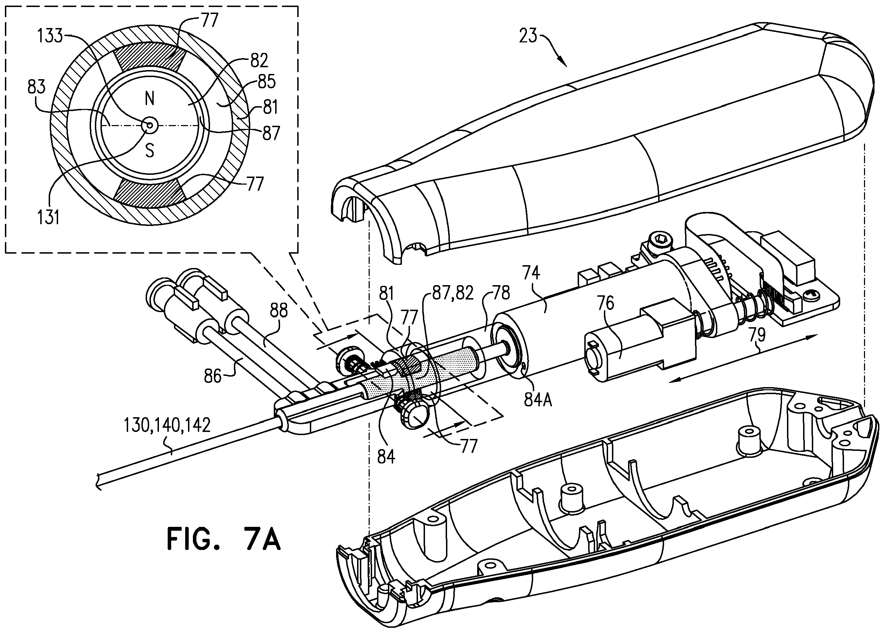

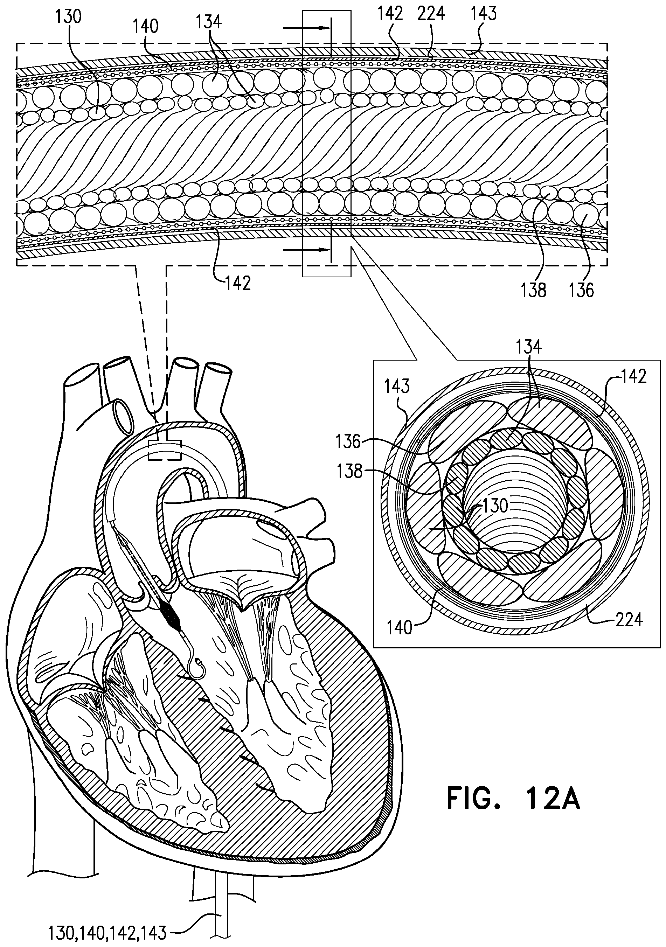

For some applications, control console 21 (shown in Fig. 1A), which typically includes a computer processor 25, drives the impeller to rotate. For example, the computer processor may control a motor 74 (shown in Fig. 7A), which is disposed within motor unit 23 (shown in Fig. 1A) and which drives the impeller to rotate via a drive cable 130 (shown in Fig. 12A). For some applications, the computer processor is configured to detect a physiological parameter of the subject (such as left-ventricular pressure, cardiac afterload, rate of change of left-ventricular pressure, etc.) and to control rotation of the impeller in response thereto, as described in further detail hereinbelow. Typically, the operations described herein that are performed by the computer processor, transform the physical state of a memory, which is a real physical article that is in communication with the computer processor, to have a different magnetic polarity, electrical charge, or the like, depending on the technology of the memory that is used. Computer processor 25 is typically a hardware device programmed with computer program instructions to produce a special-purpose computer. For example, when programmed to perform the techniques described herein, computer processor 25 typically acts as a special-purpose, ventricular-assist computer processor and/or a special-purpose, blood-pump computer processor.

For some applications, a purging system 29 (shown in Fig. 1A) drives a fluid (e.g., a glucose solution) to pass through portions of ventricular assist device 20, for example, in order to cool portions of the device, to purge and/or lubricate interfaces between rotating parts and stationary bearings, and/or in order to wash debris from portions of the device.

Typically, along distal portion 102 of pump-outlet tube 24, a frame 34 is disposed within the pump-outlet tube around impeller 50. The frame is typically made of a shape-memory alloy, such as nitinol. For some applications, the shape-memory alloy of the frame is shape set such that at least a portion of the frame (and thereby distal portion 102 of tube 24) assumes a generally circular, elliptical, or polygonal cross-sectional shape in the absence of any forces being applied to distal portion 102 of tube 24. By assuming its generally circular, elliptical, or polygonal cross-sectional shape, the frame is configured to hold the distal portion of the pump-outlet tube in an open state. Typically, during operation of the ventricular assist device, the distal portion of the pump-outlet tube is configured to be placed within the subject's body, such that the distal portion of the pump-outlet tube is disposed at least partially within the left ventricle.

For some applications, along proximal portion 106 of pump-outlet tube 24, the frame is not disposed within the pump-outlet tube, and the pump-outlet tube is therefore not supported in an open state by frame 34. Pump-outlet tube 24 is typically made of a blood-impermeable collapsible material. For example, pump-outlet tube 24 may include polyurethane, polyester, and/or silicone. Alternatively or additionally, the pump-outlet tube is made of polyethylene terephthalate (PET) and/or polyether block amide (e.g., PEBAX®). For some applications (not shown), the pump-outlet tube is reinforced with a reinforcement structure, e.g., a braided reinforcement structure, such as a braided nitinol tube. Typically, the proximal portion of the pump-outlet tube is configured to be placed such that it is at least partially disposed within the subject's ascending aorta. For some applications, the proximal portion of the pump-outlet tube traverses the subject's aortic valve, passing from the subject's left ventricle into the subject's ascending aorta, as shown in Fig. 1B.

As described hereinabove, the pump-outlet tube typically defines one or more blood-inlet openings 108 at the distal end of the pump-outlet tube, via which blood flows into the pump-outlet tube from the left ventricle, during operation of the impeller. For some applications, the proximal portion of the pump-outlet tube defines one or more blood-outlet openings 109, via which blood flows from the pump-outlet tube into the ascending aorta, during operation of the impeller. Typically, the pump-outlet tube defines a plurality of blood-outlet openings 109, for example, between two and eight blood-outlet openings (e.g., between two and four blood-outlet openings). During operation of the impeller, the pressure of the blood flow through the pump-outlet tube typically maintains the proximal portion of the tube in an open state. For some applications, in the event that, for example, the impeller malfunctions, the proximal portion of the pump-outlet tube is configured to collapse inwardly, in response to pressure outside of the proximal portion of the pump-outlet tube exceeding pressure inside the proximal portion of the pump-outlet tube. In this manner, the proximal portion of the pump-outlet tube acts as a safety valve, preventing retrograde blood flow into the left ventricle from the aorta.

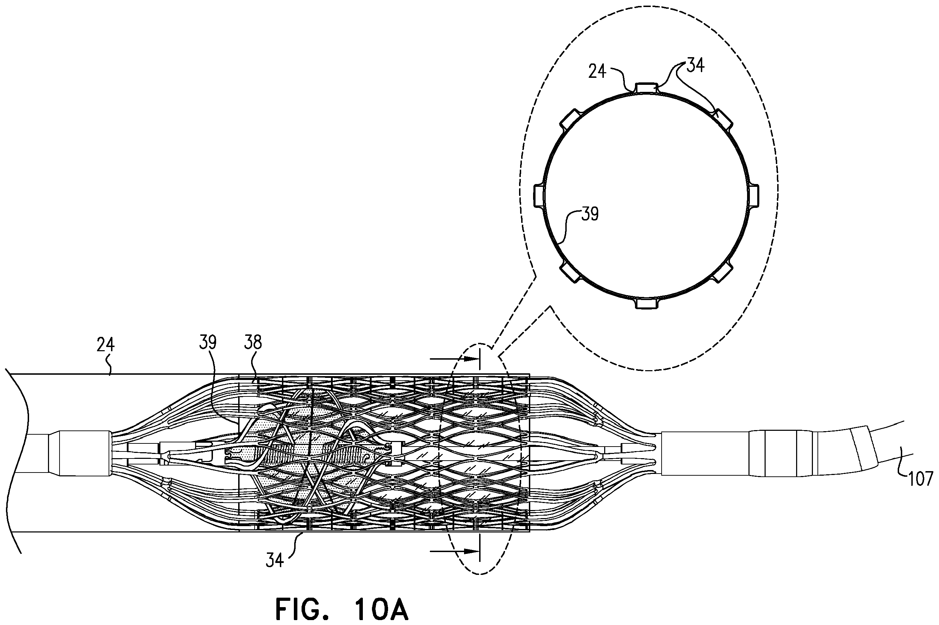

Referring again to Fig. 1C, for some applications, frame 34 is shaped such that the frame defines a proximal conical portion 36, a central cylindrical portion 38, and a distal conical portion 40. Typically, the proximal conical portion is proximally-facing, i.e., facing such that the narrow end of the cone is proximal with respect to the wide end of the cone. Further typically, the distal conical portion is distally-facing, i.e., facing such that the narrow end of the cone is distal with respect to the wide end of the cone. For some applications, within at least a portion of frame 34 (e.g., along all of, or a portion of, the central cylindrical portion of the frame), an inner lining 39 lines the frame. Fig. 1C shows an embodiment of the pump-head portion without inner lining 39, but several figures show embodiments of a pump-head portion that includes inner lining 39. In accordance with respective applications, the inner lining partially overlaps or fully overlaps with pump-outlet tube 24 over the portion of the frame that the inner lining lines, as described in further detail hereinbelow with reference to Figs. 10A-B.

Typically, pump-outlet tube 24 includes a conical proximal portion 42 and a cylindrical central portion 44. The proximal conical portion is typically proximally-facing, i.e., facing such that the narrow end of the cone is proximal with respect to the wide end of the cone. Typically, blood-outlet openings 109 are defined by pump-outlet tube 24, such that the openings extend at least partially along the proximal conical section of tube 24. For some such applications, the blood-outlet openings are teardrop-shaped, as shown in Fig. 1C. Typically, the teardrop-shaped nature of the blood-outlet openings in combination with the openings extending at least partially along the proximal conical section of tube 24 causes blood to flow out of the blood-outlet openings along flow lines that are substantially parallel with the longitudinal axis of tube 24 at the location of the blood-outlet openings.

For some applications (not shown), the diameter of pump-outlet tube 24 changes along the length of the central portion of the pump-outlet tube, such that the central portion of the pump-outlet tube has a frustoconical shape. For example, the central portion of the pump-outlet tube may widen from its proximal end to is distal end, or may narrow from its proximal end to its distal end. For some applications, at its proximal end, the central portion of the pump-outlet tube has a diameter of between 5 and 7mm, and at its distal end, the central portion of the pump-outlet tube has a diameter of between 8 and 12 mm.

Again referring to Fig. 1C, the ventricular assist device typically includes a distal-tip element 107 that is disposed distally with respect to frame 34 and that includes an axial-shaft-receiving tube 126 and a distal-tip portion 120. Typically, the axial-shaft receiving tube is configured to receive a distal portion of an axial shaft 92 of the pump-head portion during axial back-and-forth motion of the axial shaft (as described in further detail hereinbelow), and/or during delivery of the ventricular assist device. (Typically, during delivery of the ventricular assist device, the frame is maintained in a radially-constrained configuration, which typically causes the axial shaft to be disposed in a different position with respect to the frame relative to its disposition with respect to the frame during operation of the ventricular assist device). Typically, distal-tip portion 120 is configured to assume a curved shape upon being deployed within the subject's left ventricle, e.g., as shown in Fig. 1C. For some applications, the curvature of the distal-tip portion is configured to provide an atraumatic tip to ventricular assist device 20. Alternatively or additionally, the distal-tip portion is configured to space blood-inlet openings 108 of the ventricular assist device from walls of the left ventricle.

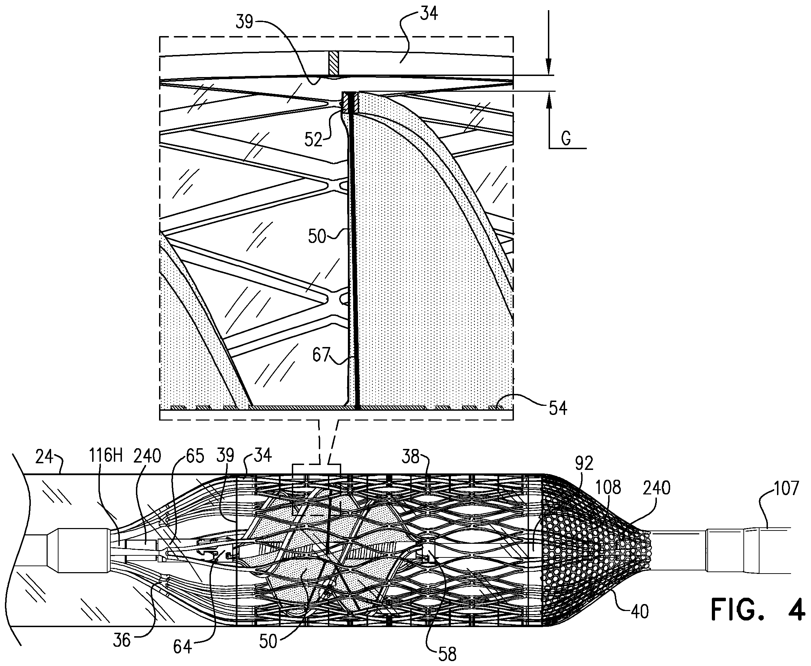

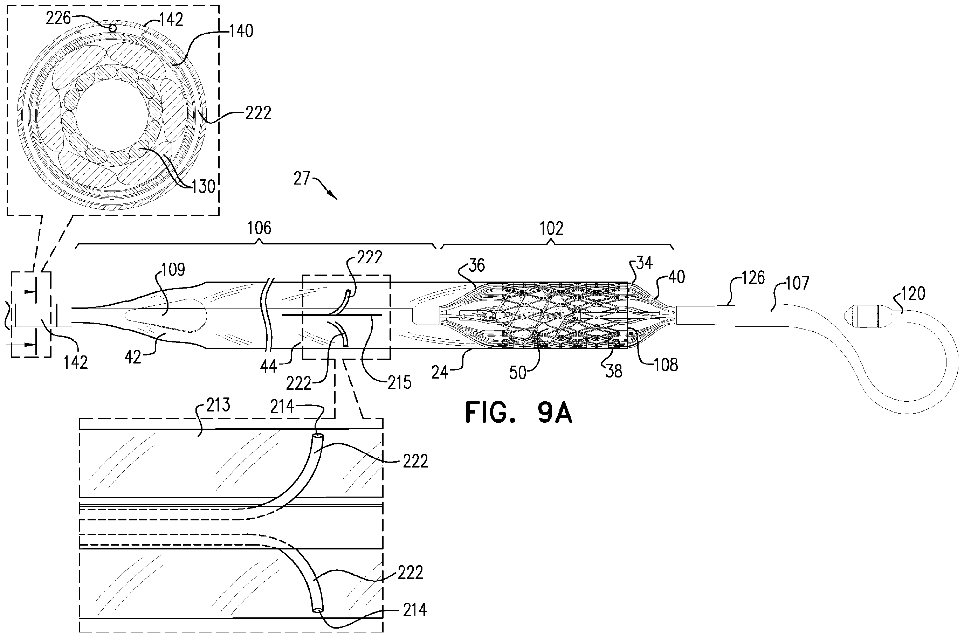

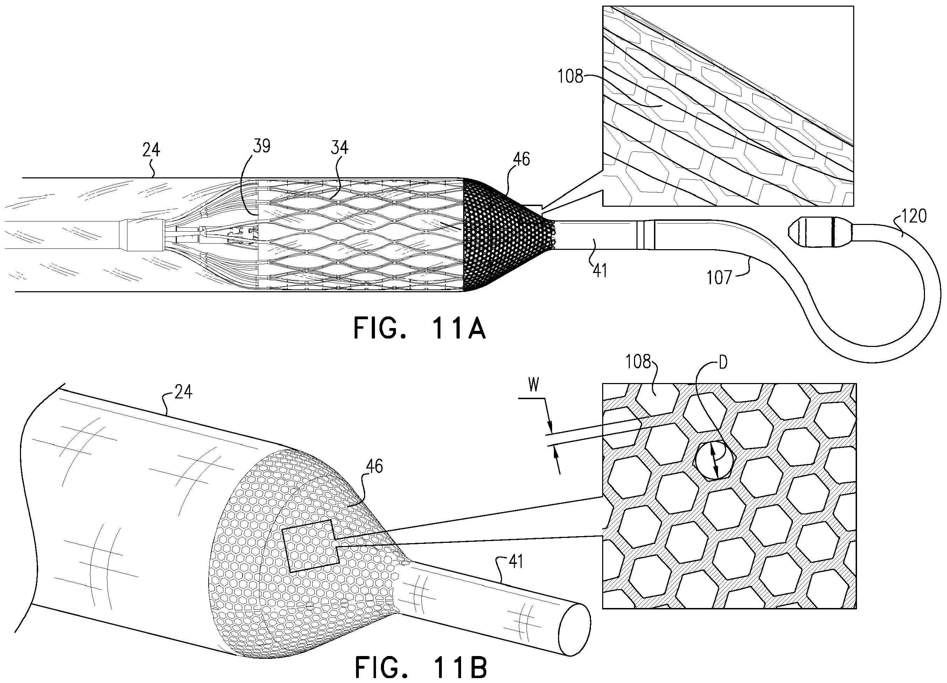

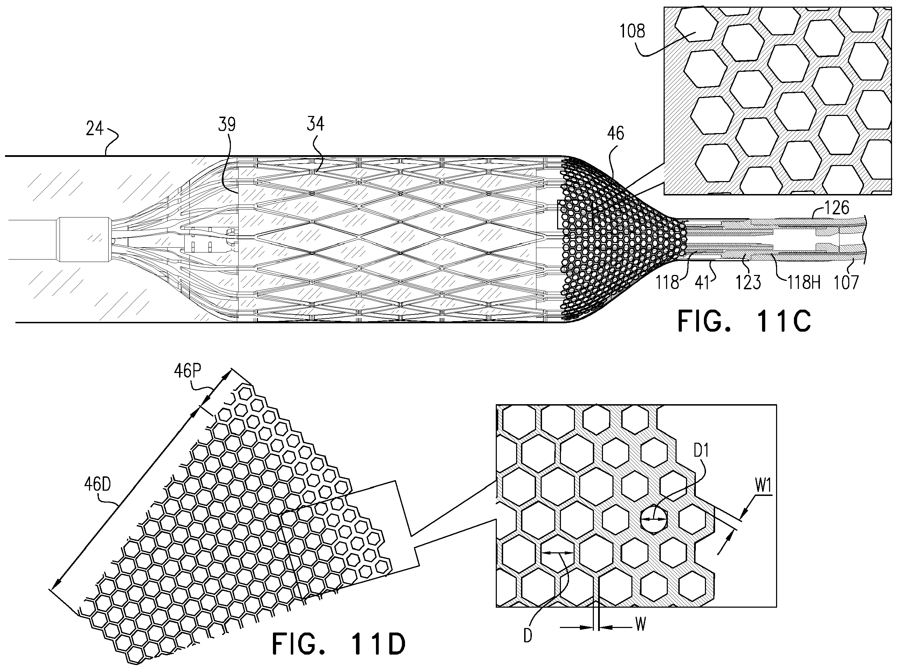

As shown in the enlarged portion of Fig. 1B, for some applications, pump-outlet tube 24 extends to the end of distal conical portion 40 of the frame, and the pump-outlet tube defines a plurality of lateral blood-inlet openings 108, as described in further detail hereinbelow. For such applications, the pump-outlet tube typically defines a distal conical portion that is distally facing, i.e., such that the narrow end of the cone is distal with respect to the wide end of the cone. For some such applications (not shown), the pump-outlet tube defines two to four lateral blood-inlet openings (e.g., four lateral blood-inlet openings, as shown). Typically, for such applications, each of the blood-inlet openings defines an area of more than 20 square mm (e.g., more than 30 square mm), and/or less than 60 square mm (e.g., less than 50 square mm), e.g., 20-60 square mm, or 30-50 square mm. Alternatively or additionally, the outlet tube defines a greater number of smaller lateral blood-inlet openings, e.g., more than 10 blood-inlet openings, more than 50 blood-inlet openings, more than 200 blood-inlet openings, or more than 400 blood-inlet openings, e.g., 50-100 blood-inlet openings, 100-400 blood-inlet openings, or 400-600 blood-inlet openings. For some such applications, each of the blood-inlet openings defines an area of more than 0.05 square mm (e.g., more than 0.1 square mm), and/or less than 3 square mm (e.g., less than 1 square mm), e.g., 0.05-3 square mm, or 0.1-1 square mm. Alternatively, each of the blood-inlet openings defines an area of more than 0.1 square mm (e.g., more than 0.3 square mm), and/or less than 5 square mm (e.g., less than 1 square mm), e.g., 0.1-5 square mm, or 0.3-1 square mm. Such applications are described in further detail hereinbelow, for example, with reference to Figs. 11A-E. In general, the scope of the present disclosure includes combining a pump-outlet-tube that defines a single axially-facing blood-inlet-opening 108 as shown in Fig. 1C (and as also shown in Figs. 9A-B, 10A-B, and 16A-C), or a pump-outlet-tube that defines a plurality of laterally-facing blood-inlet-openings 108 as shown in Fig. 1B (and as also shown in Figs. 4, 5A-B, 6A-D, 11A-E, and 13) in combination with other features of the ventricular assist device that are described herein, *mutatis mutandis.*

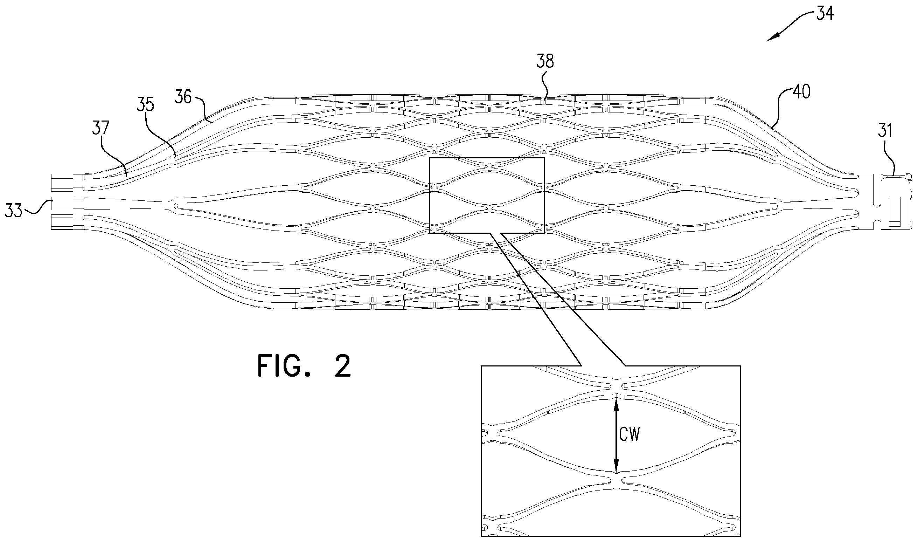

Reference is now made to Fig. 2, which is schematic illustration of frame 34 that houses an impeller of ventricular assist device 20, in accordance with some applications of the present invention. Frame 34 is typically made of a shape-memory alloy, such as nitinol, and the shape-memory alloy of the frame is shape set such that the central portion of the frame (and thereby tube 24) assumes a generally circular, elliptical, or polygonal cross-sectional shape in the absence of any forces being applied to pump-outlet tube 24. By assuming its generally circular, elliptical, or polygonal cross-sectional shape, the frame is configured to hold the distal portion of the tube in an open state.

Typically, the frame is a stent-like frame, in that it comprises struts that, in turn, define cells. Further typically, the frame is covered with pump-outlet tube 24, and/or covered with an inner lining 39, described hereinbelow, with reference to Figs. 10A-B. As described hereinbelow, for some applications impeller 50 undergoes axial back-and-forth motion with respect to frame 34. Typically, over the course of the motion of the impeller with respect to the frame the location of the portion of the impeller that defines the maximum span of the impeller is disposed within central cylindrical portion 38 of frame 34. In some cases, if the cells of the central cylindrical portion 38 of frame 34 are too large, then pump-outlet tube 24, and/or inner lining 39 gets stretched between edges of the cells, such that the pump-outlet tube 24, and/or inner lining 39 does not define a circular cross-section. For some applications, if this occurs in the region in which the portion of the impeller that defines the maximum span of the impeller is disposed, this results in a substantially non-constant gap between the edges of the impeller blades and tube 24 (and/or inner lining) at that location, over the course of a rotation cycle of the impeller. For some applications, this may lead to increased hemolysis relative to if there were a substantially constant gap between the edges of the impeller blades and tube 24 (and/or inner lining) at that location, over the course of the rotation cycle of the impeller.

Referring to Fig. 2, at least partially in view of the issues described in the above paragraph, within central cylindrical portion 38 of frame 34, the frame defines a large number of relatively small cells. Typically, when the frame is disposed in its non-radially-constrained configuration, the maximum cell width CW of the each of the cells (i.e., the distance from the inner edge of the strut at the central junction on one side of the cell to the inner edge of the strut at the central junction on the other side of the cell, as measured around the circumference of cylindrical portion 38) within the cylindrical portion of the frame is less than 2 mm, e.g., between 1.4 mm and 1.6 mm, or between 1.6 and 1.8 mm. Since the cells are relatively small, inner lining 39 defines a substantially circular cross-section within the cylindrical portion of the frame.

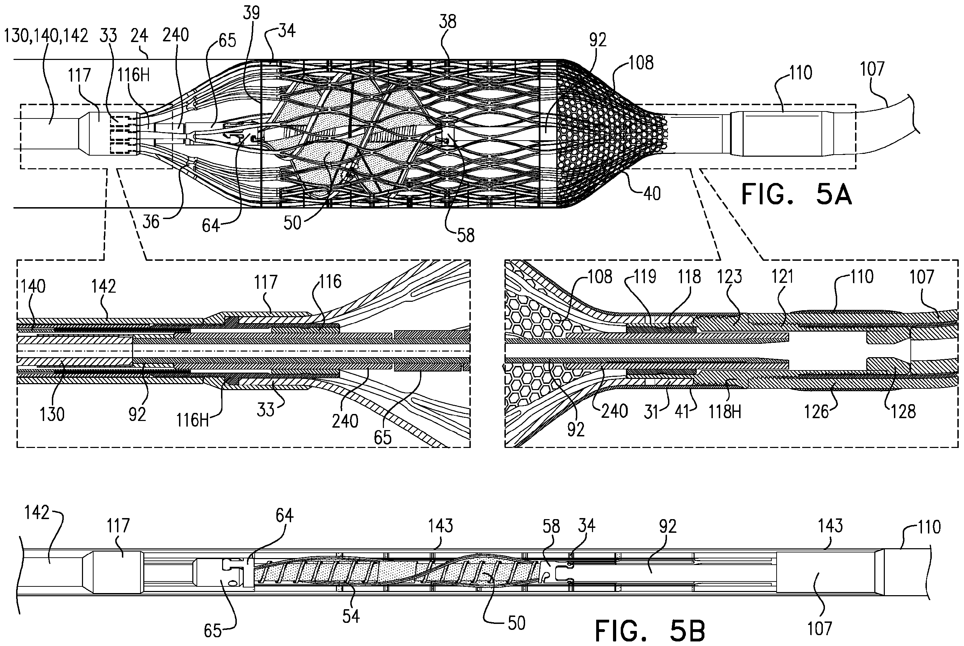

Still referring to Fig. 2, and starting from the distal end of the frame (which is to the right of the figure), typically the frame defines the following portions (a) coupling portion 31 via which the frame is coupled to a distal bearing housing 118H (shown in Fig. 5A) of the ventricular assist device, (b) distal conical portion 40, (c) central cylindrical portion 38, (d) proximal conical portion 36, and (e) proximal strut junctions 33. As illustrated, as the frame transitions from a proximal end of the frame toward the center of the frame (e.g., as the frame transitions from proximal strut junctions 33, through proximal conical portion 36, and to central cylindrical portion 38), struts 37 of the frame pass through junctions 35, at which the two struts branch from a single strut, in a Y-shape. As described in further detail hereinbelow, typically frame 34 is placed in a radially-constrained (i.e., crimped) configuration within delivery catheter 143 by the frame being axially elongated. Moreover, the frame typically transmits its radial narrowing to the impeller, and the impeller becomes radially constrained by becoming axially elongated within the frame. For some applications, the struts of the frame being configured in the manner described above facilitates transmission of axial elongation from the delivery catheter (or other device that is configured to crimp the frame) to the frame, which in turn facilitates transmission of axial elongation to the impeller. This is because the pairs of struts that branch from each of junctions 35 are configured to pivot about the junction and move closer to each other such as to close.

Still referring to Fig. 2, during the assembly of the ventricular assist device, initially distal coupling portion 31 is coupled to a distal bearing housing 118H (shown in Fig. 5A), e.g., via a snap-fit mechanism. For some applications proximal strut junctions 33 are still maintained in open states at this stage, in order for the impeller to be placed within the frame via the proximal end of the frame. Typically, the structure of frame 34 shown in Fig. 2 is used in applications in which pump-outlet tube extends to the distal end of frame 34 (e.g., as shown in Fig. 1B). In such cases, the impeller cannot be inserted via the distal end of the frame, since the distal end of the frame is covered by pump-outlet tube 24. During the assembly of the ventricular assist device, subsequent to the impeller being inserted via the proximal end of the frame, the proximal strut junctions are closed. For some applications, the proximal strut junctions are closed around the outside of a proximal bearing housing 116H (shown in Fig. 5A), as described in further detail hereinbelow with reference to Figs. 5A-B. Typically, a securing element 117 (e.g., a ring shown in Fig. 5A) holds the strut junctions in their closed configurations around the outside of proximal bearing housing 116H.

Typically, when disposed in its non-radially constrained configuration, frame 34 has a total length of more than 25 mm (e.g., more than 30 mm), and/or less than 50 mm (e.g., less than 45 mm), e.g., 25-50 mm, or 30-45 mm. Typically, when disposed in its radially-constrained configuration (within delivery catheter 143), the length of the frame increases by between 2 and 5 mm. Typically, when disposed in its non-radially constrained configuration, the central cylindrical portion of frame 34 has a length of more than 10 mm (e.g., more than 12 mm), and/or less than 25 mm (e.g., less than 20 mm), e.g., 10-25 mm, or 12-20 mm. For some applications, a ratio of the length of the central cylindrical portion of the frame to the total length of the frame is more than 1:4 and/or less than 1:2, e.g., between 1:4 and 1:2.

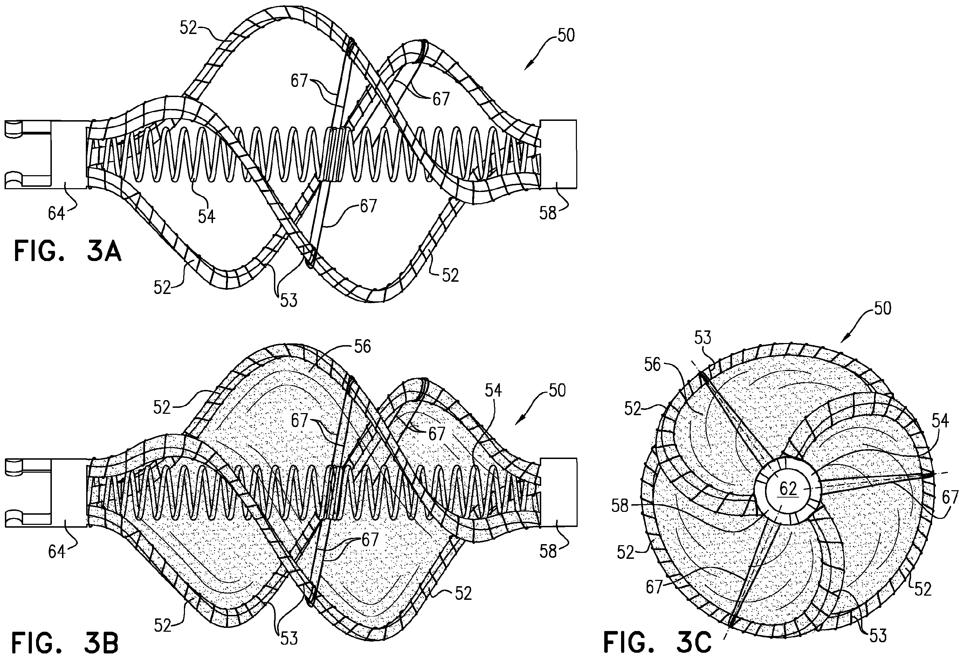

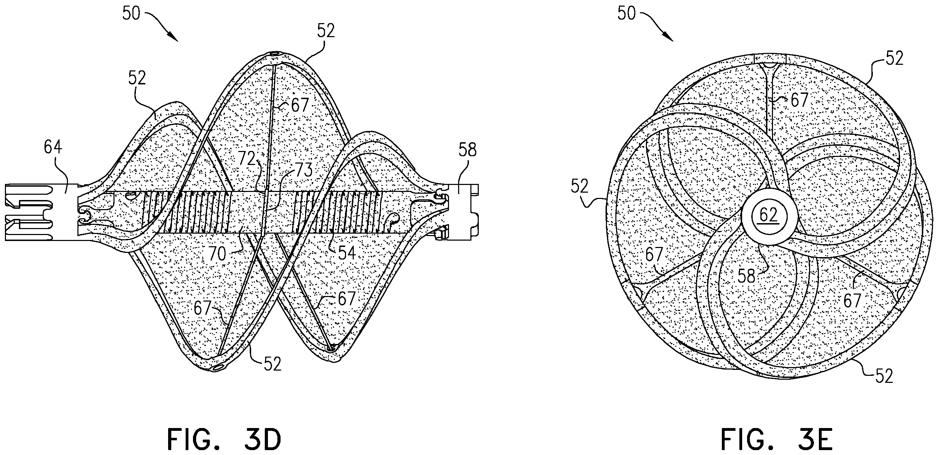

Reference is now made to Figs. 3A-E, which are schematic illustrations of impeller 50 or portions thereof, in accordance with some applications of the present invention. Typically, the impeller includes at least one outer helical elongate element 52, which winds around a central axial spring 54, such that the helix defined by the helical elongate element is coaxial with the central axial spring. Typically, the impeller includes two or more helical elongate elements (e.g., three helical elongate elements, as shown in Figs. 3A-C). For some applications, the helical elongate elements and the central axial spring are made of a shape-memory material, e.g., a shape-memory alloy, such as nitinol. Typically, each of the helical elongate elements and the central axial spring support a film 56 of a material (e.g., an elastomer, such as polyurethane, and/or silicone) therebetween. For some applications, the film of material includes pieces of nitinol embedded therein, for example in order to strengthen the film of material. For illustrative purposes, the impeller is shown in the absence of the material in Fig. 3A. Figs. 3B and 3C show respective views of the impeller with the material supported between the helical elongate elements and the spring. Figs. 3D and 3E show similar respective views of the impeller to those shown in Figs. 3B and 3C, but with certain features of the impeller differing from those shown in Figs. 3B and 3C, as elaborated upon hereinbelow.

Each of the helical elongate elements, together with the film extending from the helical elongate element to the spring, defines a respective impeller blade, with the helical elongate elements defining the outer edges of the blades, and the axial spring defining the axis of the impeller. Typically, the film of material extends along and coats the spring. For some applications, sutures 53 (e.g., polyester sutures, shown in Figs. 3A-C) are wound around the helical elongate elements. Typically, the sutures are configured to facilitate bonding between the film of material (which is typically an elastomer, such as polyurethane, or silicone) and the helical elongate element (which is typically a shape-memory alloy, such as nitinol). For some applications, sutures (e.g., polyester sutures, not shown) are wound around spring 54. Typically, the sutures are configured to facilitate bonding between the film of material (which is typically an elastomer, such as polyurethane, or silicone) and the spring (which is typically a shape-memory alloy, such as nitinol).

Typically, proximal ends of spring 54 and helical elongate elements 52 extend from a proximal bushing (i.e., sleeve bearing) 64 of the impeller, such that the proximal ends of spring 54 and helical elongate elements 52 are disposed at a similar radial distance from the longitudinal axis of the impeller, as each other. Similarly, typically, distal ends of spring 54 and helical elongate elements 52 extend from a distal bushing 58 of the impeller, such that the distal ends of spring 54 and helical elongate elements 52 are disposed at a similar radial distance from the longitudinal axis of the impeller, as each other. The helical elongate elements typically rise gradually from the proximal bushing before reaching a maximum span and then falling gradually toward the distal bushing. Typically, the helical elongate elements are symmetrical along their lengths, such that the rising portions of their lengths are symmetrical with respect to the falling portions of their lengths. Typically, the impeller defines a lumen 62 therethrough (shown in Fig. 3C), with the lumen typically extending through, and being defined by, spring 54, as well as proximal bushing 64 and distal bushing 58, of the impeller.

Reference is now made to Fig. 4, which is a schematic illustration of impeller 50 disposed inside frame 34 of ventricular assist device 20, in accordance with some applications of the present invention. For some applications, within at least a portion of frame 34 (e.g., along all of, or a portion of, central cylindrical portion 38 of the frame), inner lining 39 lines the frame. In accordance with respective applications, the inner lining partially overlaps or fully overlaps with pump-outlet tube 24 over the portion of the frame that the inner lining lines , as described in further detail hereinbelow with reference to Figs. 9A-B.

As shown in Fig. 4, typically there is a gap G, between the outer edge of impeller 50 and inner lining 39, even at a location at which the span of the impeller is at its maximum. For some applications, it is desirable that the gap between the outer edge of the blade of the impeller and inner lining 39 be relatively small, in order for the impeller to efficiently pump blood from the subject's left ventricle into the subject's aorta. (It is noted that, by virtue of the relatively small gap between the outer edge of impeller 50 and inner lining 39 even at a location at which the span of the impeller is at its maximum, as well as the shape of the impeller, the impeller functions as an axial-flow impeller, with the impeller pumping blood in the axial direction from a distal end of pump-outlet tube 24 to the proximal end of the pump-outlet tube.) It is also desirable that a gap between the outer edge of the blade of the impeller and the inner surface of frame 34 be maintained throughout the rotation of the impeller within frame 34, for example, in order to reduce the risk of hemolysis.

For some applications, when impeller 50 and frame 34 are both disposed in non-radially-constrained configurations and prior to operation of the impeller, gap G between the outer edge of the impeller and the inner lining 39, at the location at which the span of the impeller is at its maximum, is greater than 0.05 mm (e.g., greater than 0.1 mm), and/or less than 1 mm (e.g., less than 0.4 mm), e.g., 0.05-1 mm, or 0.1-0.4 mm. For some applications, when the impeller is disposed in its non-radially-constrained configurations and prior to operation of the impeller, the outer diameter of the impeller at the location at which the outer diameter of the impeller is at its maximum is more than 7 mm (e.g., more than 8 mm), and/or less than 10 mm (e.g., less than 9 mm), e.g., 7-10 mm, or 8-9 mm. For some applications, when frame 34 is disposed in its non-radially-constrained configuration, the inner diameter of frame 34 (as measured from the inside of inner lining 39 on one side of the frame to the inside of inner lining on the opposite side of the frame) is greater than 7.5 mm (e.g., greater than 8.5 mm), and/or less than 10.5 mm (e.g., less than 9.5 mm), e.g., 7.5-10.5 mm, or 8.5-9.5 mm. For some applications, when the frame is disposed in its non-radially-constrained configuration, the outer diameter of frame 34 is greater than 8 mm (e.g., greater than 9 mm), and/or less than 13 mm (e.g., less than 12 mm), e.g., 8-13 mm, or 9-12 mm.

Typically, an axial shaft 92 passes through the axis of impeller 50, via lumen 62 of the impeller. Further typically, the axial shaft is rigid, e.g., a rigid tube. For some applications, proximal bushing 64 of the impeller is coupled to the shaft such that the axial position of the proximal bushing with respect to the shaft is fixed, and distal bushing 58 of the impeller is slidable with respect to the shaft. For example, the proximal bushing may be coupled to a coupling element 65 disposed on the axial shaft (shown in Fig. 4), for example via a snap-fit mechanism. (Alternatively, distal bushing 58 of the impeller is coupled to the shaft such that the axial position of the distal bushing with respect to the shaft is fixed, and proximal bushing 64 of the impeller is slidable with respect to the shaft.) The axial shaft itself is radially stabilized via a proximal radial bearing 116 and a distal radial bearing 118. In turn, the axial shaft, by passing through lumen 62 defined by the impeller, radially stabilizes the impeller with respect to the inner surface of frame 34, such that even a relatively small gap between the outer edge of the blade of the impeller and the inner surface of frame 34 (e.g., a gap that is as described above) is maintained, during rotation of the impeller.

Referring again to Figs. 3A-C, for some applications, the impeller includes a plurality of elongate elements 67 extending radially from central axial spring 54 to outer helical elongate elements 52. The elongate elements are typically flexible but are substantially non-stretchable along the axis defined by the elongate elements. Further typically, each of the elongate elements is configured not to exert force upon the helical elongate element, unless force is acting upon the impeller that is causing the helical elongate element to move radially outward, such that (in the absence of the elongate element) a separation between the helical elongate element and the central axial spring would be greater than a length of the elongate element. For example, the elongate elements may include strings (such as polyester, and/or another polymer or a natural material that contains fibers) and/or wires (such as nitinol wires, and/or wires made of a different alloy, or a metal).

For some applications, the elongate elements 67 maintain helical elongate element 52 (which defines the outer edge of the impeller blade) within a given distance with respect to the central axial spring. In this manner, the elongate elements are configured to prevent the outer edge of the impeller from being forced radially outward due to forces exerted upon the impeller during the rotation of the impeller. The elongate elements are thereby configured to maintain the gap between the outer edge of the blade of the impeller and the inner surface of frame 34, during rotation of the impeller. Typically, more than one (e.g., more than two) and/or fewer than eight (e.g., fewer than four) elongate elements 67 are used in the impeller, with each of the elongate elements typically being doubled (i.e., extending radially from central axial spring 54 to an outer helical elongate element 52, and then returning from the helical elongate element back to the central axial spring). For some applications, a plurality of elongate elements, each of which extends from the spring to a respective helical elongate element and back to the spring, are formed from a single piece of string or a single wire.

Reference is now made to Figs. 3D and 3E, which are schematic illustrations of impeller 50, the impeller including a single integrated impeller-overexpansion-prevention element 72 that defines a plurality of elongate elements 67, in accordance with some applications of the present invention. For some applications, impeller-overexpansion-prevention element 72 (which defines a plurality of elongate elements 67) is used as an alternative to elongate elements 67 as shown in Figs. 3A-C. For some applications, element 72 defines a ring 73 and the plurality of elongate elements 67 extending radially from the ring. For some applications, rather than threading strings and/or wire around spring 54, ring 73 of element 72 is placed around the spring, e.g., by being placed around tube 70, which is typically disposed at the longitudinally-central location of the spring. The ends of respective elongate elements 67 are then coupled to respective helical elongate elements 52. As described hereinabove, elongate elements 67 are typically flexible but are substantially non-stretchable along the axis defined by the elongate elements. Further typically, each of elongate elements 67 is configured to substantially not resist compression. Rather, each elongate element 67 is configured to exert a tensile force upon helical elongate element 52 that prevents helical elongate element 52 from moving radially outward, such that (in the absence of elongate element 67) a separation between helical elongate element 52 and central axial spring 54 would be greater than a length of elongate element 67. When a force is acting upon the impeller that would cause the helical elongate element 52 to move radially outward (in the absence of elongate element 67), the impeller-overexpansion-prevention element is configured to prevent radial expansion of the impeller. Typically, a respective elongate element 67 is disposed within each one of the impeller blades and is configured to prevent the impeller blade from radially expanding. For some applications, element 72 is made of polyester, and/or another polymer or a natural material that contains fibers, and/or nitinol (or a similar shape-memory alloy).

It is noted that the scope of the present application includes using single integrated impeller-overexpansion-prevention element 72 with an impeller having a different construction from that shown in Figs. 3D-E. For example, the single integrated impeller-overexpansion-prevention element 72 could be used with an impeller having a differently constructed axial structure than spring 54. Typically, the axial structure defines a lumen therethrough, such that the impeller defines lumen 62 therethrough.

For some applications, the material from which the film is made is an elastomer having an ultimate elongation of more than 300 percent, e.g., more than 400 percent. Typically, the material has a relatively low molecular weight. For some applications, the material has a melt flow index (which is an indirect measure of molecular weight) of at least 4, e.g., at least 4.3. For some applications, the material has an ultimate tensile strength of more than 6000 psi, e.g., more than 7000 psi, or more than 7500 psi. For some applications, the material is a polycarbonate-based thermoplastic polyurethane, e.g., a Carbothane™. For some applications, Aromatic Carbothane™ (e.g., Aromatic Carbothane™ 75A) is used. Typically, such materials combine one or more of the following properties: no outer diameter loss caused during the dip process, resistance to fatigue, resistance to becoming misshaped by being crimped, and low outer diameter loss during crimping. Subsequently, the material is cured such that it solidifies, e.g., by being left to dry.

Typically, impeller 50 is inserted into the left ventricle transcatheterally, while impeller 50 is in a radially-constrained configuration. In the radially-constrained configuration, both helical elongate elements 52 and central axial spring 54 become axially elongated, and radially constrained. Typically film 56 of the material (e.g., silicone and/or polyurethane) changes shape to conform to the shape changes of the helical elongate elements and the axial support spring, both of which support the film of material. Typically, using a spring to support the inner edge of the film allows the film to change shape without the film becoming broken or collapsing, due to the spring providing a large surface area to which the inner edge of the film bonds. For some applications, using a spring to support the inner edge of the film reduces a diameter to which the impeller can be radially constrained, relative to if, for example, a rigid shaft were to be used to support the inner edge of the film, since the diameter of the spring itself can be reduced by axially elongating the spring.

As described hereinabove, for some applications, proximal bushing 64 of impeller 50 is coupled to axial shaft 92 such that the axial position of the proximal bushing with respect to the shaft is fixed, and distal bushing 58 of the impeller is slidable with respect to the shaft. For example, the proximal bushing may be coupled to coupling element 65 disposed on the axial shaft (shown in Fig. 4), for example via a snap-fit mechanism. For some applications, when the impeller is radially constrained for the purpose of inserting the impeller into the ventricle or for the purpose of withdrawing the impeller from the subject's body, the impeller axially elongates by the distal bushing sliding along the axial shaft distally. Alternatively (not shown), distal bushing 58 of the impeller is coupled to the shaft such that the axial position of the distal bushing with respect to the shaft is fixed, and proximal bushing 64 of the impeller is slidable with respect to the shaft. For some such applications, when the impeller is radially constrained for the purpose of inserting the impeller into the ventricle or for the purpose of withdrawing the impeller from the subject's body, the impeller axially elongates by the proximal bushing sliding along the axial shaft proximally. Subsequent to being released inside the subject's body, the impeller assumes its non-radially-constrained configuration (in which the impeller is typically disposed during operation of the impeller), which is as shown in Figs. 3A-E.

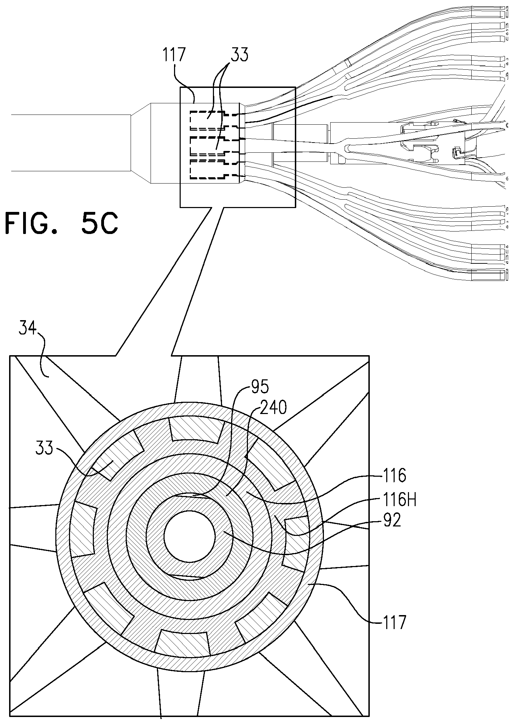

Reference is now made to Figs. 5A and 5B, which are schematic illustrations of impeller 50 and frame 34 of ventricular assist device 20, respectively in non-radially-constrained and radially-constrained states thereof, in accordance with some applications of the present invention. The impeller and the frame are typically disposed in the radially-constrained states during the transcatheteral insertion of the impeller and the frame into the subject's body, and are disposed in the non-radially-constrained states during operation of the impeller inside the subject's left ventricle. Reference is also made to Fig. 5C, which is an enlarged schematic illustration of the proximal end of the frame of the ventricular assist device, in accordance with some applications of the present invention.

As indicated in Fig. 5B, the frame and the impeller are typically maintained in radially-constrained configurations by delivery catheter 143. Typically, in the radially-constrained configuration of the impeller, the impeller has a total length of more than 15 mm (e.g., more than 20 mm), and/or less than 30 mm (e.g., less than 25 mm), e.g., 15-30 mm, or 20-25 mm. Further typically, in the non-radially constrained configuration of the impeller, the impeller has a length of more than 8 mm (e.g., more than 10 mm), and/or less than 18 mm (e.g., less than 15 mm), e.g., 8-18 mm, or 10-15 mm. Still further typically, when the impeller and frame 34 are disposed in radially-constrained configurations (as shown in Fig. 5B), the impeller has an outer diameter of less than 2 mm (e.g., less than 1.6 mm) and the frame has an outer diameter of less than 2.5 mm (e.g., less than 2.1 mm).

As described hereinabove, typically, axial shaft 92 passes through the axis of impeller 50, via lumen 62 of the impeller. Typically, proximal bushing 64 of the impeller is coupled to the shaft via a coupling element 65 such that the axial position of the proximal bushing with respect to the shaft is fixed, and distal bushing 58 of the impeller is slidable with respect to the shaft. (Alternatively, distal bushing 58 of the impeller is coupled to the shaft such that the axial position of the distal bushing with respect to the shaft is fixed, and proximal bushing 64 of the impeller is slidable with respect to the shaft.) The axial shaft itself is radially stabilized via a proximal radial bearing 116 and a distal radial bearing 118. Typically, proximal bearing housing 116H is disposed around, and houses, the proximal bearing, and distal bearing housing 118H is disposed around, and houses, the distal bearing. For some such applications, the radial bearings and the bearing housings are made of respective, different materials from each other. For example, the radial bearings may be made of a first material that has a relatively high hardness, such as ceramic (e.g., zirconia), and the bearing housings may be made of a second material that is moldable into a desired shape, such as a metal or an alloy (e.g., stainless steel, cobalt chromium, and/or nitinol).

For some applications, axial shaft 92 is made of a metal or an alloy, such as stainless steel. For some such applications, the axial shaft is covered with ceramic sleeves 240 (e.g., zirconia sleeves) along regions of the axial shaft that come into contact with either of the proximal and distal bearings 116, 118 during operation of the ventricular assist device. In this manner, the radial interfaces between the axial shaft and the proximal and distal bearings are ceramic-ceramic interfaces. As described in further detail herein, typically, the impeller and the axial shaft are configured to undergo axial back-and-forth motion during operation of the ventricular assist device. Therefore, for some applications, at locations along the axial shaft corresponding to each of the proximal and distal bearing, the axial shaft is covered with the ceramic sleeve along a length of more than 5 mm, e.g., more than 7 mm. In this manner, over the course of the axial back-and-forth motion of the axial shaft, the regions of the axial shaft that are in contact with the radial bearings are covered with the ceramic sleeves.

For some applications, along the portion of the axial shaft that is covered with the ceramic sleeve, the shaft is shaped (e.g., via milling, molding, or a different shaping process) to define one or more grooves or indents 95, as shown in the transverse cross-sectional view of Fig. 5C. Alternatively or additionally (not shown), the inner surface of the ceramic sleeve is shaped to define or more grooves or indents. For some such applications, in order to bond the sleeve to the axial shaft, an adhesive is injected into the groove or indent and the adhesive then spreads from the groove or ident across the interface between the axial shaft and the sleeve.

For some applications, the proximal bearing housing 116H and distal bearing housing 118H perform additional functions. Referring first to the proximal bearing housing, as described hereinabove, for some applications, proximal strut junctions 33 of frame 34 are closed around the outside of the proximal bearing housing. For some applications, the outer surface of the proximal bearing housing defines groves that are shaped such as to receive the proximal strut junctions. For example, as shown, the proximal strut junctions have widened heads, and the outer surface of the proximal bearing housing defines groves that are shaped to conform with the widened heads of the proximal strut junctions. Typically, securing element 117 (which typically includes a ring) holds the strut junctions in their closed configurations around the outside of proximal bearing housing 116H. For some applications, additional portions of the ventricular assist device are coupled to the proximal bearing housing. For some applications, a drive cable 130 extends from outside the subject's body to axial shaft 92, and is coupled to the axial shaft. Typically, the drive cable rotates within a first outer tube 140, which functions as a drive-cable bearing tube, and which extends from outside the subject's body to the proximal bearing housing. For some applications, the first outer tube is disposed within a second outer tube 142, which also extends from outside the subject's body to the proximal bearing housing. For some applications, first outer tube 140 and/or second outer tube 142 is coupled to the proximal bearing housing (e.g., using an adhesive). For example, first outer tube 140 may be coupled to an inner surface of the proximal bearing housing, and second outer tube 142 may be coupled to an outer surface of the proximal bearing housing.

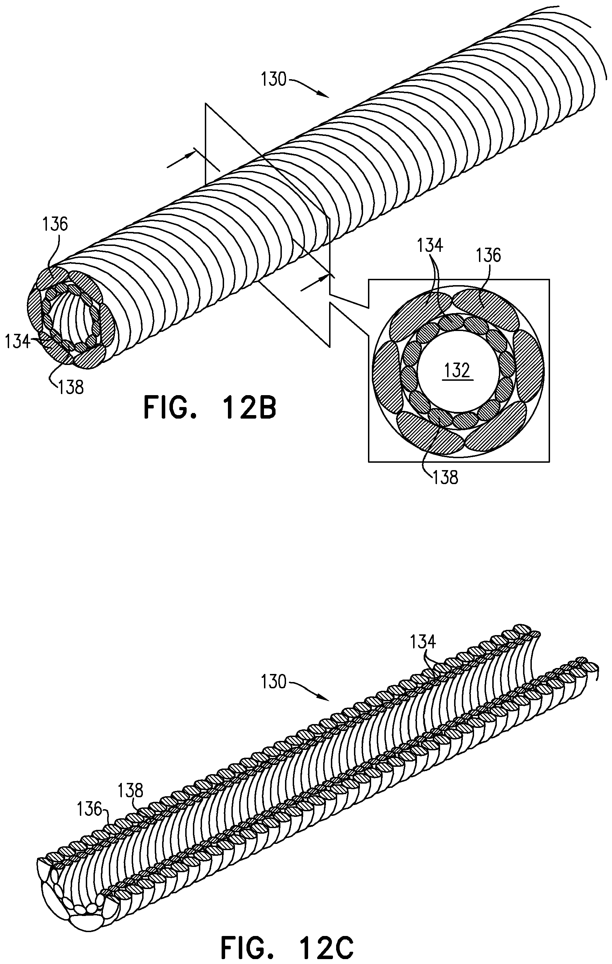

Referring now to distal bearing housing 118H, for some applications, distal coupling portion 31 of frame 34 is coupled to an outer surface of distal bearing housing 118H, e.g., via a snap-fit mechanism. For example, the outer surface of a proximal-most portion 119 of the distal bearing housing may include a snap-fit mechanism to which distal coupling portion 31 of frame 34 is coupled. For some applications, distal bearing 118 is disposed within the proximal-most portion 119 of the distal bearing housing, as shown in Fig. 5A. As described hereinabove, for some applications, pump-outlet tube 24 extends to the distal end of frame 34 and defines lateral blood-inlet openings 108. For some such applications, a coupling portion 41 (e.g., a tubular coupling portion) extends distally from the pump-outlet tube, and the coupling portion is coupled to the distal bearing housing in order to anchor the distal end of the pump-outlet tube. For some applications, an intermediate portion 123 of the distal bearing housing defines a ridged or a threaded outer surface, to which coupling portion 41 of the pump-outlet tube is coupled (e.g., via adhesive). For some applications, the outer surface is ridged in order to enhance bonding between the distal bearing housing and coupling portion 41 of the pump-outlet tube. For some applications, the outer surface is threaded in order to enhance bonding between the distal bearing housing and coupling portion 41 of the pump-outlet tube and to facilitate the application of adhesive between the outer surface and coupling portion 41 of the pump-outlet tube, as described in further detail hereinbelow with reference to Fig. 12B. For some applications, a distal portion 121 of the distal bearing housing is configured to stiffen a region of distal-tip element 107 into which the distal end of shaft 92 moves (e.g., axial-shaft-receiving tube 126, or a portion thereof). Typically, distal-tip element 107 is coupled to an outer surface of distal portion 121 of the distal bearing housing (e.g., via adhesive). For some applications, at least a portion of the outer surface of distal portion 121 of the distal bearing housing is ridged and/or threaded in order to enhance bonding between distal-tip element 107 and the distal bearing housing.

As described above, axial shaft 92 is radially stabilized via proximal radial bearing 116 and distal radial bearing 118. In turn, the axial shaft, by passing through lumen 62 defined by the impeller, radially stabilizes the impeller with respect to the inner surface of frame 34 and inner lining 39, such that even a relatively small gap between the outer edge of the blade of the impeller and inner lining 39 (e.g., a gap that is as described above) is maintained, during rotation of the impeller, as described hereinabove. Typically, the impeller itself is not directly disposed within any radial bearings or thrust bearings. Rather, bearings 116 and 118 act as radial bearings with respect to the axial shaft. Typically, pump-head portion 27 (and more generally ventricular assist device 20) does not include any thrust bearing that is configured to be disposed within the subject's body and that is configured to oppose thrust generated by the rotation of the impeller. For some applications, one or more thrust bearings are disposed outside the subject's body (e.g., within motor unit 23, shown in Figs. 1A, and 7A-C), and opposition to thrust generated by the rotation of the impeller is provided solely by the one or more thrust bearings disposed outside the subject's body. For some applications, a mechanical element and/or a magnetic element is configured to maintain the impeller within a given range of axial positions. For example, a magnet (e.g., magnet 82, described hereinbelow with reference to Fig. 7A) that is disposed at the proximal end of the drive cable (e.g., outside the subject's body) may be configured to impart axial motion to the impeller, and/or to maintain the impeller within a given range of axial positions.

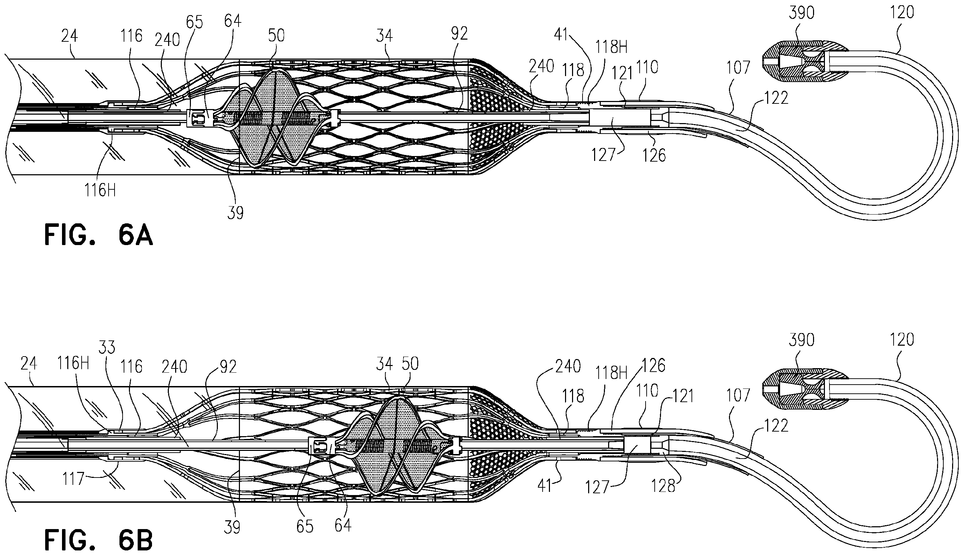

Reference is now made to Figs. 6A and 6B, which are schematic illustrations of ventricular assist device 20 at respective stages of a motion cycle of impeller 50 of the ventricular assist device with respect to frame 34 of the ventricular assist device, in accordance with some applications of the present invention. For some applications, while the impeller is pumping blood through tube 24 by rotating, axial shaft 92 (to which the impeller is fixated) is driven to move the impeller axially back-and-forth within frame 34, by the axial shaft moving in an axial back-and-forth motion, as described in further detail hereinbelow with reference to Fig. 7A. Alternatively or additionally, the impeller and the axial shaft are configured to move axially back-and-forth within frame 34 in response to forces that are acting upon the impeller, and without requiring the axial shaft to be actively driven to move in the axial back-and-forth motion. Typically, over the course of the subject's cardiac cycle, the pressure difference between the left ventricle and the aorta varies from being approximately zero during ventricular systole (hereinafter "systole") to a relatively large pressure difference (e.g., 50-70 mmHg) during ventricular diastole (hereinafter "diastole"). For some applications, due to the increased pressure difference that the impeller is pumping against during diastole (and due to the fact that drive cable 130 is stretchable), the impeller is pushed distally with respect to frame 34 during diastole, relative to the location of the impeller with respect to frame 34 during systole. In turn, since the impeller is connected to the axial shaft, the axial shaft is moved forward. During systole, the impeller (and, in turn, the axial shaft) move back to their systolic positions. In this manner, the axial back-and-forth motion of the impeller and the axial shaft is generated in a passive manner, i.e., without requiring active driving of the axial shaft and the impeller, in order to cause them to undergo this motion. Figs. 6A and 6B show the impeller and axial shaft disposed at respective positions within frame 34 during the above-described axial back-and-forth motion cycle.

For some applications, by moving in the axial back-and-forth motion, the portions of the axial shaft that are in contact with proximal bearing 116 and distal bearing 118 are constantly changing. For some such applications, in this manner, the frictional force that is exerted upon the axial shaft by the bearings is spread over a larger area of the axial shaft than if the axial shaft were not to move relative to the bearings, thereby reducing wear upon the axial shaft, *ceteris paribus.* Alternatively or additionally, by moving in the back-and-forth motion with respect to the bearing, the axial shaft cleans the interface between the axial shaft and the bearings from any residues, such as blood residues.

For some applications, at the proximal-most position of the impeller during its motion cycle, the proximal end of the impeller is disposed within the proximal conical section of frame 34, as shown in Fig. 6A. For some applications, at the distal-most position of the impeller during its motion cycle, the distal end of the impeller is disposed at the distal end of the cylindrical section of frame 34. Alternatively, even at the distal-most position of the impeller during its motion cycle, the distal end of the impeller is disposed proximal to the distal end of the cylindrical section of frame 34, as shown in Fig. 6B. Typically, over the course of the entire cardiac cycle, the section of the impeller at which the span of the impeller is at its maximum is disposed within the cylindrical portion of the frame 34. However, a proximal portion of the impeller is typically disposed within the proximal conical section of the frame during at least a portion of the cardiac cycle.

Reference is again made to Figs. 6A and 6B. Typically, distal-tip element 107 is a single integrated element that includes both axial-shaft-receiving tube 126 and distal-tip portion 120. Typically, the axial-shaft receiving tube is configured to receive a distal portion of axial shaft 92 of the pump-head portion during axial back-and-forth motion of the axial shaft (as described in further detail hereinbelow), and/or during delivery of the ventricular assist device. (Typically, during delivery of the ventricular assist device, the frame is maintained in a radially-constrained configuration, which typically causes the axial shaft to be disposed in a different position with respect to the frame relative to its disposition with respect to the frame during operation of the ventricular assist device). For some applications, distal-tip portion 120 is configured to be soft, such that the distal-tip portion is configured not to injure tissue of the subject, even if the distal-tip portion comes into contact with the tissue (e.g., tissue of the left ventricle). For example, distal-tip portion 120 or the entire distal-tip element may be made of silicone, polyethylene terephthalate (PET) and/or polyether block amide (e.g., PEBAX®). For some applications, the distal-tip portion defines a lumen 122 therethrough. For some such applications, during insertion of the ventricular assist device into the left ventricle, guidewire 10 (Fig. 1B) is first inserted into the left ventricle, for example, in accordance with known techniques. The distal-tip portion of the ventricular assist device is then guided to the left ventricle by advancing the distal-tip portion over the guidewire, with the guidewire disposed inside lumen 122. For some applications, a duckbill valve 390 (or a different type of hemostasis valve) is disposed at the distal end of lumen 122 of distal-tip portion 120.

Typically, during the insertion of the ventricular assist device into the subject's ventricle, delivery catheter 143 is placed over impeller 50 and frame 34 and maintains the impeller and the frame in their radially-constrained configurations. For some applications, distal-tip element 107 extends distally from the delivery catheter during the insertion of the delivery catheter into the subject's ventricle, as shown in Fig. 1B. For some applications, toward the proximal end of the distal-tip element, the distal-tip element has a protrusion 110. Referring to Fig. 5B (which shows the pump-head portion disposed inside delivery catheter 143), for some applications, during the insertion of the ventricular assist device into the subject's ventricle, the delivery catheter extends until the proximal side of the protrusion, such that the delivery catheter and the protrusion form a smooth continuous surface. The distal side of protrusion 110 is tapered, such that the vasculature is exposed to a tapered diameter change, and is not exposed to any edges arising from a sharp change in diameter at the interface between the delivery catheter and the distal-tip element.

For some applications, distal-tip element 107 defines an overall curvature that is similar to that of a question mark or a tennis-racket, with the distal-tip element defining a straight proximal portion and a bulge on one side of the longitudinal axis of the straight proximal portion. Typically, the ventricular assist device is introduced into the subject's ventricle over a guidewire, as described hereinabove. Distal-tip portion 120 defines lumen 122, such that the distal-tip portion is held in a straightened configuration during the introduction of the ventricular assist device into the subject's ventricle (e.g., as shown in the left frame of Fig. 1B). For some applications, upon the guidewire being removed, distal-tip portion is configured to assume its curved shape.

Referring again to Figs. 6A-B, for some applications, axial-shaft-receiving tube 126 extends proximally from distal-tip portion 120 of distal-tip element 107. As described hereinabove, typically, the axial shaft undergoes axial back-and-forth motion during the operation of impeller 50. Axial-shaft-receiving tube 126 defines lumen 127, which is configured to receive the axial shaft when the axial shaft extends beyond distal bearing 118. For some applications, the axial shaft-receiving tube defines a stopper 128 at its distal end, the stopper being configured to prevent advancement of the axial shaft beyond the stopper. For some applications, the stopper comprises a rigid component that is inserted (e.g., embedded) into the distal end of the shaft-receiving tube. Alternatively (not shown), the stopper comprises a shoulder between lumen 127 of the axial-shaft-receiving tube and lumen 122 of distal-tip portion 120.

Typically, during normal operation of the impeller, the axial shaft does not come into contact with stopper 128, even when drive cable 130 (shown in Fig. SA) is maximally elongated (e.g., during diastole). However, stopper 128 is configured to prevent the axial shaft from protruding into the tip portion when the delivery catheter is advanced over impeller 50 and frame 34, during retraction of ventricular assist device 20 from the subject's ventricle. In some cases, during the advancement of the delivery catheter over the frame and the impeller, the drive cable is at risk of snapping. In the absence of stopper 128, in such cases, the axial shaft may protrude into the tip portion. Stopper 128 prevents this from happening, even in the event that the drive cable snaps.

It is noted that, at the proximal end of frame 34, proximal radial bearing 116 also functions as a stopper, by preventing coupling element 65 and/or proximal bushing 64 of impeller 50 from being able to move beyond the proximal radial bearing. Typically, during normal operation of the impeller, coupling element 65 and proximal bushing 64 do not come into contact with proximal radial bearing 116. However, proximal radial bearing 116 is configured to prevent coupling element 65 and/or proximal bushing 64 of impeller 50 from migrating proximally from inside the frame, for example, when the impeller and the frame are held in radially-constrained (i.e., crimped) configurations inside delivery catheter 143. Typically, the coupling element and/or the proximal bushing is proximally-extended such as to prevent a central region of the impeller (at which the span of the impeller is at its maximum) from sliding proximally into the proximal conical portion of frame 34. For example, in the systolic phase of the impeller's motion cycle (shown in Fig. 6A), if the impeller were to slide further proximally by more than a given amount, the coupling element would contact proximal radial bearing 116, thereby preventing further proximal motion of the impeller. For some applications, the coupling element and/or the proximal bushing is proximally extended such that it has a total length of more than 1.5 mm, e.g., more than 4 mm. For some applications (not shown), a separate stopper element is disposed upon axial shaft proximally with respect to coupling element and/or proximal bushing 64. Typically, the stopper is configured as described with reference to the coupling element. Namely, such that if the impeller were to slide further proximally by more than a given amount, the stopper element would contact proximal radial bearing 116, thereby preventing further proximal motion of the impeller.

Typically, during operation of the ventricular assist device, and throughout the axial back-and-forth motion cycle of the impeller, the impeller is disposed in relatively close proximity to the distal-tip portion. For example, the distance of the impeller to the distal-tip portion may be within the distal-most 50 percent, e.g., the distal-most 30 percent (or the distal-most 20 percent) of tube 24, throughout the axial back-and-forth motion cycle of the impeller.

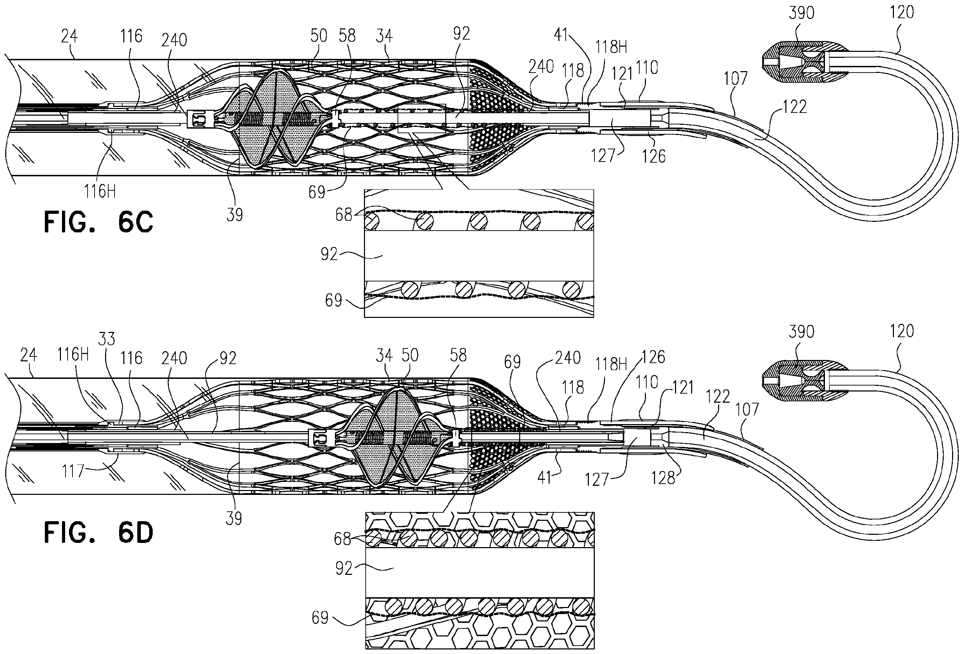

Reference is now made to Figs. 6C and 6D, which are the only schematic illustrations of a ventricular assist device that includes a motion-cushioning spring 68, in accordance with the present invention. As described in further detail hereinbelow, typically, during operation of the ventricular assist device (i.e., as the impeller is rotating), the impeller undergoes axial back-and-forth motion. For some applications, as the impeller undergoes the axial back-and-forth motion, the motion-cushioning spring is configured to act as a shock absorber, to provide cushioning to the motion. Fig. 6C shows the impeller at the systolic phase of the motion cycle of the impeller and Fig. 6D shows the impeller at the diastolic phase of its motion cycle. As shown, as the impeller moves distally from its systolic position to its diastolic position, the motion-cushioning spring becomes more compressed. For some applications, the impeller is configured to be radially constrained (i.e., crimped) by becoming axially elongated, and the motion-cushioning spring is configured to become compressed such as to accommodate the axial elongation of the impeller. Typically, when the impeller is in a radially-constrained configuration, during insertion of the pump head into the left ventricle, the impeller is axially elongated such that the distal end of the impeller is disposed further distally within frame 34 and the spring is further compressed relative to the configurations of the impeller and the spring shown in Fig. 6D.

Typically, the motion-cushioning spring is disposed around axial shaft 92 between the distal end of the impeller (e.g., distal bushing 58 of the impeller) and distal bearing 118. For some applications, the motion-cushioning spring is coupled to distal bearing 118 or the distal bearing housing 118H and extends proximally over axial shaft 92 from the distal bearing or the distal bearing housing 118H. Typically, in such cases, the motion-cushioning spring remains rotationally stationary as the impeller rotates, and the impeller is configured to rotate with respect to the motion-cushioning spring. Alternatively or additionally, the motion-cushioning spring is coupled to the distal end of the impeller (e.g., distal bushing 58 of the impeller) and/or extends distally over axial shaft 92 from the distal end of the impeller (e.g., distal bushing 58 of the impeller). For some such applications, the motion-cushioning spring is configured to rotate together with the impeller. Alternatively, the motion-cushioning spring extends from a radial bearing that is disposed around the distal end of the impeller (e.g., distal bushing 58 of the impeller), such that motion-cushioning spring remains rotationally stationary as the impeller rotates, and the impeller is configured to rotate with respect to the motion-cushioning spring.

For some applications, the motion-cushioning spring is coupled to an elastomeric material 69 (such as polyurethane, and/or silicone), such that at least a portion of axial shaft 92 that is between the distal end of the impeller and the distal radial bearing is covered by the elastomeric material. For some applications, coupling the elastomeric material to the spring reduces a risk of the generation of thrombi and/or hemolysis by the spring, relative to if the spring is not coupled to the elastomeric material. It is noted that the scope of the present disclosure includes providing the motion-cushioning spring in the absence of elastomeric material, as may be desirable in some cases.

Figs. 6C and 6D show the spring as being coated with the elastomeric material with the elastomeric material extending between adjacent windings of the spring. Alternatively, the spring is embedded within the elastomeric material. Typically, the elastomeric material is generally similar to the elastomeric material that is used for film 56 of material within impeller 50. Further typically, the elastomeric material is coupled to the motion-cushioning spring in a generally similar manner to that described hereinabove with reference to the coupling of the film of elastomeric material to the spring of the impeller. Typically, the elastomeric material is coupled to the motion-cushioning spring in such a manner that the elastomeric material changes shape (e.g., by stretching and compressing) to conform to shape changes that the motion-cushioning spring undergoes (e.g., when the motion-cushioning spring undergoes elongation and compression). Further typically, the elastomeric material is configured to undergo the above-described shape changes without the elastomeric material becoming broken or collapsing, and without the elastomeric material becoming creased when the spring is compressed.

As described hereinabove, typically purging fluid is pumped between outer tube 140 and outer tube 142. Typically, within the pump head, a portion of the purging fluid flows through the lumen defined by axial shaft 92 and then exits the axial shaft in the vicinity of distal bearing 118, in order to purge the interface between the axial shaft and the distal bearing. For some applications, the purging system is configured such that purging fluid flows proximally from the distal bearing along an interface between the axial shaft and the elastomeric material. In this manner, the interface between the axial shaft and the elastomeric material is purged and/or lubricated.

For some applications (not shown), a proximal motion-cushioning spring is disposed on the proximal side of the impeller. For some such applications, the proximal motion-cushioning spring is disposed around axial shaft 92 between the proximal end of the impeller (e.g., proximal bushing 64 of the impeller) and proximal bearing 116. For some applications, the proximal motion-cushioning spring is coupled to proximal bearing 116 or proximal bearing housing 116H and extends distally over axial shaft 92 from the proximal bearing or the proximal bearing housing. Typically, in such cases, the proximal motion-cushioning spring remains rotationally stationary as the impeller rotates, and the impeller is configured to rotate with respect to the motion-cushioning spring. Alternatively or additionally, the proximal motion-cushioning spring is coupled to the proximal end of the impeller (e.g., proximal bushing 64 of the impeller) and/or extends distally over axial shaft 92 from the proximal end of the impeller (e.g., proximal bushing 64 of the impeller). For some such applications, the motion-cushioning spring is configured to rotate together with the impeller. Alternatively, the proximal motion-cushioning spring extends from a radial bearing that is disposed around the proximal end of the impeller (e.g., proximal bushing 64 of the impeller), such that motion-cushioning spring remains rotationally stationary as the impeller rotates, and the impeller is configured to rotate with respect to the motion-cushioning spring.

For some applications, the pump head includes both a proximal motion-cushioning spring (disposed on a proximal side of the impeller) and a distal motion-cushioning spring disposed on a distal side of the impeller, such that axial movement of the impeller in either the distal or the proximal direction is cushioned by the motion-cushioning springs.

Reference is now made to Fig. 7A, which is a schematic illustration of an exploded view of motor unit 23 of ventricular assist device 20, in accordance with some applications of the present invention. For some applications, computer processor 25 of control console 21 (Fig. 1A) that controls the rotation of impeller 50 is also configured to control the back-and-forth motion of the axial shaft. For some such applications, both types of motion are generated using motor unit 23. The scope of the present invention includes controlling the back-and-forth motion at any frequency. For some applications, an indication of the subject's cardiac cycle is detected (e.g., by detecting the subject's ECG), and the back-and-forth motion of the axial shaft is synchronized to the subject's cardiac cycle.

Typically, motor unit 23 includes a motor 74 that is configured to impart rotational motion to impeller 50, via drive cable 130. As described in further detail hereinbelow, typically, the motor is magnetically coupled to the drive cable. For some applications, an axial motion driver 76 is configured to drive the motor to move in an axial back-and-forth motion, as indicated by double-headed arrow 79. Typically, by virtue of the magnetic coupling of the motor to the drive cable, the motor imparts the back-and-forth motion to the drive cable, which it turn imparts this motion to the impeller. As described hereinabove and hereinbelow, for some applications, the drive cable, the impeller, and/or the axial shaft undergo axial back-and-forth motion in a passive manner, e.g., due to cyclical changes in the pressure gradient against which the impeller is pumping blood. Typically, for such applications, motor unit 23 does not include axial motion driver 76.

For some applications, the magnetic coupling of the motor to the drive cable is as shown in Fig. 7A. As shown in Fig. 7A, a set of driving magnets 77 are coupled to the motor via a driving magnet housing 78. For some applications, the driving magnet housing includes ring 81 (e.g., a steel ring), and the driving magnets are adhered to an inner surface of the ring. For some applications a spacer 85 is adhered to the inner surface of ring 81, between the two driving magnets, as shown. A driven magnet 82 is disposed between the driving magnets such that there is axial overlap between the driving magnets and the driven magnet. The driven magnet is coupled to a pin 131, which extends to beyond the distal end of driven magnet 82, where the pin is coupled to the proximal end of drive cable 130. For example, the driven magnet may be cylindrical and define a hole therethrough, and pin 131 may be adhered to an inner surface of the driven magnet that defines the hole. For some applications, the driven magnet is cylindrical, and the magnet includes a North pole and a South pole, which are divided from each other along the length of the cylinder along a line 83 that bisects the cylinder, as shown. For some applications, the driven magnet is housed inside a cylindrical housing 87. Typically, pin 131 defines a guidewire lumen 133.

It is noted that in the application shown in Fig. 7A, the driving magnets are disposed outside the driven magnet. However, the scope of the present application includes reversing the configurations of the driving magnets and the driven magnet, *mutatis mutandis.* For example, the proximal end of the drive cable may be coupled to two or more driven magnets, which are disposed around a driving magnet, such that there is axial overlap between the driven magnets and the driving magnet.

As described hereinabove, typically purging system 29 (shown in Fig. 1A) is used with ventricular assist device 20. Typically, motor unit 23 includes an inlet port 86 and an outlet port 88, for use with the purging system. For some applications, a purging fluid is continuously or periodically pumped into the ventricular assist device via inlet port 86 and out of the ventricular assist device via outlet port 88.

Typically, magnet 82 and pin 131 are held in axially fixed positions within motor unit 23. The proximal end of the drive cable is typically coupled to pin 131 and is thereby held in an axially fixed position by the pin. Typically, drive cable 130 extends from pin 131 to axial shaft 92 and thereby at least partially fixes the axial position of the axial shaft, and in turn impeller 50. For some applications, the drive cable is somewhat stretchable. For example, the drive cable may be made of coiled wires that are stretchable. The drive cable typically allows the axial shaft (and in turn the impeller) to assume a range of axial positions (by the drive cable becoming more or less stretched), but limits the axial motion of the axial shaft and the impeller to being within a certain range of motion (by virtue of the proximal end of the drive cable being held in an axially fixed position, and the stretchability of the drive cable being limited).

As described hereinabove, for some applications, impeller 50 and axial shaft 92 are configured to move axially back-and-forth within frame 34 in response to forces that act upon the impeller, and without requiring the axial shaft to be actively driven to move in the axial back-and-forth motion. Typically, over the course of the subject's cardiac cycle, the pressure difference between the left ventricle and the aorta varies from being approximately zero during systole to a relatively large pressure difference (e.g., 50-70 mmHg) during diastole. For some applications, due to the increased pressure difference that the impeller is pumping against during diastole (and due to the drive cable being stretchable), the impeller is pushed distally with respect to frame 34 during diastole, relative to the location of the impeller with respect to frame 34 during systole. In turn, since the impeller is connected to the axial shaft, the axial shaft is moved forward. During systole, the impeller (and, in turn, the axial shaft) move back to their systolic positions. In this manner, the axial back-and-forth motion of the impeller and the axial shaft is generated in a passive manner, i.e., without requiring active driving of the axial shaft and the impeller, in order to cause them to undergo this motion.

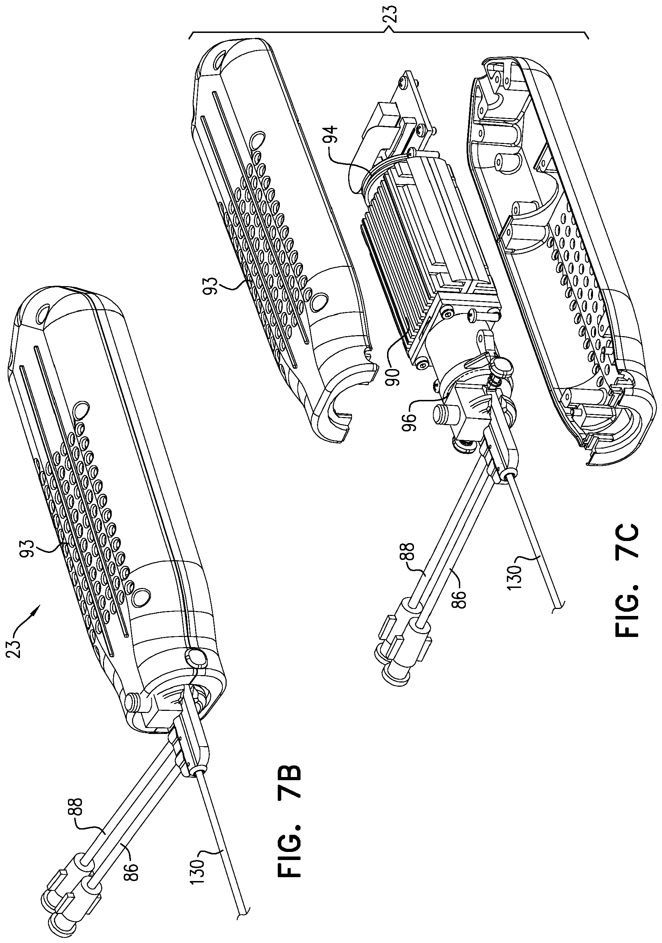

Reference is now made to Figs. 7B and 7C, which are schematic illustrations of motor unit 23, in accordance with some applications of the present invention. In general, motor unit 23 as shown in Figs. 7B and 7C is similar to that shown in Fig. 7A, and, unless described otherwise, motor unit 23 as shown in Figs. 7B and 7C contains similar components to motor unit 23 as shown in Fig. 7A. For some applications, the motor unit includes a heat sink 90 that is configured to dissipate heat that is generated by the motor. Alternatively or additionally, the motor unit includes ventilation ports 93 that are configured to facilitate the dissipation of heat that is generated by the motor. For some applications, the motor unit includes vibration dampeners 94 and 96 that are configured to dampen vibration of the motor unit that is caused by rotational motion and/or axial back-and-forth motion of components of the ventricular assist device.

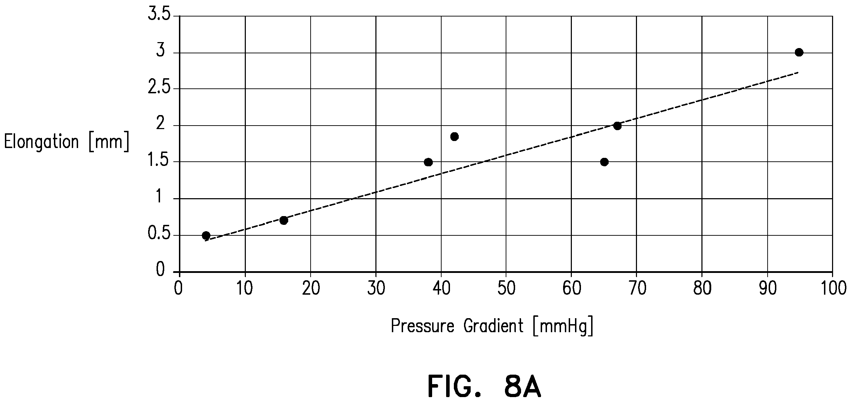

Reference is now made to Fig. 8A, which is a graph indicating variations in the length of a drive cable of a ventricular assist device, as a pressure gradient against which the impeller of the ventricular assist device varies, as measured in experiments. An impeller and a drive cable as described herein were used to pump a glycerin-based solution through chambers, with the chambers set up to replicate the left ventricle and the aorta, and the solution having properties (such as, density and viscosity) similar to those of blood. The pressure gradient against which the impeller was pumping was varied in a pulsatile manner to represent the pulsatility of the pressure gradient against which the impeller typically pumps when it is pumping blood from the left ventricle to the aorta. At the same time, movement of the drive cable was imaged and changes in the length of the drive cable were determined via analysis of the images. The graph shown in Fig. 8A indicates the changes in the length of the drive cable that were measured, as a function of the pressure gradient. As shown in Fig. 8A, as the pressure gradient against which the impeller pumped increased, the drive cable became increasingly elongated. As indicated by the results shown in Fig. 8A and as described hereinabove, it is typically the case that, in response to variations in the pressure against which the impeller is pumping blood (e.g., the pressure difference between the left ventricle and the aorta), the impeller moves back and forth with respect to frame 34. In turn, the movement of the impeller causes drive cable 130 to become more or less elongated.

For some applications, during operation of the ventricular assist device, computer processor 25 of control console 21 (Fig. 1A) is configured to measure an indication of the pressure exerted upon the impeller (which is indicative of the pressure difference between the left ventricle and the aorta), by measuring an indication of tension in drive cable 130, and/or axial motion of the drive cable. For some applications, based upon the measured indication, the computer processor detects events in the subject's cardiac cycle, determines the subject's left-ventricular pressure, and/or determines the subject's cardiac afterload. For some applications, the computer processor controls the rotation of the impeller, and/or the axial back-and-forth motion of the axial shaft in response thereto.

Referring again to Fig. 7A, for some applications, ventricular assist device 20 includes a sensor 84. For example, the sensor may include a magnetometer (e.g., a Hall sensor) that is disposed within motor unit 23, as shown in Fig. 7A. (In some cases, sensor 84 is referred to as magnetometer 84.) For some applications, it is the case that the axial back-and-forth motion of the impeller gives rise to a measurable back-and-forth motion of the inner, driven magnet 82 relative to the outer, one or more drive magnets 77, since the driven magnet is held in place with respect to the drive magnets via magnetic coupling, rather than rigid mechanical coupling. It is noted that typically the axial motion of the magnet is substantially less than that of the impeller, since the full range of motion of the impeller is not transmitted along the length of the drive cable (the drive cable typically being somewhat stretchable). For some applications, the magnetometer measures variations in the magnetic field that is generated by one of the magnets in order to measure the axial motion of drive cable 130, and, in turn, to determine the pressure against which the impeller is pumping. For example, the inner, driven magnet 82 may be axially longer than the outer, driving magnets 77. Due to the inner magnet being longer than the outer magnets, there are magnetic field lines that emanate from the inner magnet that do not pass to the outer magnets, and the magnetic flux generated by those field lines, as measured by the magnetometer, varies as the drive cable, and, in turn, the inner magnet moves axially. During operation, motor 74 rotates, creating an AC signal in the magnetometer, which typically has a frequency of between 200 Hz and 800 Hz. Typically, as the tension in the drive cable changes due to the subject's cardiac cycle, this gives rise to a low frequency envelope in the signal measured by the magnetometer, the low frequency envelope typically having a frequency of 0.5-2 Hz. For some applications, the computer processor measures the low frequency envelope, and derives the subject's cardiac cycle from the measured envelope.

For some applications, the magnetometer measurements are initially calibrated, such that the change in magnetic flux per unit change in pressure against which the impeller is pumping (i.e., per unit change in the pressure difference between the left ventricle and the aorta, or per unit change in the pressure gradient) is known. It is known that, in most subjects, at systole, the left-ventricular pressure is equal to the aortic pressure. Therefore, for some applications, the subject's aortic pressure is measured, and the subject's left-ventricular pressure at a given time is then calculated by the computer processor, based upon (a) the measured aortic pressure, and (b) the difference between the magnetic flux measured by the magnetometer at that time, and the magnetic flux measured by the magnetometer during systole (when the pressure in the left ventricle is assumed to be equal to that of the aorta). For example, the subject's aortic pressure may be measured by measuring pressure in a channel 224 defined by delivery catheter 143, as described in further detail hereinbelow. For some applications, alternative or additional physiological parameters are determined using the above-described technique. For example, events in the subject's cardiac cycle and/or the subject's cardiac afterload may be determined.

For some applications, generally similar techniques to those described in the above paragraph are used, but as an alternative to or in addition to utilizing magnetometer measurements, a different parameter is measured in order to determine left ventricular blood pressure (and/or a different physiological parameter, e.g., events in the subject's cardiac cycle and/or the subject's cardiac afterload) at a given time. For example, it is typically the case that there is a relationship between the amount of power (and/or current) that is required to power the rotation of the impeller at a given rotation rate and the pressure difference that is generated by the impeller. (It is noted that some of the pressure difference that is generated by the impeller is used to overcome the pressure gradient against which the impeller is pumping, and some of the pressure difference that is generated by the impeller is used to actively pump the blood from the left ventricle to the aorta, by generating a positive pressure difference between the left ventricle and the aorta. Moreover, the relationship between the aforementioned components typically varies over the course of the cardiac cycle.) For some applications, calibration measurements are performed, such that the relationship between (a) power (and/or current) consumption by the motor that is required to rotate the impeller at a given rotation rate and (b) the pressure difference that is generated by the impeller, is known. For some applications, the subject's aortic pressure is measured, and the subject's left-ventricular pressure at a given time is then calculated by the computer processor, based upon (a) the measured aortic pressure, (b) the power (and/or current) consumption by the motor that is required to rotate the impeller at a given rotation rate at that time, and (c) the predetermined relationship between power (and/or current) consumption by the motor that is required to rotate the impeller at a given rotation rate and the pressure difference that is generated by the impeller. For some applications, the above-described technique is performed while maintaining the rotation rate of the impeller at a constant rate. Alternatively or additionally, the rotation rate of the impeller is varied, and the variation of the rotation rate of the impeller is accounted for in the above-described calculations. For some applications, alternative or additional physiological parameters are determined using the above-described technique. For example, events in the subject's cardiac cycle and/or the subject's cardiac afterload may be determined.

Typically, tube 24 has a known cross-sectional area (when the tube is in an open state due to blood flow through the tube). For some applications, the flow through tube 24 that is generated by the impeller is determined based on the determined pressure difference that is generated by the impeller, and the known cross-sectional area of the tube. For some applications, such flow calculations incorporate calibration parameters in order to account for factors such as flow resistance that are specific to the ventricular assist device (or type of ventricular assist device) upon which the calculations are performed. For some applications, the ventricular pressure-volume loop is derived, based upon the determined ventricular pressure.

Referring again to Fig. 7A, for some applications, in addition to magnetometer 84, which is configured to measure the magnetic flux density generated by the driven magnet, a second magnetometer 84A (e.g., a second Hall sensor) measures an indication of the magnetic flux density generated by the driving magnet. For some applications, the second magnetometer measures magnetic flux density of the motor, which is indicative of the flux density cycle of the driving magnet, since the motor directly drives the driving magnet to rotate. Typically, as the impeller rotates such as to pump blood, torque is generated upon the impeller. Further typically, the strength of the torque is dependent upon various parameters, such as the flow that is generated by the impeller, the rotational speed of the impeller, and/or the pressure gradient against which the impeller is pumping. For some applications, it is the case that the torque generated upon the impeller gives rise to a measurable torque on the inner, driven magnet 82 relative to the outer, drive magnets 77, since the driven magnet is held in place with respect to the drive magnets via magnetic coupling, rather than rigid mechanical coupling. It is noted that typically the torque generated upon the driven magnet is substantially less than that generated upon the impeller, since the torque that is generated upon the impeller is not transmitted along the length of the drive cable. However, it is typically the case that the torque that is generated upon the impeller is at least partially transmitted to the driven magnet via the drive cable.

The torque that is transmitted to the driven magnet typically gives rise to a phase difference between the signal that is measured by magnetometer 84 (which measures magnetic flux density of the driven magnet) and the signal that is measured by second magnetometer 84A (which measures magnetic flux density of the motor and/or the driving magnet). For some applications, as the torque upon the impeller varies, this gives rise to a variation in the phase difference between the signal that is measured by magnetometer 84 and the signal that is measured by second magnetometer 84A. For some applications, the computer processor detects the variation in the aforementioned phase difference, and determines a physiological parameter of the subject, at least partially in response thereto. For example, at least partially based upon variations in the phase difference, the computer processor may determine the difference between the subject's left-ventricular pressure and the subject's aortic pressure, the subject's left ventricular pressure, an event in the subject's cardiac cycle, the subject's cardiac afterload, and/or a different physiological parameter. For some applications, the technique described in the present paragraph is used as an alternative to the above-described technique for using magnetic flux density measurements and/or power consumption measurements to determine physiological parameters. Alternatively, two or more of these techniques are used in combination with each other. For example, the subject's physiological parameters may be determined based upon a mathematical model that incorporates two or more measurements, and/or one of the techniques may be used to validate estimations of the subject's physiological parameters that are made using another one of the techniques.

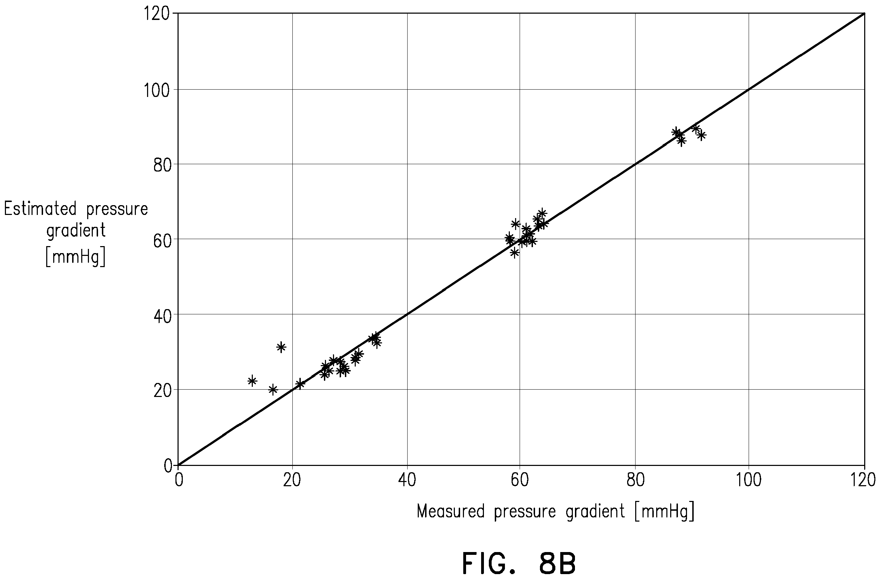

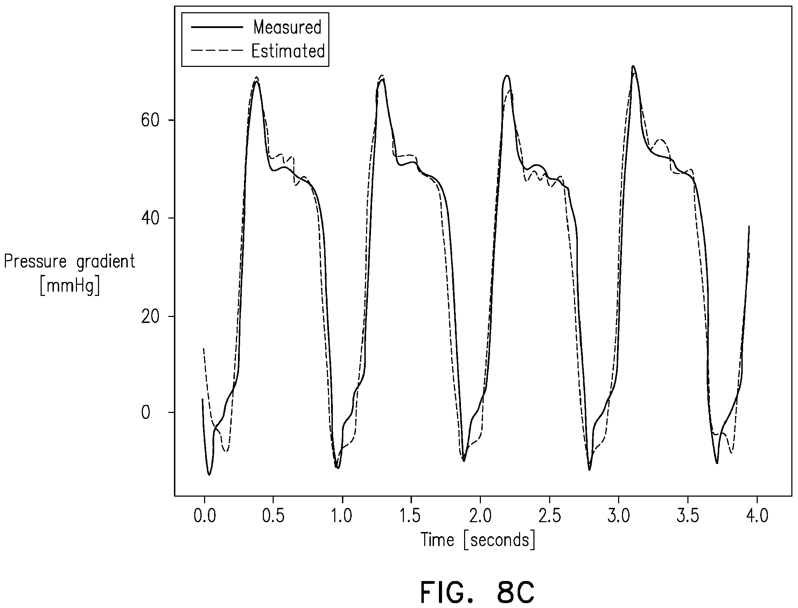

Reference is now made to Figs. 8B and 8C, which are graphs that demonstrate the correlation between the phase difference signal and the pressure gradient against which impeller 50 pumps, in accordance with some applications of the present invention.

The graph shown in Fig. 8B shows the results of an experiment in which a ventricular assist device as described herein was used to pump blood against respective pressure gradients within a static in vitro system (i.e., in which when each measurement was taken, the pressure gradient was constant). A linear regression model was used to estimate the pressure gradient against which the impeller was pumping based upon a combination of the phase difference signal the magnetic flux amplitude signal, and the current consumed by the motor. The graph shown in Fig. 8B shows the estimated pressure gradient versus the measured pressure gradient. As shown the linear regression model which incorporates phase difference measurements provides a reliable method for estimating the pressure gradient against which the impeller is pumping.

The graph shown in Fig. 8C shows the results of an experiment in which a ventricular assist device as described herein was used to pump blood against respective pressure gradients within a pulsatile in vitro system (i.e., in which the pressure gradient was varied in a pulsatile manner). A space state model was used to estimate the pressure gradient against which the impeller was pumping based upon a combination of the phase difference signal the magnetic flux amplitude signal, and the current consumed by the motor. The graph shown in Fig. 8C shows the estimated pressure gradient overlaid upon the measured pressure gradient. As shown a space state model which incorporates phase difference measurements provides a reliable method for estimating the pressure gradient against which the impeller is pumping.

In accordance with the above, and in accordance with some applications of the invention, a magnetic phase difference between the one or more driven magnets and the one or more drive magnets is measured, and a physiological parameter of the subject is determined, at least partially in response thereto. For example, at least partially based upon variations in the phase difference, the computer processor may determine the difference between the subject's left-ventricular pressure and the subject's aortic pressure, the subject's left ventricular pressure, an event in the subject's cardiac cycle, the subject's cardiac afterload, and/or a different physiological parameter. For some applications, the physiological parameter is determined based upon the phase difference measurements in combination with one or more additional measurements, such as magnetic flux amplitude measurements, power consumed by the motor, and/or current consumed by the motor. Typically, such measurements are combined into a mathematical model, such as a linear regression model, and/or a space state model.

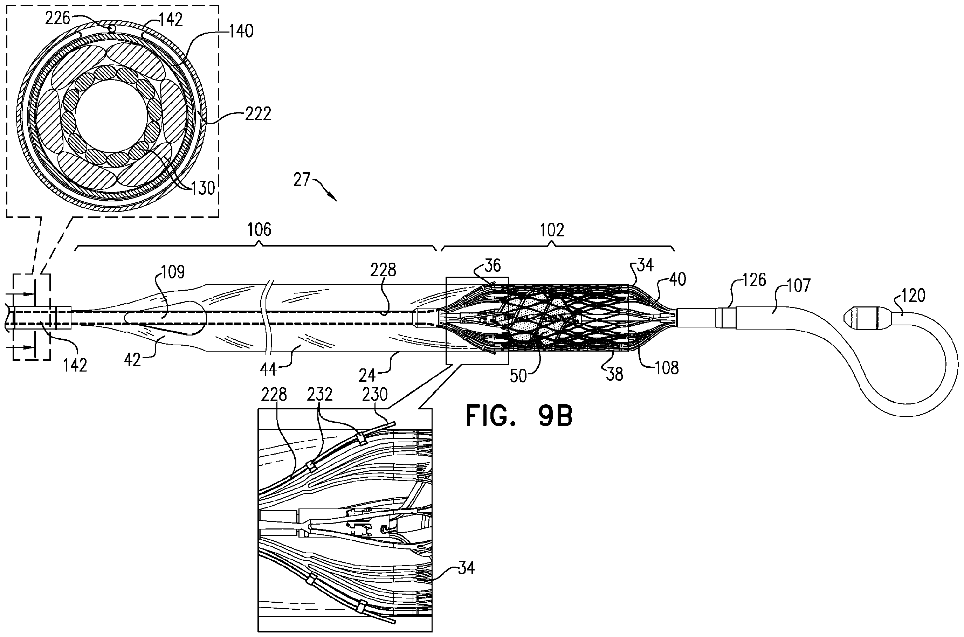

Reference is now made to Figs. 9A and 9B, which are schematic illustrations of a ventricular assist device that includes one or more blood-pressure-measurement tubes 222 and/or fibers 228, in accordance with some applications of the present invention.

Fig. 9A is a schematic illustration of a ventricular assist device that includes one or more blood-pressure-measurement tubes 222, in accordance with some applications of the present invention. As described hereinabove, typically, the ventricular assist device includes pump-outlet tube 24, which traverses the subject's aortic valve, such that a proximal end of the tube is disposed within the subject's aorta and a distal end of the tube is disposed within the subject's left ventricle. Typically, a blood pump (which typically includes impeller 50), is disposed within the subject's left ventricle within tube 24, and is configured to pump blood through tube 24 from the left ventricle into the subject's aorta. For some applications, ventricular blood-pressure-measurement tube 222 is configured to extend to at least an outer surface 213 of tube 24, such that an opening 214 at the distal end of the blood-pressure-measurement tube is in direct fluid communication with the patient's bloodstream outside tube 24. Typically, opening 214 is configured to be within the subject's left ventricle proximal to the blood pump (e.g., proximal to impeller 50). A pressure sensor 216 (illustrated schematically in Fig. 1A) measures pressure of blood within the ventricular blood-pressure-measurement tube. Typically, by measuring pressure of blood within the left ventricular blood-pressure-measurement tube, the pressure sensor thereby measures the subject's blood pressure outside tube 24 (i.e., left ventricular blood pressure). Typically, blood-pressure-measurement tube 222 extends from outside the subject's body to opening 214 at the distal end of the tube, and pressure sensor 216 is disposed toward a proximal end of the tube, e.g., outside the subject's body. For some applications, computer processor 25 (Fig. 1A), receives an indication of the measured blood pressure and controls the pumping of blood by the impeller, in response to the measured blood pressure.

For some applications, the ventricular assist device includes two or more such ventricular blood-pressure-measurement tubes 222, e.g., as shown in Fig. 9A. For some applications, based upon the blood pressure as measured within each of the left ventricular blood-pressure-measurement tubes, computer processor 25 determines whether the opening of one of the two or more ventricular blood-pressure-measurement tubes is occluded. This may occur, for example, due to the opening coming into contact with the wall of the interventricular septum, and/or a different intraventricular portion. Typically, in response to determining that the opening of one of the two or more ventricular blood-pressure-measurement tubes is occluded, the computer processor determines the subject's left-ventricular pressure based upon the blood pressure measured within a different one of the two or more ventricular blood-pressure-measurement tubes.

For some applications, outer tube 142 defines a groove 215 in a portion of the outer surface of the outer tube that is configured to be disposed within tube 24. Typically, during insertion of the ventricular assist device into the subject's body, the portion of ventricular blood-pressure-measurement tube 222 that extends from within tube 24 to at least an outer surface of tube 24, is configured to be disposed within the groove, such that the portion of the ventricular blood-pressure-measurement tube does not protrude from the outer surface of the outer tube.

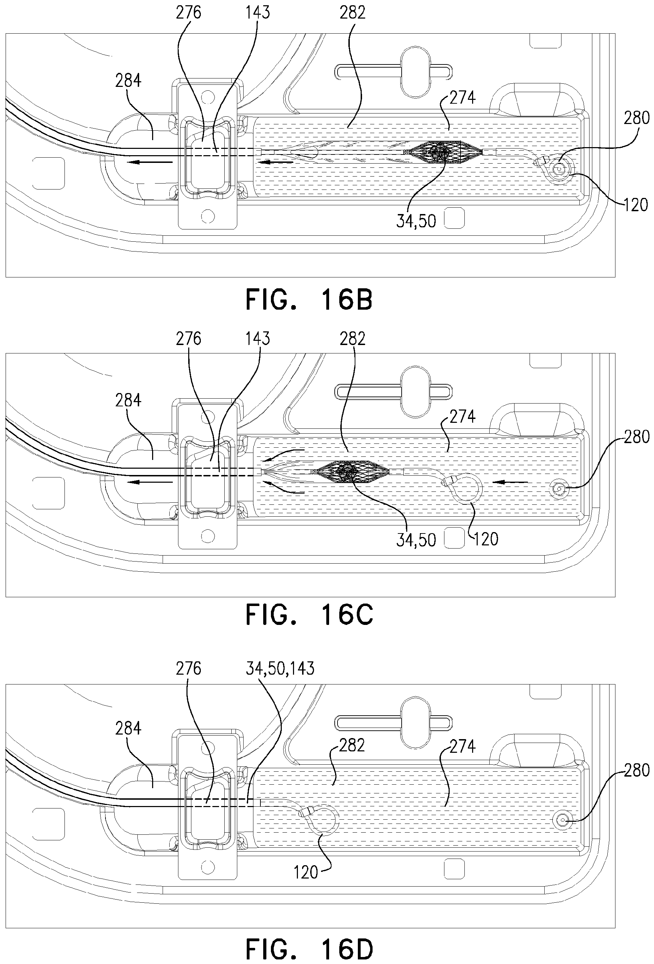

For some applications (not shown), distal portions of blood-pressure-measurement tubes 222 are disposed on the outside of pump-outlet tube 24. For example, blood-pressure-measurement tubes 222 may extend from outer tube 142 to the proximal end of pump-outlet tube 24, and thereafter the blood pressure measurement tubes may be built into the outer surface of tube pump-outlet tube 24, as shown in Fig. 16D of US 10,881,770 to Tuval, which is incorporated herein by reference, for example.

As described hereinabove, for some applications, drive cable 130 extends from a motor outside the subject's body to axial shaft 92 upon which impeller 50 is disposed. Typically, the drive cable is disposed within first outer tube 140 and second outer tube 142, as described hereinabove. For some applications, a proximal portion of blood-pressure-measurement tube 222 comprises a channel between first outer tube 140 and second outer tube 142, as shown in the cross-section of Fig. 9A. In this regard, it is noted that blood-pressure-measurement tube should be understood to refer to a continuous lumen extending from pressure sensor 216 to the outside of pump-outlet tube 24 within the subject's left ventricle, regardless of whether there are changes in the structure of the lumen along the length of the lumen. As described hereinabove, typically purging fluid is also pumped between outer tube 140 and outer tube 142, and for some applications, the purging fluid is pumped via channel 226. Typically, blood-pressure-measurement tube 222 occupies more of the cross-sectional area defined between outer tube 140 and outer tube 142 than purging fluid channel 226, as shown in Fig. 9A. For example, the ratio of (a) the cross-sectional area defined between outer tube 140 and outer tube 142 that is occupied by blood-pressure-measurement tube to (b) the cross-sectional area defined between outer tube 140 and outer tube 142 that is occupied purging fluid channel 226 is typically more than 3:2, more than 3:1, or more than 5:1. For some applications, blood-pump-measurement tube occupies a relatively large proportion of the cross-sectional area defined between outer tube 140 and outer tube 142, in order for the blood pressure outside of pump-outlet tube 24 within the subject's left ventricle to be conveyed proximally to pressure sensor 216.

Referring to Fig. 9B, for some applications, an optical fiber 228 is configured to extend to at least an outer surface 213 of tube 24, such that the distal end 230 of the optical fiber is directly exposed to the patient's bloodstream outside tube 24. Typically, the optical fiber extends from a proximal end of the fiber that is outside the subject's body (e.g., within motor unit 23) to distal end 230. Further typically, a light source and light detector (not shown) are disposed at the proximal end of the optical fiber and are configured to detect blood pressure at the distal end of the optical fiber by directing light via the optical fiber and detecting reflected light.

Typically, distal end 230 of optical fiber 228 is configured to be within the subject's left ventricle proximal to the blood pump (e.g., proximal to impeller 50). Typically, by measuring pressure of blood at distal end 230 of optical fiber 228, the pressure sensor thereby measures the subject's blood pressure outside tube 24 (i.e., left ventricular blood pressure). For some applications, computer processor 25 (Fig. 1A), receives an indication of the measured blood pressure and controls the pumping of blood by the impeller, in response to the measured blood pressure.

For some applications, the ventricular assist device includes two or more such optical fibers 228, e.g., as shown in Fig. 9B. For some applications, based upon the blood pressure as measured using each of the optical fibers, computer processor 25 determines whether the distal end of one of the optical fibers is not exposed to the left ventricular bloodstream. This may occur, for example, due to the distal end of one of the optical fibers coming into contact with the wall of the interventricular septum, and/or a different intraventricular portion. Typically, in response to determining that the distal end of the one of the optical fibers is not exposed to the left ventricular bloodstream, the computer processor determines the subject's left-ventricular pressure based upon the blood pressure measured using a different one of the two or more optical fibers 228.

For some applications, along the length of outer tube 142, the optical fibers are disposed within the outer tube. Typically, at the distal end of outer tube, the optical fibers are coupled to proximal conical portion 36 of frame 34, such that the optical fibers extend radially to the outer surface of pump-outlet tube 24. For example, as shown in Fig. 9B, the optical fibers may be sutured or tied to the proximal conical portion of frame 34, using coupling elements 232 (e.g., strings). For some applications (not shown), distal portions of optical fibers 228 are disposed on the outside of pump-outlet tube 24. For example (not shown), optical fibers 228 may extend from outer tube 142 to the proximal end of pump-outlet tube 24, and thereafter the optical fibers may be coupled to the outer surface of tube pump-outlet tube 24.

Referring to both blood-pressure measurement tube 222 and optical fiber 228, it is noted that the distal end of the tube or the fiber is typically in direct fluid communication with left-ventricular bloodstream of the subject at a location that is proximal to the proximal-most portion of blood-inlet opening(s) 108 (e.g., at least 1 cm, or at least 1.5 cm proximal to the proximal-most portion of blood-inlet opening(s) 108). Thus, the distal end of the tube or the fiber is typically exposed to blood that has a pressure that reflects the blood pressure of the left ventricle itself and that is not affected by any pressure variations that are generated in the vicinity of the blood-inlet openings as a result of fluid flow dynamics generated at the blood-inlet openings.

In accordance with the techniques described hereinabove, typically computer processor 25 is configured to determine the pressure and flow related parameters of the subject's left ventricle, e.g., using any one of the techniques described herein for determining flow through the ventricular assist device in combination with any one of the techniques described herein for determining left-ventricular pressure. For some applications, using the aforementioned parameters, the computer processor is configured to estimate the subject's native cardiac output (i.e., stroke volume), total cardiac output, arterial compliance, and/or peripheral resistance. For some applications, the aforementioned parameters are determined using a mathematical model that represents the aorta and/or the left ventricle as a dynamic vascular system.

For example, in accordance with the Windkessel model of the aorta, the relationship between flow and pressure in the aorta may be described by the following equation:

$$\frac{\partial p}{\partial t} + \frac{p}{RC} = \frac{Q}{C}\left(1 + \frac{Z_c}{R}\right) + Z_c \frac{\partial Q}{\partial t} \qquad \text{(Equation 1)}$$

where $p(t)$, $Q(t)$ are the instantaneous arterial pressure and flow rate respectively, and the variables $C$, $Z_c$, $R$ are the aortic compliance, characteristic impedance, and peripheral resistance respectively.

During operation of ventricular assist-device 20, Equation 1 can be rewritten as follows:

$$\frac{\partial(p_n + p_p)}{\partial t} + \frac{(p_n + p_p)}{RC} = \frac{(Q_n + Q_p)}{C}\left(1 + \frac{Z_c}{R}\right) + Z_c\frac{\partial(Q_n + Q_p)}{\partial t} \qquad \text{(Equation 2)}$$

where the subscripts *n, p* represent the contributions of the native heart function and the ventricular-assist-device function, respectively, to flow rate and aortic pressure.

For some applications, in a first step, the vascular parameters ($C$, $Z_c$, $R$) of the subject are estimated. In order to this, the speed at which the impeller is rotated is varied during one or more vascular-parameter determination period(s). Typically, the vascular-parameter determination period(s) is for the duration of a few cardiac cycles (e.g., 1-10, or 2-6 cardiac cycles), or during diastole only for a few cardiac cycles (e.g., 1-10, or 2-6 cardiac cycles). It is assumed that during the vascular-parameter determination period(s), the subject's parameters remain substantially unchanged. (Alternatively, any changes in the subject's vascular parameters resulting from the change in speed at which the impeller is rotated are accounted for, using a mathematical model that models such changes.)

For the two operational conditions of the ventricular assist device (i.e., when the impeller is operating at its regular operational speed, and when the impeller is rotating at the varied speed during the vascular-parameter determination period(s)), Equation 2 may be rewritten as Equations 3 and 4 respectively, where the 1 and 2 notations included in the subscript represent the first and second operational conditions.

$$\frac{\partial(p_n + p_{p1})}{\partial t} + \frac{(p_n + p_{p1})}{RC} = \frac{(Q_n + Q_{p1})}{C}\left(1 + \frac{Z_c}{R}\right) + Z_c\frac{\partial(Q_n + Q_{p1})}{\partial t} \qquad \text{(Equation 3)}$$

$$\frac{\partial(p_n + p_{p2})}{\partial t} + \frac{(p_n + p_{p2})}{RC} = \frac{(Q_n + Q_{p2})}{C}\left(1 + \frac{Z_c}{R}\right) + Z_c\frac{\partial(Q_n + Q_{p2})}{\partial t} \qquad \text{(Equation 4)}$$

By subtracting Equation 4 from Equation 3, the effect of the ventricular assist device on the vascular system may be characterized, as in Equation 5.

$$\frac{\partial(p_{p1} - p_{p2})}{\partial t} + \frac{(p_{p1} - p_{p2})}{RC}$$
$$= \frac{(Q_{p1} - Q_{p2})}{C}\left(1 + \frac{Z_c}{R}\right) + Z_c\frac{\partial(Q_{p1} - Q_{p2})}{\partial t} \qquad \text{(Equation 5)}$$

In accordance with the techniques described hereinabove, typically computer processor 25 is configured to determine the pressure and flow related parameters of the subject's left ventricle, e.g., using any one of the techniques described herein for determining flow through the ventricular assist device in combination with any one of the techniques described herein for determining left-ventricular pressure. Therefore, only the vascular parameters (aortic compliance, characteristic impedance, and peripheral resistance) are unknown. Typically, using the known values of the pressure and flow related parameters of the subject's left ventricle, the subject's vascular parameters (aortic compliance, characteristic impedance, and peripheral resistance) are estimated, e.g., using model identification techniques (such as linear regression).

For some application, subsequent to the subject's vascular parameters being estimated, the total cardiac output and the subject's native cardiac output are estimated based upon Equation 3, using the pressure and flow related parameters of the subject's left ventricle (which are typically determined in real time using the sensors described herein), as well as the subject's vascular parameters (which are determined by varying the speed at which the impeller is rotated, as described hereinabove).

For some applications, generally similar techniques to those described above are used, but using a different mathematical model to represent a dynamic vascular system of the subject (e.g., the subject's aorta, and/or the subject's left ventricle), such as to determine vascular parameters, native cardiac output, and/or alternative or additional physiological parameters. Typically, the mathematical model is of the same type as the Windkessel model, in that it accounts for the shape of the aortic waveform in terms of the interaction between the stroke volume and the compliance of the aorta.

In general, the scope of the present application includes a method in which:

a) one or more pressure-related parameters and/or flow-related parameters (e.g., the subject's aortic pressure, the subject's left ventricular pressure, and/or flow through the ventricular assist device) are determined using one or more of the sensing apparatus and methods described herein;

b) the speed at which the impeller is rotated is varied during one or more vascular-parameter determination period(s);

c) a mathematical model representing a dynamic vascular system of the subject (e.g., the subject's aorta, and/or the subject's left ventricle) is applied to the two operational conditions of the ventricular assist device (i.e., when the impeller is operating at its regular operational speed, and when the impeller is rotating at the varied speed during the vascular-parameter determination period(s));

d) based upon the difference between the mathematical model at each of the two operational conditions of the ventricular assist device, as well as the known pressure-related parameters and/or flow-related parameters, vascular parameters of the subject (e.g., aortic compliance, characteristic impedance, and peripheral resistance) are estimated;

e) based upon the known pressure-related parameters and/or flow-related parameters, and the estimated vascular parameters of the subject (e.g., aortic compliance, characteristic impedance, and peripheral resistance), the subject's native cardiac output and/or an additional cardiac parameter is estimated.

More generally, the above steps may be summarized as follows:

a) blood is pumped from the subject's left ventricle to the subject's aorta, using the percutaneous left ventricular assist device;

b) one or more pressure-related parameters and/or flow-related parameters are detected using the percutaneous left ventricular assist device (e.g., using the apparatus and methods for sensing described herein);

c) a rate at which blood is pumped by the percutaneous left ventricular assist device is varied during one or more vascular-parameter determination period(s), and a mathematical model representing a dynamic vascular system of the subject is applied to when the percutaneous left ventricular assist device is pumping blood at respective rates;

d) based upon a difference between the mathematical model as applied when the percutaneous left ventricular assist device is pumping blood at the respective rates, as well as the pressure-related parameters and/or flow-related parameters, vascular parameters of the subject are estimated;

e) based upon the pressure-related parameters and/or flow-related parameters, and the estimated vascular parameters of the subject, the subject's native cardiac output and/or an additional cardiac parameter is estimated.

The above-described steps are typically repeated during the operation of the ventricular assist device as often as needed.

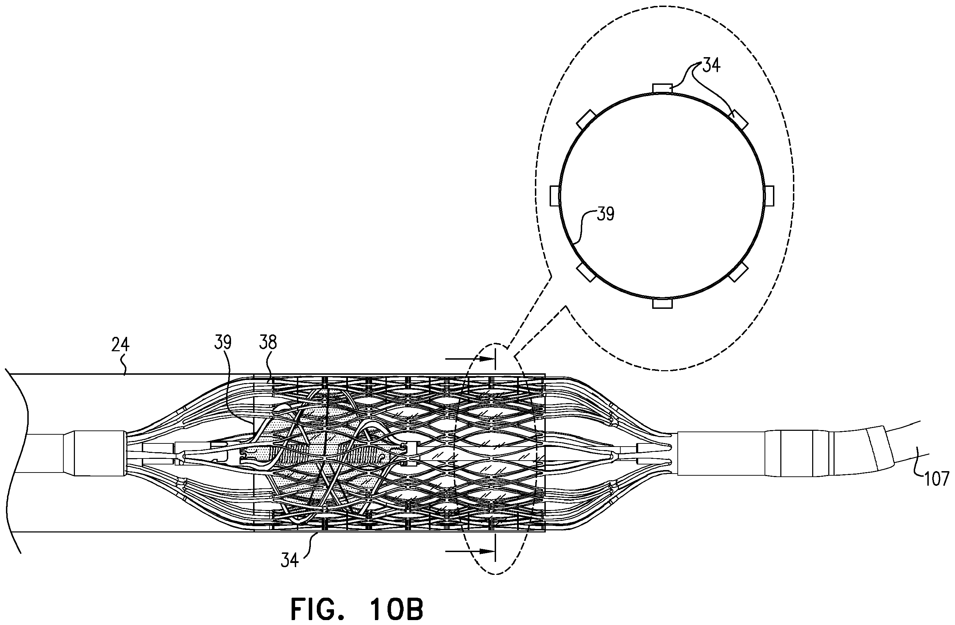

Reference is now made to Figs. 10A and 10B, which are schematic illustrations of ventricular assist device 20, the device including inner lining 39 that lines the inside of frame 34 that houses impeller 50, in accordance with some applications of the present invention. For some applications, inner lining 39 is disposed inside frame 34, in order to provide a smooth inner surface (e.g., a smooth inner surface having a substantially circular cross-sectional shape) through which blood is pumped by impeller. Typically, by providing a smooth surface, the covering material reduces hemolysis that is caused by the pumping of blood by the impeller, relative to if the blood were pumped between the impeller and struts of frame 34. For some applications, inner lining includes polyurethane, polyester, and/or silicone. Alternatively or additionally, the inner lining includes polyethylene terephthalate (PET) and/or polyether block amide (PEBAX®).

Typically, the inner lining is disposed over the inner surface of at least a portion of central cylindrical portion 38 of frame 34. For some applications, pump-outlet tube 24 also covers central cylindrical portion 38 of frame 34 around the outside of the frame, for example, such that pump-outlet tube 24 and inner lining 39 overlap over at least 50 percent of the length of the inner lining, for example, over the entire length of the cylindrical portion of frame 34, e.g., as shown in Fig. 10A. For some applications, there is only partial overlap between pump-outlet tube 24 and inner lining 39, e.g., as shown in Fig. 10B. For example, pump-outlet tube 24 may overlap with inner lining along less than 50 percent (e.g., along less than 25 percent) of the length of the inner lining. For some such applications, during insertion of ventricular assist device 20 into the subject's body, the impeller is advanced distally within frame 34, such that the impeller is not disposed within the area of overlap between the pump-outlet tube and the inner lining, such that there is no longitudinal location at which the impeller, pump-outlet tube 24, frame 34, and inner lining 39 all overlap with each other. As shown in Figs. 10A and 10B, for some applications a single axially-facing blood inlet opening 108 is defined at the distal end of the pump-outlet tube and/or the inner lining. Alternatively, the inner lining is disposed over the inner surface of at least a portion of central cylindrical portion 38 of frame 34, and the pump-outlet tube extends to the distal end of the frame and defines a plurality of lateral blood-inlet openings 108. Such applications are described in further detail hereinbelow with reference to Figs. 11A-E, for example.

Typically, over the area of overlap between inner lining 39 and pump-outlet tube 24, the inner lining is shaped to form a smooth surface (e.g., in order to reduce hemolysis, as described hereinabove), and pump-outlet tube 24 is shaped to conform with the struts of frame 34 (e.g., as shown in the cross-section in Fig. 10A). Further typically, the inner lining has a substantially circular cross-section (for example, due to the relatively small cell width within the central cylindrical portion of the frame, as described hereinabove, with reference to Fig. 2). For some applications, over the area of overlap between inner lining 39 and pump-outlet tube 24, the pump-outlet tube and the inner lining are coupled to each other, e.g., via vacuum, via an adhesive, and/or using a thermoforming procedure.

For some applications, inner lining 39 and pump-outlet tube 24 are made of different materials from each other. For example, the inner lining may be made of polyurethane, and the pump-outlet tube may be made of polyether block amide (PEBAX®). Typically, for such applications, the material from which the inner lining is made has a higher thermoforming temperature than that of the material from which the pump-outlet tube is made. Alternatively, inner lining 39 and pump-outlet tube 24 are made of the same material as each other. For example, the both the inner lining and the pump-outlet tube may be made of may be made of polyurethane or polyether block amide (PEBAX®).

For some applications, the pump-outlet tube and the inner lining are bonded to each other and/or the frame in the following manner. For some applications, the inner lining is directly bonded to the inner surface of the frame, before the pump-outlet tube is bonded to the outside of the frame. It is noted that, by bonding the inner lining directly to the inner surface of the frame, (rather than simply bonding the inner lining to the pump-outlet tube and thereby sandwiching the frame between the inner lining to the pump-outlet tube), any air bubbles, folds, and other discontinuities in the smoothness of the surface provided by the inner lining are typically avoided. For some applications, similar techniques to those described hereinabove, for enhancing bonding between the elastomeric film and the helical elongate elements of the impeller, are used to enhance bonding between the inner lining and the inner surface of the frame. For some applications, initially, the frame is treated so as to enhance bonding between the inner lining and the inner surface of the frame. For some applications, the treatment of the frame includes applying a plasma treatment to the frame (e.g., to the inner surface of the frame), dipping the frame in a coupling agent that has at least two functional groups that are configured to bond respectively with the frame and with the material form which the inner lining is made (e.g., silane solution), and/or dipping the frame in a solution that contains the material from which the inner lining is made (e.g., polyurethane solution). For some applications, the inner lining is made of an elastomeric material (e.g., polyurethane) and the coupling agent is a silane solution, such as a solution of n-(2-aminoethyl)-3-aminopropyltrimethoxysilane, with the silane containing a first functional group (e.g., (OH)) which is configured to bond with the frame (which is typically made of an alloy, such a nitinol), and the silane containing a second functional group (e.g., (NH2)) which is configured to bond with the elastomeric material.

For some applications, subsequently, a solution that contains the material from which the inner lining is made (e.g., polyurethane solution) is sprayed over the central cylindrical portion of the frame. Once the inner surface of the frame has been treated, the inner lining is bonded to the inner surface of the central cylindrical portion of the frame (e.g., to the inner surface of a central cylindrical portion of the frame). Typically, the inner lining (which is shaped as a tube), is placed over a mandrel, the frame is placed over the inner lining, and pressure is applied by a heat shrinking process. Further typically, the assembly of the inner lining and the frame is heated in an oven.

Subsequent to the inner lining having been bonded to the frame, a portion of pump-outlet tube 24 is placed around the outside of the frame. As described above, for some applications, inner lining 39 and pump-outlet tube 24 are made of different materials from each other. For example, the inner lining may be made of polyurethane, and the pump-outlet tube may be made of polyether block amide (PEBAX®). Typically, for such applications, the material from which the inner lining is made has a higher thermoforming temperature than that of the material from which the pump-outlet tube is made. For some applications, in order to mold pump-outlet tube 24 to conform with the struts of frame 34, without causing the inner lining to deform, the frame is heated to a temperature that is above the thermoforming temperature of pump-outlet tube 24 but below the thermoforming temperature of inner lining 39.

Typically, the frame is heated from inside the frame, using the mandrel. Typically, while the frame is heated to the aforementioned temperature, an outer tube (which is typically made from silicone) applies pressure to pump-outlet tube 24 that causes pump-outlet tube 24 to be pushed radially inwardly, in order to cause the pump-outlet tube to conform with the shapes of the struts of the frame, as shown in the cross-section of Fig. 10A. For some applications, during this stage, the mandrel that is placed inside the inner lining and which heats the inner lining is shorter than the length of the inner lining. The mandrel is typically placed within the inner lining such that margins are left outside of the mandrel at each of the ends of the inner lining. Typically, the inner lining acts as a shield to protect the pump-outlet tube from being overheated and becoming damaged by the heating of the mandrel. Placing the inner lining on the mandrel in the aforementioned manner prevents the mandrel from coming into direct contact with the frame and/or the pump-outlet tube. For some applications, the combination of the frame, the inner lining, and the portion of pump-outlet tube 24 disposed around the frame is subsequently shape set to a desired shape and dimensions using shape setting techniques that are known in the art.

Reference is now made to Figs. 11A-E, which are schematic illustrations of pump-outlet tube 24 or a portion thereof, the pump-outlet tube being configured to define lateral blood-inlet openings 108 at a distal end thereof, in accordance with some applications of the present invention. For some applications, the pump-outlet tube extends substantially until the distal end of distal conical portion 40 of frame 34. For such applications, the pump-outlet tube typically defines a distal conical portion 46 which is distally facing, i.e., facing such that the narrow end of the cone is distal with respect to the wide end of the cone. Typically, the pump-outlet tube includes coupling portion 41 (e.g., a tubular coupling portion, as shown), which extends distally from the pump-outlet tube. As described hereinabove, the coupling portion is coupled to the distal bearing housing in order to anchor the distal end of the pump-outlet tube.

For some applications (not shown), the pump-outlet tube defines two to four lateral blood-inlet openings. Typically, for such applications, each of the blood-inlet openings defines an area of more than 20 square mm (e.g., more than 30 square mm), and/or less than 60 square mm (e.g., less than 50 square mm), e.g., 20-60 square mm, or 30-50 square mm. Alternatively or additionally, the outlet tube defines a greater number of smaller blood-inlet openings 108, e.g., more than 10 blood-inlet openings, more than 50 blood-inlet openings, more than 100 blood-inlet openings, or more than 150 blood-inlet openings, e.g., 50-100 blood-inlet openings, 100-150 blood-inlet openings, or 150-200 blood-inlet openings. For some applications, the blood-inlet openings are sized such as (a) to allow blood to flow from the subject's left ventricle into the tube and (b) to block structures from the subject's left ventricle from entering into the frame. Typically, for such applications, the distal conical portion 46 of pump-outlet tube 24 is configured to reduce a risk of structures from the left ventricle (such as chordae tendineae, trabeculae carneae, and/or papillary muscles) entering into frame 34 and potentially being damaged by the impeller and/or the axial shaft, and/or causing damage to the left ventricular assist device. Therefore, for some applications, the blood-inlet openings are shaped such that, in at least one direction, the widths (or spans) of the openings are less than 1 mm, e.g., 0.1-1 mm, or 0.3-0.8 mm. By defining such a small width (or span), it is typically the case that structures from the left ventricle (such as chordae tendineae, trabeculae carneae, and/or papillary muscles) are blocked from entering into frame 34. For some such applications, each of the blood-inlet openings defines an area of more than 0.05 square mm (e.g., more than 0.1 square mm), and/or less than 3 square mm (e.g., less than 1 square mm), e.g., 0.05-3 square mm, or 0.1-1 square mm. Alternatively, each of the blood-inlet openings defines an area of more than 0.1 square mm (e.g., more than 0.3 square mm), and/or less than 5 square mm (e.g., less than 1 square mm), e.g., 0.1-5 square mm, or 0.3-1 square mm.

Typically, the portion of the pump-outlet tube that defines the blood-inlet openings has a porosity of more than 40 percent, e.g., more than 50 percent, or more than 60 percent (where porosity is defined as the percentage of the area of this portion that is porous to blood flow). Thus, on the one hand, the blood-inlet openings are relatively small (in order to prevent structures of the left ventricular from entering the frame), but on the other hand, the porosity of the portion of the pump-outlet tube that defines the blood-inlet openings is relatively high, such as to allow sufficient blood flow into the pump-outlet tube.

For some applications, each of the blood-inlet openings has a circular or a polygonal shape. For some applications, each of the blood-inlet openings has a hexagonal shape, as shown in Figs. 11A-D. Typically, using openings having a hexagonal shape allows the portion of the pump-outlet tube that defines the blood-inlet openings to have a relatively high porosity (e.g., as described hereinabove), while providing the portion of the pump-outlet tube that defines the blood-inlet openings with sufficient material between the blood-inlet openings to prevent tearing and/or stretching of the material. As shown in Fig. 11B, for some applications, a width W of gaps between adjacent hexagonal (or other polygonal) holes is more than 0.01 mm (e.g., more than 0.04 mm), and/or less than 0.1 mm (e.g., less than 0.08 mm), for example, 0.01-0.1 mm, or 0.04-0.08 mm. For some applications, the distance D between opposing sides of each of the hexagons (or other types of polygons) is more than 0.2 mm (e.g., more than 0.4 mm) and/or less than 0.8 mm (e.g., less than 0.6 mm), e.g., 0.2-0.8 mm, or 0.4-0.6 mm. As indicated in Fig. 11B, typically each of the polygons encloses a circle (such that any structure that cannot pass through such a circle would be unable to pass through the polygon). Typically, the diameter of the circle enclosed by the polygon is the equivalent of distance D, e.g., more than 0.2 mm (e.g., more than 0.4 mm) and/or less than 0.8 mm (e.g., less than 0.6 mm), e.g., 0.2 - 0.8 mm, or 0.4 - 0.6 mm.

Fig. 11D shows a segment of distal conical portion 46 of pump-outlet tube 24, in accordance with some applications of the present invention. In the view shown in Fig. 11D, the segment is laid out flat for illustrative purposes. As shown in Fig. 11D, for some applications, within a proximal region 46P of distal conical portion 46 of pump-outlet tube 24, the widths W1 of the gaps between the hexagonal (or other type of polygonal) holes are larger than widths W of the gaps between the hexagonal (or other type of polygonal) holes within a distal region 46D of distal conical portion 46 of the pump-outlet tube. For some applications, the ratio of the widths of gaps between adjacent blood-inlet openings with the proximal region of the distal portion of the pump-outlet tube to the widths of gaps between adjacent blood-inlet openings within the distal region of the distal portion of the pump-outlet tube is greater than 3:2, e.g., between 3:2 and 5:2. Typically, for such applications, within proximal region 46P of distal conical portion 46 of pump-outlet tube 24, a distance D1 between opposing sides of each of the hexagons (or other type of polygons) is smaller than distance D between opposing sides of each of the hexagons (or other type of polygons) within distal region 46D of distal conical portion 46 of the pump-outlet tube. (As described hereinabove, typically, distances D and D1 also represent the diameter of a circle that is enclosed by the respectively sized polygons.) For some applications, the ratio of the diameter of a circle enclosed by each of the blood-inlet openings with the distal region of the distal portion of the pump-outlet tube to a diameter of a circle enclosed by each of the blood-inlet openings with the proximal region of the distal portion of the pump-outlet tube is greater than 7:6, e.g., between 7:6 and 4:3. Further typically, within distal region 46D, the distal conical portion of pump-outlet tube 24, has a higher porosity than within proximal region 46P of the distal conical portion 46 of the pump-outlet tube. For example, the ratio of the porosity within distal region 46D to the porosity within proximal region 46P is more than 4:3, or more than 3:2. For some applications, the proximal region extends along a length of more than 0.5 mm, and/or less than 2 mm (e.g., less than 1.5 mm), for example, between 0.5 - 2 mm or 0.5 - 1.5 mm. For some applications, the total length of the distal conical portion is more than 6 mm and/or or less than 12 mm (e.g., less than 10 mm), for example between 6-12 mm, or 6 - 10 mm.

As described hereinabove with reference to Figs. 9A-B, typically, the pump-outlet tube is coupled to frame 34 via heating. For some applications, within the proximal region 46P of distal conical portion 46 of pump-outlet tube 24, the gaps between the blood-inlet holes are wider and/or the blood-inlet holes are smaller than within distal region 46D, and/or the porosity is lower than within distal region 46D, in order to prevent and/or reduce damage (e.g., tearing, thinning, and/or stretching) that may be caused to the material that defines the blood-inlet holes from being damaged during the above-described heating process. For some applications, as a result of the above-described heating process being applied, any difference between the sizes of the gaps between the blood-inlet holes and/or the sizes of the blood-inlet holes themselves and/or porosity between distal region 46D and proximal region 46P is reduced or even removed.

Typically, width W of the gaps between the hexagonal (or other type of polygonal) holes and distance D between opposing sides of each of the hexagons (or other type of polygons) within distal region 46D of distal conical portion 46 of the pump-outlet tube are as described hereinabove. For some applications, width W1 of gaps between adjacent hexagonal (or other polygonal) holes within proximal region 46P of distal conical portion 46 of pump-outlet tube 24 is more than 0.05 mm (e.g., more than 0.07 mm), and/or less than 0.2 mm (e.g., less than 0.15 mm), for example, 0.05 - 0.2 mm, or 0.07 - 0.15 mm. For some applications, distance D1 between opposing sides of each of the hexagons (or other types of polygons) within proximal region 46P of distal conical portion 46 of pump-outlet tube 24 is more than 0.1 mm (e.g., more than 0.3 mm) and/or less than 0.6 mm (e.g., less than 0.5 mm), e.g., 0.1-0.6 mm, or 0.3-0.5 mm.

The scope of the present disclosure includes having non-uniformly sized and/or shaped lateral blood-inlet openings (e.g., circular, rectangular, polygonal, and/or hexagonal lateral blood-inlet openings), disposed in any arrangement along the distal conical portion 46 of the pump-outlet tube. Similarly, the scope of the present disclosure includes a distal conical portion 46 of the pump-outlet tube that defines lateral blood-inlet openings being arranged such that the distal conical portion has a non-uniform porosity, with the porosity varying over different regions of the distal conical portion. For some applications, the shapes and/or sizes of the lateral blood-inlet openings, and/or the porosity of the distal conical portion, is varied such as to account for varying blood flow dynamics at different regions of the distal conical portion. Alternatively or additionally, the shapes and/or sizes of the lateral blood-inlet openings, and/or the porosity of the distal conical portion, is varied such as to account for changes in the shape of the distal conical portion along its length.

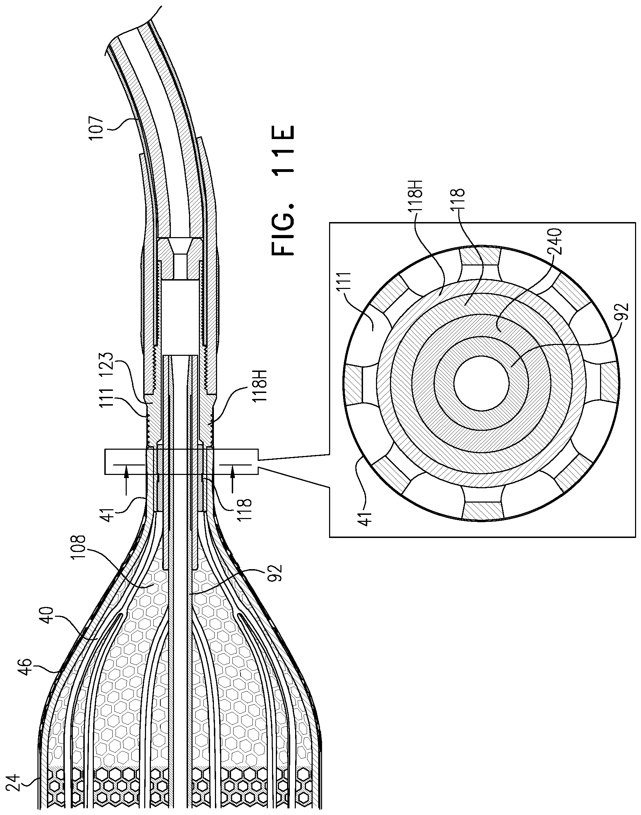

Reference is now made to Fig. 11E, which is an enlarged schematic illustration of the interface between the distal end of pump-outlet tube 24 and distal-tip element 107. Typically, the pump-outlet tube includes a coupling portion 41 (e.g., a tubular coupling portion, as shown), which extends distally from the pump-outlet tube. As described hereinabove, the coupling portion is coupled to distal bearing housing 118H in order to anchor the distal end of the pump-outlet tube. Also as described hereinabove, typically, the pump-outlet tube is coupled to the outside of the central cylindrical portion of the frame. For some applications, distal conical portion 46 of the pump-outlet tube is not itself bonded to distal conical portion 40 of the frame. Rather, distal conical portion 46 of the pump-outlet tube is held in place with respect to distal conical portion 40 of the frame, by virtue of coupling portion 41 being coupled to distal bearing housing 118H and the pump-outlet tube being coupled to the outside of the central cylindrical portion of the frame. Alternatively, the distal conical portion 46 of the pump-outlet tube is directly coupled to distal conical portion 40 of the frame (e.g., via heat shrinking).

As described hereinabove, for some applications, coupling portion 41 is coupled to the outer surface of portion 123 of distal bearing housing 118H. For some applications, coupling portion 41 defines a hole 111 (e.g., toward the distal end of the coupling portion), as shown in Fig. 11E. For some applications, adhesive is applied between coupling portion 41 and the outer surface of portion 123 of distal bearing housing 118H, via the hole. For some application, the outer surface of portion 123 of distal bearing housing 118H is threaded. Typically, the threaded outer surface allows the adhesive to gradually and uniformly spread between coupling portion 41 and the outer surface of portion 123 of distal bearing housing 118H. Further typically, the coupling portion is transparent, such that the spread of the adhesive is visible through the coupling portion. Therefore, for some applications, once the adhesive has sufficiently spread between coupling portion 41 and the outer surface of portion 123 of distal bearing housing 118H (e.g., once the outer surface of portion 123 has been covered with the adhesive), application of the adhesive is terminated.

Reference is now made to Figs. 12A, 12B, and 12C, which are schematic illustrations of drive cable 130 of ventricular assist device 20, in accordance with some applications of the present invention. Typically, the rotational motion of the of the motor is transmitted to the axial shaft via the drive cable. Typically, the drive cable extends from motor unit 23 (which is typically disposed outside the subject's body) to the proximal end of axial shaft 92 (with the connection between the distal end of the drive cable and the proximal end of the axial shaft being shown in one of the enlarged portions of Fig. 5A for example). For some applications, the drive cable includes a plurality of wires 134 that are disposed in a coiled configuration in order to impart sufficient strength and flexibility to the drive cable, such that a portion of the cable is able to be maintained within the aortic arch, while the cable is rotating and moving in the axial back-and-forth motion. For some applications, the drive cable includes a plurality of coaxial layers of coiled wires. For example, as shown in Figs. 12A-C, the drive cable may include an outer layer 136 and an inner layer 138, which are coaxial with each other and each of which comprises coiled wires.

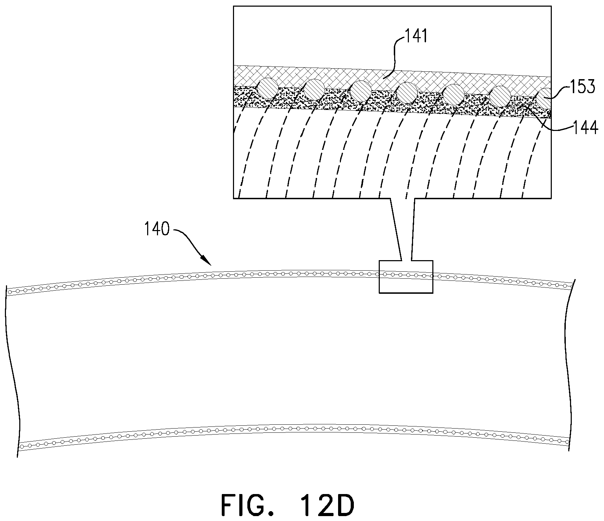

The drive cable is typically disposed within a first outer tube 140, which is configured to remain stationary while the drive cable undergoes rotational and/or axial back-and-forth motion. The first outer tube is configured to effectively act as a bearing tube for the drive cable, along the length of the drive cable. As such, first outer tube is also referred to herein as the drive-cable bearing tube. The drive-cable bearing tube is described in further detail hereinbelow with reference to Fig. 12D. For some applications, the drive-cable bearing tube is disposed within a second outer tube 142, which is typically made of a material having greater flexibility than that of the drive-cable bearing tube (e.g., nylon, and/or polyether block amide), and typically has a thickness that is greater than that of the drive cable bearing tube.

Typically, during insertion of the impeller and the frame into the left ventricle, impeller 50 and frame 34 are maintained in radially-constrained configurations by delivery catheter 143. As described hereinabove, in order for the impeller and the frame to assume non-radially-constrained configurations, the delivery catheter is retracted. For some applications, as shown in Fig. 12A, the delivery catheter remains in the subject's aorta during operation of the left ventricular device, and outer tube 142 is disposed inside the delivery catheter. (Although Fig. 12A shows the distal end of the delivery catheter disposed within the aortic arch, for some application, the distal end of the delivery catheter is disposed within the descending aorta during operation of the left ventricular device.) For some applications, during operation of the left ventricular device, a channel 224 is defined between delivery catheter 143 and outer tube 142. (It is noted that the channel as shown in Fig. 12A is not to scale, for illustrative purposes.) For some such applications, the subject's aortic blood pressure is measured by measuring the pressure of blood within channel 224. For example, pressure sensor 216 (illustrated schematically in Fig. 1A) may be in fluid communication with channel 224, and may be configured to measure the subject's aortic pressure by measuring the pressure of blood within channel 224. Typically, in order to retract the left ventricular device from the subject's body, the delivery catheter is advanced over the impeller and the frame, such that the impeller and the frame assume their radially-constrained configurations. The catheter is then withdrawn from the subject's body.

Reference is now made to Fig. 12D, which is a schematic illustration of first outer tube 140, which acts as a drive-cable bearing tube, in accordance with some applications of the present invention. For some applications, the drive-cable bearing tube includes an outer layer 141 and inner layer 144, each of which is typically made of a biocompatible polymeric material, and a coil 153 embedded between the outer and inner layers. For some applications, outer layer 141 is made of Pebax, inner layer 144 is made of PTFE and/or polyimide (e.g., a mixture of PTFE and/or polyimide), and the coil is made of an alloy, such as stainless steel. Typically, the inner layer includes materials that are configured to provide low levels of friction and high wear resistance. Further typically, the outer layer is configured to provide additional strength to the drive-cable bearing tube, while still providing the drive-cable bearing tube with sufficient flexibility that it is able to conform with the curvature of the aortic arch, for example. Typically, the coil is configured such as to maintain a substantially circular cross-section for the drive-cable bearing tube, even within regions in which the drive-cable bearing tube undergoes a substantial curve (e.g., within the aortic arch). Typically, in the absence of the coil, the drive-cable bearing tube would have a tendency to flatten and form an elliptical cross-section within such regions.

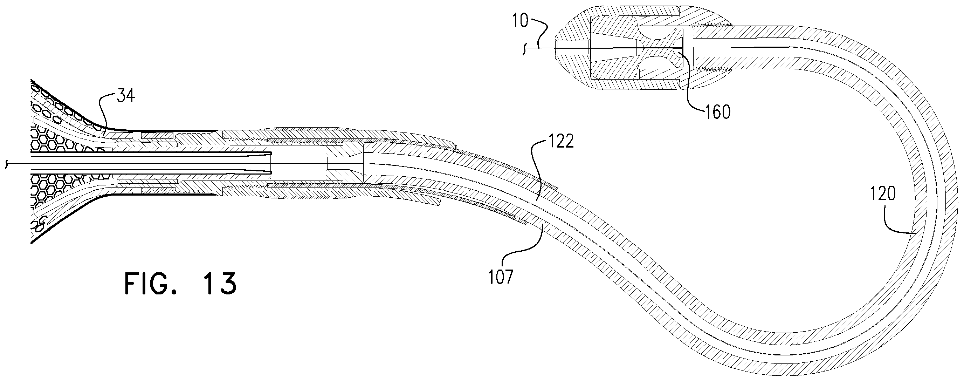

Reference is now made to Fig. 13, which is a schematic illustration of a ventricular assist device with guidewire 10 disposed within guidewire lumen 122, in accordance with some applications of the present invention. For some such applications, during insertion of the ventricular assist device into the left ventricle, guidewire 10 is first inserted into the left ventricle, for example, in accordance with known techniques. The distal-tip portion of the ventricular assist device is then guided to the left ventricle by advancing the distal-tip portion over the guidewire, with the guidewire disposed inside lumen 122. For some applications, a duckbill valve 390 (or a different type of hemostasis valve) is disposed at the distal end of lumen 122 of distal-tip portion 120.

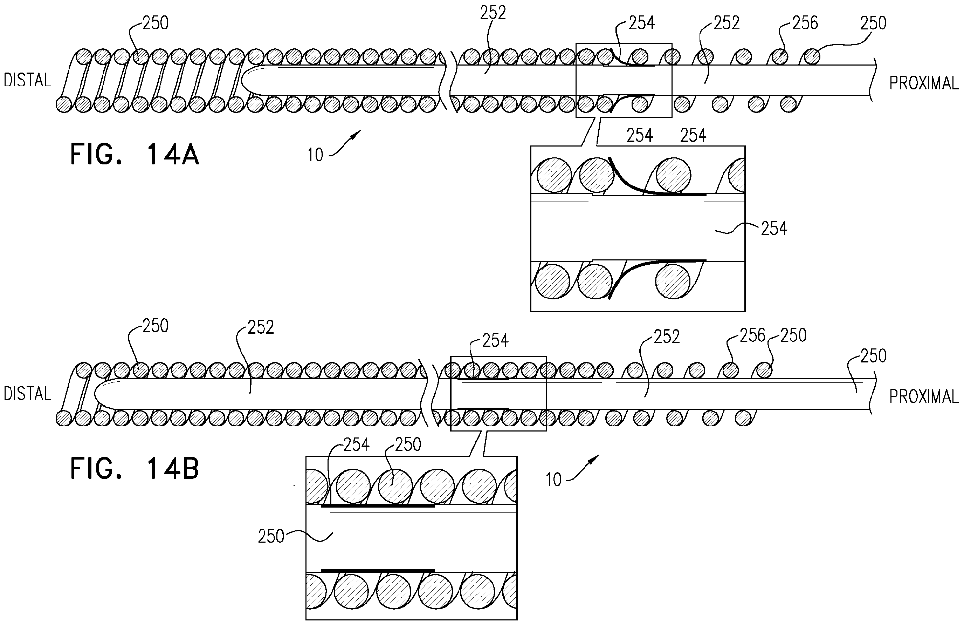

Reference is now made to Figs. 14A and 14B, which are schematic illustrations of guidewire 10, the guidewire being configured to have two states, in accordance with some applications of the present invention. As described with reference to Fig. 13, typically, the distal end of the ventricular assist device is guided to the left ventricle over guidewire 10. Typically, during the advancement of the guidewire through the subject's vasculature and when the tip of the guidewire is disposed within the subject's left ventricle, it is desirable for the tip of the guidewire to be flexible, in order to avoid injury to the subject's vasculature and/or left ventricle.

Subsequent to the distal end of the ventricular assist device being guided to the left ventricle, impeller 50 and frame 34 of the ventricular assist device are deployed, by retracting the delivery catheter, which typically causes the impeller and frame to assume non-radially-constrained configurations. Typically, at this stage, the tip of the guidewire is retracted proximally through the distal tip portion 120 and is retracted proximally from the pump portion. In some cases, after the guidewire has been retracted in the above-described manner, it is desirable to reinsert the guidewire through distal tip portion 120, and out of duckbill valve 160, which is disposed toward a distal end of the distal tip portion. For example, this may be desirable if the pump portion migrates from its correct position and requires repositioning within the left ventricle. However, if the tip of the guidewire is still flexible (as described above), it is not possible to advance the tip of the guidewire through the narrow tip of the proximally-facing duckbill valve 160. Therefore, for some applications, the guidewire is configured such that its tip can define two states: a flexible state, and a stiff state.

For some applications, guidewire 10 includes an outer coil 250 and an inner stiffening wire 252. When the distal end of the guidewire should be in a flexible state, the inner stiffening wire is retracted from the distal end of the guidewire, such that there is a distal portion of the outer coil that does not have the stiffening wire disposed therein. For some applications, the guidewire is releasably locked into this state, in order to prevent the distal end of the guidewire from becoming rigid and damaging the subject's vasculature and/or left ventricle. For example, as shown in Fig. 14A, for some applications, one or more locking elements 254 extend radially from a proximal portion of the stiffening wire and protrude between windings of the outer coil, such as to prevent the stiffening wire from being advanced. For some applications, at the proximal end 256 of the outer coil, there are larger gaps between the windings of the coil than at portions of the outer coil that are more distal, such that the locking elements protrude between windings of the outer coil at the proximal end. For some applications, in order to convert the distal end of the guidewire to a rigid state, locking elements 254 are pushed radially inwardly such that they are disposed within the outer coil, and the stiffening wire is advanced distally, such that the stiffening wire becomes disposed within the outer coil even at the distal end of the guidewire. For some applications, the locking elements remain disposed within the outer coil as the stiffening wire is advanced distally because the locking elements are now disposed within a portion of the outer coil at which the windings of the coil are disposed sufficiently close to each other that they block the locking elements from protruding from between them.

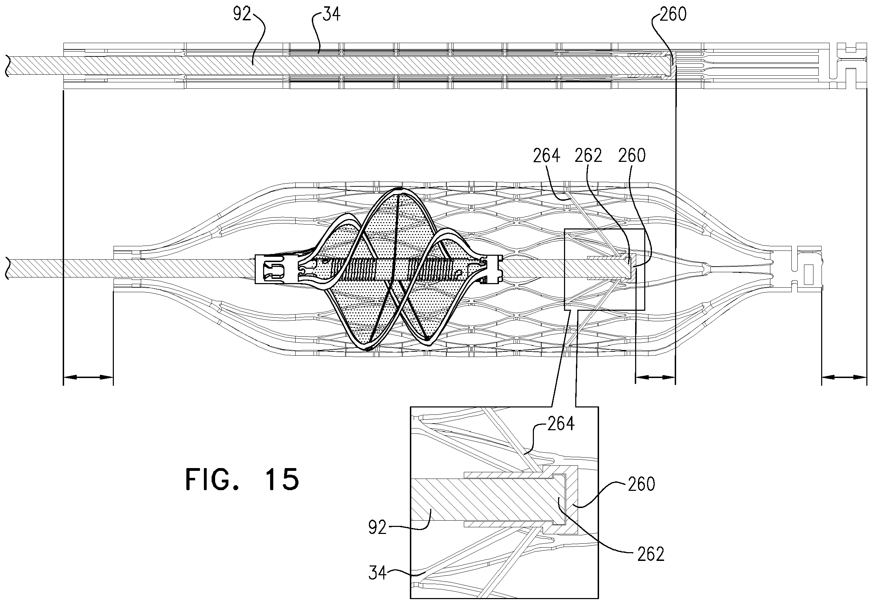

Reference is now made to Fig. 15, which is a schematic illustration of a ventricular assist device 20, the ventricular assist device including a distal thrust bearing 260, in accordance with some applications of the present invention. Typically, bearing 260 functions as both a distal radial bearing and a distal thrust bearing. The upper view of the ventricular device shown in Fig. 15 shows the device in its radially-constrained (i.e., crimped) configuration, while the lower view shows the device in its non-radially constrained configuration, with the arrows representing the movement of respective portions of the device between these two configurations. Some applications of the present invention are described hereinabove as being directed toward a ventricular assist device that does not include any thrust bearing disposed within the subject's body and that is configured to allow axial back-and-forth motion of impeller 50 and axial shaft 92. For some alternative applications, the ventricular assist device does include a thrust bearing that is configured to prevent axial shaft 92 from undergoing axial motion in response to variations in the pressure gradient against which the impeller is pumping (and, typically, to thereby prevent the impeller from undergoing axial motion in response to variations in the pressure gradient against which the impeller is pumping).

For some applications, thrust bearing 260 is disposed within frame 34 as shown. For example, the thrust bearing may be disposed within the cylindrical portion of the frame or within the distal conical portion of the frame. For some applications at a distal end of axial shaft, the axial shaft defines a widened portion 262 that is configured to engage the thrust bearing and to prevent the axial shaft (and thereby prevent the impeller) from undergoing axial motion. (In addition, the widened portion of the axial shaft is constrained radially by bearing 260, such that the bearing also functions as a distal radial bearing.) Typically, the thrust bearing is coupled to the frame via connecting struts 264, which extend radially inwardly from the frame to the thrust bearing. Typically, in order to manufacture frame 34, the frame is cut from a tube of a shape-memory alloy, such as nitinol. For some applications, connecting struts 264 are cut from the tube from which the frame is cut, such that the frame and the connecting struts form a single integral body, without requiring coupling to each other (e.g., via adhesive, welding, etc.). In general, for some applications, the frame and the connecting struts are cut from a single piece of a material such as to form a single integral body. For some applications, connecting struts 264 as well as thrust bearing 260 itself are cut from the tube from which the frame is cut, such that the frame, the connecting struts, and the thrust bearing form a single integral body, without requiring coupling to each other (e.g., via adhesive, welding, etc.). In general, for some applications, the frame, the connecting struts, and thrust bearing 260 itself are cut from a single piece of a material such as to form a single integral body.

For some applications, a spring that is generally similar to motion-cushioning spring 68 (shown in Figs. 6C-D) is used in combination with thrust bearing 260 (or with a differently designed thrust bearing that is disposed distally of the impeller). For some applications, the spring assists in stabilizing the impeller (e.g., the distal end of the impeller) with respect to the thrust bearing, subsequent to the radial expansion of the impeller. Thus, the spring functions as an impeller-stabilizing spring. For some applications, the impeller is configured to be radially constrained (i.e., crimped) by becoming axially elongated, and the spring is configured to become compressed such as to accommodate the axial elongation of the impeller. Typically, when the impeller is in a radially-constrained configuration during insertion of the pump head into the left ventricle, the impeller is axially elongated such that the distal end of the impeller is disposed distally within frame 34 and the impeller-stabilizing spring is compressed in order to accommodate the movement of the distal end of the impeller.

Typically, the impeller-stabilizing spring is disposed around axial shaft 92 between the distal end of the impeller (e.g., distal bushing 58 of the impeller) and thrust bearing 260. For some applications, the impeller-stabilizing spring is coupled to thrust bearing 260 and extends proximally over axial shaft 92 from thrust bearing 260. Typically, in such cases, the impeller-stabilizing spring remains rotationally stationary as the impeller rotates, and the impeller is configured to rotate with respect to the impeller-stabilizing spring. Alternatively or additionally, the impeller-stabilizing spring is coupled to the distal end of the impeller (e.g., distal bushing 58 of the impeller) and/or extends distally over axial shaft 92 from the distal end of the impeller (e.g., distal bushing 58 of the impeller). For some such applications, the impeller-stabilizing spring is configured to rotate together with the impeller. Alternatively, the impeller-stabilizing spring extends from a radial bearing that is disposed around the distal end of the impeller (e.g., distal bushing 58 of the impeller), such that impeller-stabilizing spring remains rotationally stationary as the impeller rotates, and the impeller is configured to rotate with respect to the impeller-stabilizing spring. For some applications, the impeller-stabilizing spring is coupled to (e.g., coated with or embedded within) elastomeric material 69 (also shown in Figs. 6C-D), with the combination of the elastomeric material and the spring typically being formed and functioning in a generally similar manner to that described hereinabove with reference to motion-cushioning spring 68.

For some applications, an impeller-stabilizing spring is disposed around the axial shaft on a proximal side of the impeller, e.g., as described hereinabove with reference to Figs. 6C-D. For some applications, an impeller-stabilizing spring is disposed around the axial shaft on both proximal and distal sides of the impeller. For some applications, the combination of proximal and distal impeller-stabilizing springs disposed around the axial shaft function to hold the impeller in place axially and/or limit axial movement of the impeller, when the pump head is expanded.

For some applications, thrust bearing 260 is used in combination with a pump-outlet tube 24 that is configured as shown in, and described with reference to, Figs. 11A-E, with the pump-outlet tube defining lateral blood-inlet openings 108 at a distal end thereof. As described hereinabove, for some applications, the blood-inlet openings are sized such as (a) to allow blood to flow from the subject's left ventricle into the tube and (b) to block structures from the subject's left ventricle from entering into the frame. Typically, for such applications, the distal conical portion 46 of pump-outlet tube 24 is configured to reduce a risk of structures from the left ventricle (such as chordae tendineae, trabeculae carneae, and/or papillary muscles) entering into frame 34 and potentially being damaged by the impeller and/or the axial shaft, and/or causing damage to the left ventricular assist device. For such applications, the dimensions and other characteristics of the pump-outlet tube and the blood-inlet openings are typically generally as described with reference to Figs. 11A-E.

As shown in Fig. 15, when thrust bearing 260 is disposed within frame 34, axial shaft 92 does not extend to the distal end of frame 34. For example, as shown in Fig. 5, in the non-radially-constrained configuration of the frame, the distal end of the axial shaft is disposed within the cylindrical portion of the frame, which is typically proximal to blood-inlet openings of the ventricular assist device. For some such applications, even if structures of the left ventricle (such as chordae tendineae, trabeculae carneae, and/or papillary muscles) enter into the frame via the blood-inlet openings, any damage to the axial shaft or other portions of the ventricular assist device are reduced or prevented by virtue of the axial shaft terminating proximally to the blood-inlet openings. For some such applications, even if structures of the left ventricle (such as chordae tendineae, trabeculae carneae, and/or papillary muscles) enter into the frame via the blood-inlet openings, any injury to such structures is reduced or prevented by virtue of the axial shaft terminating proximally to the blood-inlet openings.

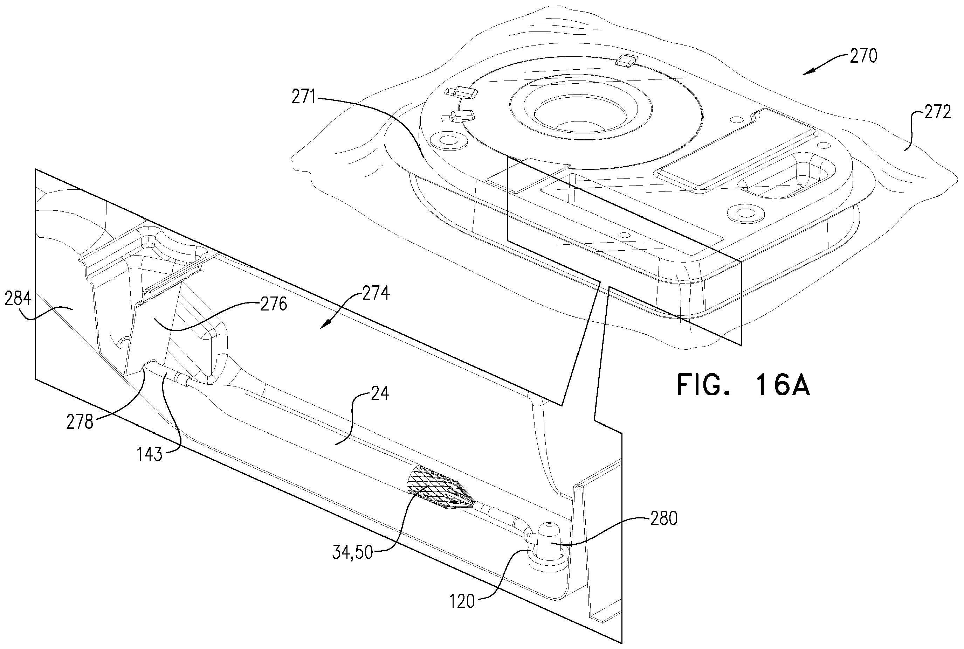

Reference is now made to Figs. 16A, 16B, 16C, and 16D, which are schematic illustrations of packaging 270 for packaging ventricular assist device 20, in accordance with some applications of the present invention. Typically, packaging 270 is packaged within a sealed outer wrapping 272, such as to enhance the sterility of the packaging. Packaging 270 is typically shaped to define a pump-head chamber 274, in which the pump head (e.g., impeller 50 and frame 34) of the ventricular asst device are packaged. For some applications, a catheter-securement piece 276 is reversibly coupled to the packaging 270. The catheter-securement piece is shaped to define a hole 278. As part of the process of packaging the ventricular assist device within packaging 270, the distal end of delivery catheter 143 is secured within the hole, by coupling the catheter-securement piece to the packaging with the distal end of the delivery catheter disposed within the hole. For some applications, the pump-head chamber defines a vertical protrusion 280, around which distal-tip portion 120 is placed, such as to secure the distal end of the pump head within the pump-head chamber.

For some applications, in order to unpackage the ventricular assist device and prepare it for use, a top cover 271 of the packaging is removed, such as to expose pump-head chamber 274. For some applications, the pump-head chamber is then filled within a solution 282, such as saline, as indicated in Fig. 16B. Subsequently, the pump head is retracted into the distal end of delivery catheter 143, such as to radially constrain the pump head, as shown in transition from Fig. 16B to Fig. 16D. For some applications, the catheter-securement piece is configured to secure the distal end of the delivery catheter to a surface 284 that is slanted downwards, such that the distal end of the catheter is oriented in a downwards slant. Typically, this reduces a likelihood of air bubbles entering the distal end of the delivery catheter as the pump head is retracted into the distal end of delivery catheter, relative to if the distal end of the catheter were to be disposed in a horizontal orientation.

With regards to all aspects of ventricular assist device 20 described with reference to Figs. 1A-15, it is noted that, although Figs. 1A and 1B show ventricular assist device 20 in the subject's left ventricle, for some applications, ventricular assist device 20 is placed inside the subject's right ventricle, such that the device traverses the subject's pulmonary valve, and techniques described herein are applied, *mutatis mutandis.* For some applications, components of device 20 are applicable to different types of blood pumps. For example, aspects of the present invention may be applicable to a pump that is used to pump blood from the vena cava and/or the right atrium into the right ventricle, from the vena cava and/or the right atrium into the pulmonary artery, and/or from the renal veins into the vena cava. Such aspects may include features of tube 24 (e.g., the curvature of the tube), impeller 50, features of pump-head portion 27, drive cable 130, etc. Alternatively or additionally, device 20 and/or a portion thereof (e.g., impeller 50, even in the absence of tube 24) is placed inside a different portion of the subject's body, in order to assist with the pumping of blood from that portion. For example, device 20 and/or a portion thereof (e.g., impeller 50, even in the absence of tube 24) may be placed in a blood vessel and may be used to pump blood through the blood vessel. For some applications, device 20 and/or a portion thereof (e.g., impeller 50, even in the absence of tube 24) is configured to be placed within the subclavian vein or jugular vein, at junctions of the vein with a lymph duct, and is used to increase flow of lymphatic fluid from the lymph duct into the vein, *mutatis mutandis.* Since the scope of the present invention includes using the apparatus and methods described herein in anatomical locations other than the left ventricle and the aorta, the ventricular assist device and/or portions thereof are sometimes referred to herein (in the specification and the claims) as a blood pump.

The scope of the present invention includes combining any of the apparatus according to the appended claims with any of the apparatus described in one or more of the following applications:

International Application No. PCT/IB2022/051990 to Tuval, entitled "Ventricular assist device," filed March 07, 2022, which claims priority from:

US Provisional Patent Application 63/158,708 to Tuval, entitled "Ventricular assist device," filed March 09, 2021, and

US Provisional Patent Application 63/254,321 to Tuval, entitled "Ventricular assist device," filed October 11, 2021.

International Application No. PCT/IB2021/052590 to Zipory (published as WO 21/198881), entitled "Centrifugal and mixed-flow impellers for use with a blood pump," filed March 29, 2021, which claims priority from US 63/003,955 to Zipory, entitled "Ventricular assist device," filed April 02, 2020.

US 2022/0226632 to Tuval, entitled "Ventricular assist device," which is the US national phase of PCT Application No. PCT/IB2021/052857 (published as WO 21/205346), filed April 06, 2021, which claims priority from:

US Provisional Patent Application 63/006,122 to Tuval, entitled "Ventricular assist device," filed April 07, 2020;

US Provisional Patent Application 63/114,136 to Tuval, entitled "Ventricular assist device," filed Nov. 16, 2020; and

US Provisional Patent Application 63/129,983 to Tuval, entitled "Ventricular assist device," filed Dec. 23, 2020.

US 2020/0237981 to Tuval, entitled "Distal tip element for a ventricular assist device," filed January 23, 2020, which claims priority from:

US Provisional Patent Application 62/796,138 to Tuval, entitled "Ventricular assist device," filed Jan. 24, 2019;

US Provisional Patent Application 62/851,716 to Tuval, entitled "Ventricular assist device," filed May 23, 2019;

US Provisional Patent Application 62/870,821 to Tuval, entitled "Ventricular assist device," filed July 05, 2019; and

US Provisional Patent Application 62/896,026 to Tuval, entitled "Ventricular assist device," filed Sep. 05, 2019.

US 2019/0209758 to Tuval, which is a continuation of International Application No. PCT/IB2019/050186 to Tuval (published as WO 19/138350), entitled "Ventricular assist device," filed January 10, 2019, which claims priority from:

US Provisional Patent Application 62/615,538 to Sohn, entitled "Ventricular assist device," filed January 10, 2018;

US Provisional Patent Application 62/665,718 to Sohn, entitled "Ventricular assist device," filed May 02, 2018;

US Provisional Patent Application 62/681,868 to Tuval, entitled "Ventricular assist device," filed June 07, 2018; and

US Provisional Patent Application 62/727,605 to Tuval, entitled "Ventricular assist device," filed September 06, 2018;

US 2019/0269840 to Tuval, which is the US national phase of International Patent Application PCT/IL2017/051273 to Tuval (published as WO 18/096531), filed November 21, 2017, entitled "Blood pumps," which claims priority from US Provisional Patent Application 62/425,814 to Tuval, filed November 23, 2016;

US 2019/0175806 to Tuval, which is a continuation of International Application No. PCT/IL2017/051158 to Tuval (published as WO 18/078615), entitled "Ventricular assist device," filed October 23, 2017, which claims priority from US 62/412,631 to Tuval filed October 25, 2016, and US 62/543,540 to Tuval, filed August 10, 2017;

US 2019/0239998 to Tuval, which is the US national phase of International Patent Application PCT/IL2017/051092 to Tuval (published as WO 18/061002), filed September 28, 2017, entitled "Blood vessel tube," which claims priority from US Provisional Patent Application 62/401,403 to Tuval, filed September 29, 2016;

US 2018/0169313 to Schwammenthal, which is the US national phase of International Patent Application PCT/IL2016/050525 to Schwammenthal (published as WO 16/185473), filed May 18, 2016, entitled "Blood pump," which claims priority from US Provisional Patent Application 62/162,881 to Schwammenthal, filed May 18, 2015, entitled "Blood pump;"

US 2017/0100527 to Schwammenthal, which is the US national phase of International Patent Application PCT/IL2015/050532 to Schwammenthal (published as WO 15/177793), filed May 19, 2015, entitled "Blood pump," which claims priority from US Provisional Patent Application 62/000,192 to Schwammenthal, filed May 19, 2014, entitled "Blood pump;"

US Patent US 10,039,874 to Schwammenthal, which is the US national phase of International Patent Application PCT/IL2014/050289 to Schwammenthal (published as WO 14/141284), filed March 13, 2014, entitled "Renal pump," which claims priority from (a) US Provisional Patent Application 61/779,803 to Schwammenthal, filed March 13, 2013, entitled "Renal pump," and (b) US Provisional Patent Application 61/914,475 to Schwammenthal, filed December 11, 2013, entitled "Renal pump;"

US Patent 9,764,113 to Tuval, issued September 19, 2017, entitled "Curved catheter," which claims priority from US Provisional Patent Application 61/914,470 to Tuval, filed December 11, 2013, entitled "Curved catheter;" and

US Patent 9,597,205 to Tuval, which is the US national phase of International Patent Application PCT/IL2013/050495 to Tuval (published as WO 13/183060), filed June 06, 2013, entitled "Prosthetic renal valve," which claims priority from US Provisional Patent Application 61/656,244 to Tuval, filed June 06, 2012, entitled "Prosthetic renal valve."

It will be appreciated by persons skilled in the art that the present invention is not limited to what has been particularly shown and described hereinabove. Rather, the scope of the present invention is only defined by the following claims.

**Claims**

1. An apparatus comprising:

a ventricular assist device (20) comprising:

an impeller (50) configured to be placed inside a left ventricle of a subject, the impeller (50) defining a lumen (62) therethrough;

a frame (34) configured to be disposed around the impeller (50);

a proximal bearing (116) disposed at a proximal end of the frame (34) and a distal bearing (118) disposed at a distal end of the frame (34);

an axial shaft (92) passing through the proximal bearing (116), the lumen (62) defined by the impeller (50), and the distal bearing (118); **characterised by**

a motion-cushioning spring (68) disposed around the axial shaft (92) between a distal end of the impeller (50) and the distal bearing (118), the motion-cushioning spring (68) being configured to cushion axial motion that the impeller (50) undergoes.

2. The apparatus according to claim 1, wherein the impeller (50) is configured to undergo axial back-and-forth motion while the impeller (50) is rotating, and wherein the motion-cushioning spring (68) is configured to provide cushioning to the axial back-and-forth motion.

3. The apparatus according to claim 1, wherein the impeller (50) is configured to be radially constrained by becoming axially elongated and wherein the motion-cushioning spring (68) is configured to become compressed such as to accommodate the axial elongation of the impeller (50).

4. The apparatus according to claim 1, wherein the motion-cushioning spring (68) is coupled to a distal end of the impeller (50).

5. The apparatus according to claim 1, wherein the ventricular assist device further comprises a proximal motion-cushioning spring (68) disposed around the axial shaft (92) between a proximal end of the impeller (50) and the proximal bearing (116), the motion-cushioning spring (68) being configured to cushion axial motion that the impeller (50) undergoes in a proximal direction.

6. The apparatus according to any one of claims 1-5, wherein the motion-cushioning spring (68) is coupled to the distal bearing (118).

7. The apparatus according to claim 6, further comprising a distal bearing housing (118H) disposed around the distal bearing (118), wherein the motion-cushioning spring (68) is coupled to the distal bearing (118) via the distal bearing housing (118H).

8. The apparatus according to any one of claims 1-5, further comprising an elastomeric material (69) that is coupled to the motion-cushioning spring (68), such that at least a portion of the axial shaft (92) between a distal end of the impeller (50) and the distal bearing (118) is covered by a combination of the motion-cushioning spring (68) and the elastomeric material (69).

9. The apparatus according to claim 8, wherein the ventricular assist device comprises a purging system that is configured to pump a purging fluid through a lumen defined by the axial shaft (92), such that at least a portion of the purging fluid flows proximally through an interface between the axial shaft (92) and the combination of the motion-cushioning spring (68) and the elastomeric material (69).

10. The apparatus according to claim 8, wherein the elastomeric material (69) is coupled to the motion-cushioning spring (68) in such a manner that the elastomeric material (69) changes shape to conform to shape changes that the motion-cushioning spring (68) undergoes.

11. The apparatus according to claim 10, wherein the elastomeric material (69) is configured to undergo the changes in shape without the elastomeric material (69) becoming broken or collapsing.

12. The apparatus according to claim 10, wherein the elastomeric material (69) is configured not to become creased as a result of the motion-cushioning spring (68) being compressed.

13. The apparatus according to any one of claims 1-5, wherein the ventricular assist device further comprises a pump-outlet tube (24) configured to traverse an aortic valve of the subject, such that a proximal portion of the pump-outlet tube (24) is disposed within an aorta of the subject and a distal portion of the pump-outlet tube (24) is disposed within the subject's left ventricle, the distal portion of the pump-outlet tube (24) extending to the distal end of the frame (34) and defining one or more lateral blood inlet openings (108) that are configured to allow blood to flow from the subject's left ventricle into the pump-outlet tube.

14. The apparatus according to claim 13, wherein the distal portion of the pump-outlet tube defines (24) more than 10 blood-inlet openings (108) that are sized such as (a) to allow blood to flow from the subject's left ventricle into the tube and (b) to block structures from the subject's left ventricle from entering into the frame (34).

15. The apparatus according to claim 14, wherein the distal portion of the pump-outlet tube (24) defines more than 50 blood-inlet openings (108) that are sized such as (a) to allow blood to flow from the subject's left ventricle into the tube and (b) to block structures from the subject's left ventricle from entering into the frame (34).

**Patentansprüche**

1. Einrichtung, umfassend:
   eine ventrikuläre Hilfsvorrichtung (20), umfassend:

   ein Laufrad (50), das dazu konfiguriert ist, in einem linken Ventrikel eines Subjekts platziert zu werden, wobei das Laufrad (50) ein Lumen (62) dadurch definiert,
   einen Rahmen (34), der dazu konfiguriert ist, um das Laufrad (50) angeordnet zu werden;
   ein proximales Lager (116), das an einem proximalen Ende des Rahmens (34) angeordnet ist, und ein distales Lager (118), das an einem distalen Ende des Rahmens (34) angeordnet ist;
   eine axiale Welle (92), die durch das proximale Lager (116), das durch das Laufrad (50) definierte Lumen (62) und das distale Lager (118) verläuft; **gekennzeichnet durch**
   eine bewegungsdämpfende Feder (68), die zwischen einem distalen Ende des Laufrads (50) und dem distalen Lager (118) um die axiale Welle (92) angeordnet ist, wobei die bewegungsdämpfende Feder (68) dazu konfiguriert ist, eine axiale Bewegung, die das Laufrad (50) durchläuft, zu dämpfen.

2. Einrichtung nach Anspruch 1, wobei das Laufrad (50) dazu konfiguriert ist, eine axiale Hin- und Herbewegung zu durchlaufen, während sich das Laufrad (50) dreht, und wobei die bewegungsdämpfende Feder (68) dazu konfiguriert ist, eine Dämpfung für die axiale Hin- und Herbewegung bereitzustellen.

3. Einrichtung nach Anspruch 1, wobei das Laufrad (50) dazu konfiguriert ist, durch eine axiale Verlängerung radial eingeschränkt zu werden, und wobei die bewegungsdämpfende Feder (68) dazu konfiguriert ist, zusammengedrückt zu werden, um die axiale Verlängerung des Laufrads (50) aufzunehmen.

4. Einrichtung nach Anspruch 1, wobei die bewegungsdämpfende Feder (68) an ein distales Ende des Laufrads (50) gekoppelt ist.

5. Einrichtung nach Anspruch 1, wobei die ventrikuläre Hilfsvorrichtung ferner eine proximale bewegungsdämpfende Feder (68) umfasst, die zwischen einem proximalen Ende des Laufrads (50) und dem proximalen Lager (116) um die axiale Welle (92) angeordnet ist, wobei die bewegungsdämpfende Feder (68) dazu konfiguriert ist, eine axiale Bewegung, die das Laufrad (50) in einer proximalen Richtung durchläuft, zu dämpfen.

6. Einrichtung nach einem der Ansprüche 1-5, wobei die bewegungsdämpfende Feder (68) an das distale Lager (118) gekoppelt ist.

7. Einrichtung nach Anspruch 6, ferner umfassend ein distales Lagergehäuse (118H), das um das distale Lager (118) angeordnet ist, wobei die bewegungsdämpfende Feder (68) über das distale Lagergehäuse (118H) an das distale Lager (118) gekoppelt ist.

8. Einrichtung nach einem der Ansprüche 1-5, ferner umfassend ein Elastomermaterial (69), das an die bewegungsdämpfende Feder (68) gekoppelt ist, sodass mindestens ein Teil der axiale Welle (92) zwischen einem distalen Ende des Laufrads (50) und dem distalen Lager (118) durch eine Kombination aus der bewegungsdämpfenden Feder (68) und dem Elastomermaterial (69) abgedeckt wird.

**9.** Einrichtung nach Anspruch 8, wobei die ventrikuläre Hilfsvorrichtung ein Spülsystem umfasst, das dazu konfiguriert ist, eine Spülflüssigkeit durch ein durch die axiale Welle (92) definiertes Lumen zu pumpen, sodass mindestens ein Teil der Spülflüssigkeit proximal durch eine Schnittstelle zwischen der axiale Welle (92) und der Kombination aus der bewegungsdämpfenden Feder (68) und dem Elastomermaterial (69) fließt.

**10.** Einrichtung nach Anspruch 8, wobei das Elastomermaterial (69) auf eine solche Weise an die bewegungsdämpfende Feder (68) gekoppelt ist, dass das Elastomermaterial (69) seine Form ändert, um sich an Formänderungen anzupassen, welche die bewegungsdämpfende Feder (68) durchläuft.

**11.** Einrichtung nach Anspruch 10, wobei das Elastomermaterial (69) dazu konfiguriert ist, Formänderungen zu durchlaufen, ohne dass das Elastomermaterial (69) bricht oder kollabiert.

**12.** Einrichtung nach Anspruch 10, wobei das Elastomermaterial (69) dazu konfiguriert ist, keine Falten als Folge des Zusammendrückens der bewegungsdämpfenden Feder (68) zu bilden.

**13.** Einrichtung nach einem der Ansprüche 1-5, wobei die ventrikuläre Hilfsvorrichtung ferner einen Pumpenauslassschlauch (24) umfasst, der dazu konfiguriert ist, eine Aortenklappe des Subjekts zu durchqueren, sodass ein proximaler Teil des Pumpenauslassschlauchs (24) in einer Aorta des Subjekts angeordnet ist und ein distaler Teil des Pumpenauslassschlauchs (24) in dem linken Ventrikel des Subjekts angeordnet ist, wobei sich der distale Teil des Pumpenauslassschlauchs (24) zu dem distalen Ende des Rahmens (34) erstreckt und eine oder mehrere seitliche Bluteinlassöffnungen (108) definiert, die dazu konfiguriert sind, zu ermöglichen, dass Blut aus dem linken Ventrikel des Subjekts in den Pumpenauslassschlauch fließt.

**14.** Einrichtung nach Anspruch 13, wobei der distale Teil des Pumpenauslassschlauchs mehr als 10 Bluteinlassöffnungen (108) definiert (24), die so bemessen sind, dass (a) ermöglicht wird, dass Blut aus dem linken Ventrikel des Subjekts in den Schlauch fließt, und (b) verhindert wird, dass Strukturen aus der linken Ventrikel des Subjekts in den Rahmen (34) eindringen.

**15.** Einrichtung nach Anspruch 14, wobei der distale Teil des Pumpenauslassschlauchs (24) mehr als 50 Bluteinlassöffnungen (108) definiert, die so bemessen sind, dass (a) ermöglicht wird, dass Blut aus dem linken Ventrikel des Subjekts in den Schlauch fließt, und (b) verhindert wird, dass Strukturen aus dem linken Ventrikel des Subjekts in den Rahmen (34) eindringen.

**Revendications**

**1.** Appareil comprenant :
un dispositif d'assistance ventriculaire (20) comprenant :

une hélice (50) conçue pour être placée à l'intérieur d'un ventricule gauche d'un sujet, l'hélice (50) définissant une lumière (62) à travers celle-ci ;
une armature (34) conçue pour être disposée autour de l'hélice (50) ;
un palier proximal (116) disposé au niveau d'une extrémité proximale de l'armature (34) et un palier distal (118) disposé au niveau d'une extrémité distale de l'armature (34) ;
un arbre axial (92) passant à travers le palier proximal (116), la lumière (62) définie par l'hélice (50) et le palier distal (118) ; **caractérisé par**
un ressort amortisseur de mouvement (68) disposé autour de l'arbre axial (92) entre une extrémité distale de l'hélice (50) et le palier distal (118), le ressort amortisseur de mouvement (68) étant conçu pour amortir le mouvement axial que l'hélice (50) subit.

**2.** Appareil selon la revendication 1, ladite hélice (50) étant conçue pour subir un mouvement axial de va-et-vient pendant que l'hélice (50) tourne, et ledit ressort amortisseur de mouvement (68) étant conçu pour fournir un amortissement du mouvement axial de va-et-vient.

**3.** Appareil selon la revendication 1, ladite hélice (50) étant conçue pour être contrainte radialement en s'allongeant axialement et ledit ressort amortisseur de mouvement (68) étant conçu pour être comprimé de façon à s'adapter à l'allongement axial de l'hélice (50).

**4.** Appareil selon la revendication 1, ledit ressort amortisseur de mouvement (68) étant couplé à une extrémité distale de l'hélice (50).

**5.** Appareil selon la revendication 1, ledit dispositif d'assistance ventriculaire comprenant en outre un ressort amortisseur de mouvement proximal (68) disposé autour de l'arbre axial (92) entre une extrémité proximale de l'hélice (50) et le palier proximal (116), le ressort amortisseur de mouvement (68) étant conçu pour amortir le mouvement axial que l'hélice (50) subit dans une direction proximale.

**6.** Appareil selon l'une quelconque des revendications 1 à 5, ledit ressort amortisseur de mouvement (68) étant couplé au palier distal (118).

**7.** Appareil selon la revendication 6, comprenant en outre un boîtier de palier distal (118H) disposé autour du palier distal (118), ledit ressort amortisseur de mouvement (68) étant couplé au palier distal (118) par l'intermédiaire du boîtier de palier distal (118H).

**8.** Appareil selon l'une quelconque des revendications 1-5, comprenant en outre un matériau élastomère (69) qui est couplé au ressort amortisseur de mouvement (68), de sorte qu'au moins une partie de l'arbre axial (92) entre une extrémité distale de l'hélice (50) et le palier distal (118) soit recouverte par une combinaison du ressort amortisseur de mouvement (68) et du matériau élastomère (69).

**9.** Appareil selon la revendication 8, ledit dispositif d'assistance ventriculaire comprenant un système de purge qui est conçu pour pomper un fluide de purge à travers une lumière définie par l'arbre axiale (92), de sorte qu'au moins une partie du fluide de purge s'écoule de manière proximale à travers une interface entre l'arbre axial (92) et la combinaison du ressort amortisseur de mouvement (68) et du matériau élastomère (69).

**10.** Appareil selon la revendication 8, ledit matériau élastomère (69) étant couplé au ressort amortisseur de mouvement (68) d'une manière telle que le matériau élastomère (69) change de forme pour se conformer aux changements de forme que le ressort amortisseur de mouvement (68) subit.

**11.** Appareil selon la revendication 10, ledit matériau élastomère (69) étant conçu pour subir les changements de forme sans que le matériau élastomère (69) ne se brise ou ne s'effondre.

**12.** Appareil selon la revendication 10, ledit matériau élastomère (69) étant conçu pour ne pas se froisser à la suite de la compression du ressort amortisseur de mouvement (68).

**13.** Appareil selon l'une quelconque des revendications 1-5, ledit dispositif d'assistance ventriculaire comprenant en outre un tube de sortie de pompe (24) conçu pour traverser une valve aortique du sujet, de sorte qu'une partie proximale du tube de sortie de pompe (24) est disposée à l'intérieur d'une aorte du sujet et une partie distale du tube de sortie de pompe (24) est disposée à l'intérieur du ventricule gauche du sujet, la partie distale du tube de sortie de pompe (24) s'étendant jusqu'à l'extrémité distale de l'armature (34) et définissant une ou plusieurs ouvertures d'entrée de sang latérales (108) qui sont conçues pour permettre au sang de circuler à partir du ventricule gauche du sujet jusque dans le tube de sortie de pompe.

**14.** Appareil selon la revendication 13, ladite partie distale du tube de sortie de pompe (24) définissant plus de 10 ouvertures d'entrée de sang (108) qui sont dimensionnées de façon (a) à permettre la circulation du sang à partir du ventricule gauche du sujet jusque dans le tube et (b) à empêcher les structures du ventricule gauche du sujet d'entrer dans l'armature (34).

**15.** Appareil selon la revendication 14, ladite partie distale du tube de sortie de pompe (24) définissant plus de 50 ouvertures d'entrée de sang (108) qui sont dimensionnées de manière (a) à permettre la circulation du sang à partir du ventricule gauche du sujet jusque dans le tube et (b) à empêcher les structures du ventricule gauche du sujet d'entrer dans l'armature (34).

FIG. 1A

**FIG. 1B**

FIG. 1C

FIG. 2

EP 4 271 461 B1

FIG. 3A

FIG. 3B

FIG. 3C

EP 4 271 461 B1

FIG. 3D

FIG. 3E

EP 4 271 461 B1

FIG. 4

FIG. 5A

FIG. 5B

FIG. 5C

117    33

34

95
240

33

116
116H
92

117

FIG. 6A

FIG. 6B

FIG. 6C

FIG. 6D

FIG. 7A

FIG. 7B

FIG. 7C

EP 4 271 461 B1

FIG. 8A

FIG. 8B

FIG. 8C

FIG. 9A

FIG. 9B

FIG. 10A

EP 4 271 461 B1

FIG. 10B

FIG. 11A

FIG. 11B

FIG. 11C

FIG. 11D

EP 4 271 461 B1

FIG. 11E

EP 4 271 461 B1

FIG. 12A

FIG. 12B

FIG. 12C

FIG. 12D

FIG. 13

EP 4 271 461 B1

FIG. 14A

FIG. 14B

FIG. 15

FIG. 16A

EP 4 271 461 B1

FIG. 16B

FIG. 16C

FIG. 16D

## REFERENCES CITED IN THE DESCRIPTION

### Patent documents cited in the description

- US 20200237982 A1
- US 10881770 B, Tuval
- WO IB2022051990 A, Tuval
- US 63158708, Tuval
- US 63254321, Tuval
- WO IB2021052590 A, Zipory
- WO 21198881 A
- US 63003955 B, Zipory
- US 20220226632 A, Tuval
- WO IB2021052857 A
- WO 21205346 A
- US 63006122, Tuval
- US 63114136, Tuval
- US 63129983, Tuval
- US 20200237981 A, Tuval
- US 62796138, Tuval
- US 62851716, Tuval
- US 62870821, Tuval
- US 62896026, Tuval
- US 20190209758 A, Tuval
- WO IB2019050186 A, Tuval
- WO 19138350 A
- US 62615538, Sohn
- US 62665718, Sohn
- US 62681868, Tuval
- US 62727605, Tuval
- US 20190269840 A, Tuval
- IL 2017051273 W, Tuval
- WO 18096531 A
- US 62425814, Tuval
- US 20190175806 A, Tuval
- IL 2017051158 W, Tuval
- WO 18078615 A
- US 62412631 B, Tuval
- US 62543540 B, Tuval
- US 20190239998 A, Tuval
- IL 2017051092 W, Tuval
- WO 18061002 A
- US 62401403, Tuval
- US 20180169313 A, Schwammenthal
- IL 2016050525 W, Schwammenthal
- WO 16185473 A
- US 62162881, Schwammenthal
- US 20170100527 A, Schwammenthal
- IL 2015050532 W, Schwammenthal
- WO 15177793 A
- US 62000192, Schwammenthal
- US 10039874 B, Schwammenthal
- IL 2014050289 W, Schwammenthal
- WO 14141284 A
- US 61779803, Schwammenthal
- US 61914475, Schwammenthal
- US 9764113 B, Tuval
- US 61914470, Tuval
- US 9597205 B, Tuval
- IL 2013050495 W, Tuval
- WO 13183060 A
- US 61656244, Tuval